# EUROPEAN PATENT SPECIFICATION

(11) **EP 1 419 155 B1**
(45) Date of publication and mention of the grant of the patent: **08.11.2006**
(21) Application number: 02764786.6
(22) Date of filing: 25.07.2002
(51) Int. Cl.: C07D 471/04

(54) **PIPERIDINE DERIVATIVES AS ANTIBACTERIAL AGENTS**
PIPERIDINDERIVATE ALS ANTIBAKTERIELLE MITTEL
DERIVES DE PIPERIDINE COMME AGENTS ANTIBACTERIENS

(30) Priority: 26.07.2001 GB 0118238
(43) Date of publication of application: 19.05.2004
(73) Proprietor: SMITHKLINE BEECHAM PLC, Brentford, Middlesex TW8 9GS (GB)
(72) Inventor: DAVIES, David, Thomas, GlaxoSmithKline, Harlow, Essex CM19 5AW (GB); JONES, Graham, Elgin, GlaxoSmithKline, Harlow, Essex CM19 5AW (GB); MARKWELL, Roger, Edward, GlaxoSmithKline, Harlow, Essex CM19 5AW (GB); PEARSON, Neil, David, GlaxoSmithKline, Harlow, Essex CM19 5AW (GB)
(74) Representative: Valentine, Jill Barbara
(86) International application number: PCT/EP2002/008319
(87) International publication number: WO 2003/010138

(56) References cited:
- WO-A-00/21948
- WO-A-00/21952
- WO-A-00/43383
- WO-A-01/07433
- WO-A-02/40474

## Description

This invention relates to novel compounds, compositions containing them and their use as antibacterials.

WO99/37635, WO00/21948, WO00/21952, WO00/43383, WO00/78748, WO01/07433, WO01/07432, WO01/25227, WO0208224, WO0224684, PCT/GB01/05653, PCT/GB01/05661 and WO02040474 disclose quinoline and naphthyridine derivatives having antibacterial activity.

This invention provides a compound of formula (I) or a pharmaceutically acceptable derivative thereof: 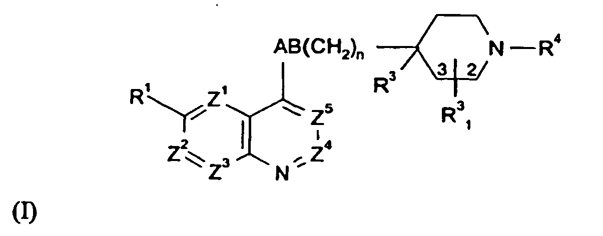 wherein:
one of Z¹, Z², Z³, Z⁴ and Z⁵ is N, one is CR^{1a} and the remainder are CH, or one or two of Z¹, Z², Z³, Z⁴ and Z⁵ are independently CR^{1a} and the remainder are CH;
R¹ and R^{1a} are independently selected from hydrogen; hydroxy; (C₁₋₆) alkoxy optionally substituted by (C₁₋₆)alkoxy, amino, piperidyl, guanidino or amidino any of which is optionally N-substituted by one or two (C₁₋₆)alkyl, acyl or (C₁₋₆)alkylsulphonyl groups, CONH₂, hydroxy, (C₁₋₆)alkylthio, heterocyclylthio, heterocyclyloxy, arylthio, aryloxy, acylthio, acyloxy or (C₁₋₆)alkylsutphonyloxy; (C₁₋₆)alkoxy-substituted (C₁₋₆)alkyl; halogen; (C₁₋₆)alkyl; (C₁₋₆)alkylthio; trifluromethyl; trifluoromethoxy; nitro; azido; acyl; acyloxy; acylthio; (C ₁₋₆)alkylsulphonyl; (C ₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups, provided that when Z¹, Z², Z³, Z⁴ and Z⁵ are CR^{1a} or CH, then R¹ is not hydrogen; or when Z⁵ is CR^{1a}, R^{1a} may instead be cyano, hydroxymethyl or carboxy;
R³ is:
   carboxy; (C ₁₋₆)alkoxycarbonyl; aminocarbonyl wherein the amino group is optionally substituted by hydroxy, (C₁₋₆)alkyl, hydroxy(C₁₋₆)alkyl, aminocarbonyl(C₁₋₆)alkyl, (C₂₋₆)alkenyl, (C₁₋₆)alkylsulphonyl, trifluoromethylsulphonyl, (C₂₋₆)alkenylsulphonyl, (C_{1- 6})alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbony](C₁₋₆)alkyl or (C₂₋₆)alkenyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; or 5-oxo-1,2,4-oxadiazol-3-yl; or
   (C₁₋₄)alkyl or ethenyl optionally substituted with any of the groups listed above for R³ and/or 0 to 2 groups R¹² independently selected from:
      halogen; (C₁₋₆)alkylthio; trifluoromethyl; (C₁₋₆)alkoxycarbonyl; (C₁₋₆)alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; amino optionally mono- or disubstituted by (C₁₋₆)alkoxycarbonyl, (C ₁₋₆)alkylcarbonyl, (C₂₋₆)alkenyloxycarbonyl, (C₂₋₆)alkenylcarbonyl, (C ₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl; aminocarbonyl wherein the amino group is optionally substituted by (C₁₋₆)alkyl, hydroxy(C₁₋₆)alkyl, aminocarbonyl(C₁₋₆)alkyl, (C₂₋₆)alkenyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; oxo; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₂₋₆)alkenyl; or
      hydroxy or thiol optionally substituted by (C₁₋₆)alkyl, (C₁₋₄)alkoxy(C₁₋₄)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; or
      amino optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl; or
      halogen or trifluoromethyl;
      in addition when R³ is disubstituted with a hydroxy or amino containing substituent and a carboxy containing substituent these may optionally together form a cyclic ester or amide linkage, respectively;
   R¹⁰ is selected from (C₁₋₄)alkyl and (C₂₋₄)alkenyl either of which may be optionally substituted by a group R¹² as defined above; carboxy; aminocarbonyl wherein the amino group is optionally substituted by hydroxy, (C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C ₁₋₆)alkylcarbonyl, (C₂₋₆)alkenyloxycarbonyl or (C₂₋₆)alkenylcarbonyl and optionally further substituted by (C ₁₋₆)alkyl or (C₂₋₆)alkenyl; (C₁₋₆)alkylsulphonyl; trifluoromethylsulphonyl; (C₂₋₆)alkenylsulphonyl; (C₁₋₆)alkoxycarbonyl; (C ₁₋₆)alkylcarbonyl; (C₂₋₆)alkenyloxycarbonyl; and (C₂₋₆)alkenylcarbonyl;
   R³₁ is in the 2- or 3-position and is hydrogen or a group listed above for R³, provided that R³₁ in the 2-position is not optionally substituted hydroxyl, amino, trifluoromethyl or halogen;
   R⁴ is a group -U-V-R⁵₂ where R⁵₂ is an optionally substituted bicyclic carbocyclic or heterocyclic ring system (A): 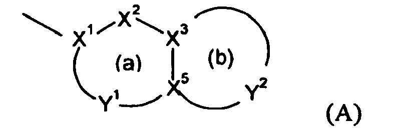
   containing up to four heteroatoms in each ring in which
   at least one of rings (a) and (b) is aromatic;
   X¹ is C or N when part of an aromatic ring or CR¹⁴ when part of a non aromatic ring;
   X² is N, NR¹³, O, S(O)ₓ, CO or CR¹⁴ when part of an aromatic or non-aromatic ring or may in addition be CR¹⁴R¹⁵ when part of a non aromatic ring;
   X³ and X⁵ are independently N or C;
   Y¹ is a 0 to 4 atom linker group each atom of which is independently selected
   from N, NR¹³, O, S(O)ₓ, CO and CR¹⁴ when part of an aromatic or non-aromatic ring or may additionally be CR¹⁴R¹⁵ when part of a non aromatic ring,
   Y² is a 2 to 6 atom linker group, each atom of Y² being independently selected from N, NR¹³, O, S(O)ₓ, CO and CR¹⁴ when part of an aromatic or non-aromatic ring or may additionally be CR¹⁴R¹⁵ when part of a non aromatic ring;
   each of R¹⁴ and R¹⁵ is independently selected from: H; (C₁₋₄)alkylthio; halo; carboxy(C ₁₋₄)alkyl; halo(C ₁₋₄)alkoxy; halo(C₁₋₄)alkyl; (C ₁₋₄)alkyl; (C₂₋₄)alkenyl; (C₁₋₄)alkoxycarbonyl; formyl; (C ₁₋₄)alkylcarbonyl; (C₂₋₄)alkenyloxycarbonyl; (C₂₋₄)alkenylcarbonyl; (C ₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C ₁₋₄)alkyl or (C₂₋₄)alkenyl; aryl; aryl(C ₁₋₄)alkyl; aryl(C₁₋₄)alkoxy;
   each R¹³ is independently H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl (C₁₋₄)alkyl; arylcarbonyl; heteroarylcarbonyl; (C ₁₋₄)alkoxycarbonyl; (C₁₋₄)alkylcarbonyl; formyl; (C₁₋₆)alkylsulphonyl; or aminocarbonyl wherein the amino group is optionally substituted by (C ₁₋₄)alkoxycarbonyl, (C ₁₋₄)alkylcarbonyl, (C₂₋₄)alkenyloxycarbonyl, (C₂₋₄)alkenylcarbonyl, (C ₁₋₄)alkyl or (C₂₋₄)alkenyl and optionally further substituted by (C₁₋₄)alkyl or (C₂₋₄)alkenyl;
   each x is independently 0, 1 or 2;
   U is CO, SO₂ or CH₂ and V is CR¹⁷R¹⁸ or U is CH₂ and V is CO, C=NOR¹⁹ or SO₂; R¹⁷ and R¹⁸ are independently selected from hydrogen, hydroxy optionally substituted by (C ₁₋₆)alkyl, (C₂₋₆)alkenyl, (C ₁₋₆)alkoxycarbonyl, (C ₁₋₆)alkylcarbonyl, (C₂₋₆)alkenyloxycarbonyl, (C₂₋₆)alkenylcarbonyl or aminocarbonyl wherein the amino group is optionally substituted by (C ₁₋₆)alkyl, (C₂₋₆)alkenyl, (C ₁₋₆)alkylcarbonyl or (C₂₋₆)alkenylcarbonyl; and amino optionally mono- or disubstituted by (C₁₋₆)alkoxycarbonyl, (C ₁₋₆)alkylcarbonyl, (C₂₋₆)alkenyloxycarbonyl, (C₂₋₆)alkenylcarbonyl, (C ₁₋₆)alkyl, (C₂₋₆)alkenyl, (C ₁₋₆)alkylsulphonyl, (C₂₋₆)alkenylsulphonyl or aminocarbonyl wherein the amino group is optionally substituted by (C ₁₋₆)alkyl or (C₂₋₆)alkenyl; R¹⁹ is hydrogen or is selected from (C₁₋₄)alkyl and (C₂₋₄)alkenyl optionally substituted with any of the substituents listed above for R³ (C ₁₋₄)alkyl or ethenyl; n is 0 or 1 and AB is NR¹¹CO, CONR¹¹, CO-CR⁸R⁹, CR⁶R⁷-CO, O-CR⁸R⁹, CR⁶R⁷⁻O , NR¹¹-CR⁸R⁹, CR⁶R⁷- NR¹¹, NR¹¹SO₂, CR⁶R⁷-SO₂ or CR⁶R⁷-CR⁸R⁹, or n is 0 and AB is NH-CO-NH or NH-CO-O;
   or n is 0 and AB is CR⁶R⁷SO₂NR¹¹, CR⁶R⁷CONR¹¹ or CR⁶R⁷CH₂NR¹¹; provided that when n=0 and AB is linked by a heteroatom to piperidine, R³ is optionally substituted (C₁₋₄)alkyl or ethenyl;
   provided that R⁶ and R⁷, and R⁸ and R⁹ are not both optionally substituted hydroxy or amino;
   and wherein:
   each of R⁶, R⁷ , R⁸ and R⁹ is independently selected from: H; (C₁₋₆)alkoxy; (C₁₋₆)alkylthio; halo; trifluoromethyl; azido; (C₁₋₆)alkyl; (C₂₋₆)alkenyl; (C₁₋₆)alkoxycarbonyl; (C ₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C₁₋₆)alkylsulphonyl; (C₂₋₆)alkenylsulphonyl; or (C₁₋₆)aminosulphonyl wherein the amino group is optionally substituted by (C ₁₋₆)alkyl or (C₂₋₆)alkenyl;
   or R⁶ and R⁸ together represent a bond and R⁷ and R⁹ are as above defined;
   and each R¹¹ is independently H; trifluoromethyl; (C ₁₋₆)alkyl; (C₂₋₆)alkenyl; (C₁₋₆)alkoxycarbonyl; (C ₁₋₆)alkylcarbonyl; or aminocarbonyl wherein the amino group is optionally substituted by (C ₁₋₆)alkoxycarbonyl, (C ₁₋₆)alkylcarbonyl, (C₂₋₆)alkenyloxycarbonyl, (C₂₋₆)alkenylcarbonyl, (C ₁₋₆)alkyl or (C₂₋₆)alkenyl and optionally further substituted by (C₁₋₆)alkyl or (C₂₋₆)alkenyl;
   or where one of R³ or R³₁ and R⁶, R⁷, R⁸ or R⁹ contains a carboxy group and the other contains a hydroxy or amino group they may together form a cyclic ester or amide linkage or where R³ or R³₁ contains a carboxy group and A or B is NH they may be condensed to form a cyclic amide.

The invention also provides the use of a compound of formula (I) or a pharmaceutically acceptable derivative thereof in the manufacture of a medicament for use in the treatment of bacterial infections in mammals.

The invention also provides a pharmaceutical composition, in particular for use in the treatment of bacterial infections in mammals, comprising a compound of formula (I), or a pharmaceutically acceptable derivative thereof, and a pharmaceutically acceptable carrier.

In one aspect, R¹ and R^{1a} are other than trifluoromethoxy or hydroxy substituted by (C₁₋₄)alkoxy(C₁₋₄)alkyl, and R³ is other than trifluoromethyl.

Preferably one of Z¹, Z², Z³, Z⁴ and Z⁵ is N, one is CR^{1a} and the remainder are CH, or one of Z¹, Z², Z³, Z⁴ and Z⁵ is CR^{1a} and the remainder are CH.

Preferably Z⁵ is CH or N, Z³ is CH or CF and Z¹, Z² and Z⁴ are each CH, or Z¹ is N, Z³ is CH or CF and Z², Z⁴ and Z⁵ are each CH.

When R¹ or R^{1a} is substituted alkoxy it is preferably (C₂₋₆)alkoxy substitituted by optionally N-substituted amino, guanidino, or amidino, or (C₁₋₆)alkoxy substituted by piperidyl. Suitable examples of R¹ alkoxy include methoxy, trifluoromethoxy, n-propyloxy, i-butyloxy, aminoethyloxy, aminopropyloxy, aminobutyloxy, aminopentyloxy, guanidinopropyloxy, piperidin-4-ylmethyloxy, phthalimido pentyloxy or 2-aminocarbonylprop-2-oxy. Preferably R¹ is methoxy, amino(C₃₋₅)alkyloxy, guanidino(C₃₋₅)alkyloxy, piperidyl(C₃₋₅)alkyloxy, nitro or fluoro.

Preferably R¹ and R^{1a} are independently methoxy, amino(C₃₋₅)alkyloxy, guanidino(C₃₋₅)alkyloxy, piperidyl(C₃₋₅)alkyloxy, nitro or fluoro; more preferably methoxy, amino(C₃₋₅)alkyloxy or guanidino(C₃₋₅)alkyloxy. Preferably R^{1a} is H or F. Most preferably R¹ is methoxy and R^{1a} is H or when Z³ is CR^{1a} it may be C-F.

When Z⁵ is CR^{1a}, R^{1a} is preferably hydrogen, cyano, hydroxymethyl or carboxy, most preferably hydrogen.

Preferably n is 0.

In a first aspect, R³ is optionally substituted hydroxy, optionally substituted amino, halogen or, trifluoromethyl.

In a second aspect, R³ is other than optionally substituted hydroxy, optionally substituted amino, halogen or, trifluoromethyl and n is 0.

Preferably R³ is OH, NH₂, (C₁₋₄)alkyl, (C₁₋₄)alkoxy, (C₁₋₄)alkoxy(C₁₋₄)alkoxy, carboxy, cyano, optionally substituted aminocarbonyl, (C₁₋₄)alkylcarbonyloxy, fluoro, trifluoromethyl or CH₂OH

Examples of R³ include OH, NH₂, methyl, methoxy, methoxymethoxy, carboxy, cyano, aminocarbonyl, acetoxy, fluoro, trifluoromethyl or CH₂OH.

R³ is preferably OH, NH₂, methyl or CH₂OH.

Preferred examples of R³₁ include hydrogen; (C₁₋₄)alkyl substituted with carboxy, optionally substituted hydroxy, optionally substituted aminocarbonyl, optionally substituted amino or (C₁₋₄)alkoxycarbonyl; or (C₂₋₄)alkenyl substituted with (C₁₋₄)alkoxycarbonyl or carboxy. More preferred groups for R³₁ are hydrogen, carboxymethyl, hydroxymethyl, aminocarbonylmethyl or aminocarbonyl, most preferably hydrogen. When R³₁ is in the 3 position preferred examples also include optionally substituted hydroxy or amino.

R³ and R³₁ halogen is preferably fluoro.

When R³ or R³₁ and R⁶, R⁷, R⁸ or R⁹ together form a cyclic ester or amide linkage, it is preferred that the resulting ring is 5-7 membered. It is further preferred that the group A or B which does not form the ester or amide linkage is CH₂.

When A is CH(OH) the R-stereochemistry is preferred.

Preferably A is NH, NCH₃, CH₂, CHOH, CH(NH₂), C(Me)(OH) or CH(Me).

Preferably B is CH₂ or CO.

Preferably n=0.

Most preferably:
n is 0 and either A is CHOH or CH₂ and B is CH₂ or A is NH and B is CO.

Preferably R¹¹ is hydrogen or (C₁₋₄)alkyl e.g. methyl, more preferably hydrogen.

When V is C=NOR¹⁹, R¹⁹ is preferably H.

The group -U-V- is preferably -(CH₂)₂-, CH₂CH(OH), CH₂C=NOH or CH₂CO.

Preferably R⁵₂ is an aromatic heterocyclic ring (A) having 8-11 ring atoms including 2-4 heteroatoms of which at least one is N or NR¹³ in which preferably Y² contains 2-3 heteroatoms, one of which is S and 1-2 are N, with one N bonded to X³.

Alternatively and preferably the heterocyclic ring (A) has ring (a) aromatic selected from optionally substituted benzo and pyrido and ring (b) non-aromatic and Y² has 3-5 atoms, more preferably 4 atoms, including a heteroatom bonded to X⁵ selected from O, S or NR¹³, where R¹³ is other than hydrogen, and NHCO bonded via N to X³, or O bonded to X³. The ring (a) preferably contains aromatic nitrogen, and more preferably ring (a) is pyridine. Examples of rings (A) include optionally substituted:

### (a) and (b) aromatic

1H-pytrolo[2,3-b]-pyridin-2-yl, 1H-pyrrolo[3,2-b]-pyridin-2-yl, 3H-imidazo[4,5-b]-pyrid-2-yl, 3H-quinazolin-4-one-2-yl, benzimidazol-2-yl, benzo[1,2,3]-thiadiazol-5-yl, benzo[1,2,5]-oxadiazol-5-yl, benzofur-2-yl, benzothiazol-2-yl, benzo[b]thiophen-2-yl, benzoxazol-2-yl, chromen-4-one-3-yl, imidazo[1,2-a]pyridin-2-yl, imidazo-[1,2-a]-pyrimidin-2-yl, indol-2-yl, indol-6-yl, isoquinolin-3-yl, [1,8]-naphthyridine-3-yl, oxazolo[4,5-b]-pyridin-2-yl, quinolin-2-yl, quinolin-3-yl, quinoxalin-2-yl, indan-2-yl, naphtbalen-2-yl, 1,3-dioxo-isoindol-2yl, benzimidazol-2-yl, benzothiophen-2-yl, 1H-benzotriazol-5-yl, 1H-indol-5-yl, 3H-benzooxazol-2-one-6-yl, 3H-benzooxazol-2-thione-6-yl, 3H-benzothiazol-2-one-5-yl, 3H-quinazolin-4-one-2-yl, 3H-quinazolin-4-one-6-yl, 4-oxo-4H-pyrido[1,2-a]pyrimidin-3-yl, benzo[1,2,3]thiadiazol-6-yl, benzo[1,2,5]thiadiazol-5-yl, benzo[1,4]oxazin-2-one-3-yl, benzothiazol-5-yl, benzothiazol-6-yl, cinnolin-3-yl, imidazo[1,2-a]pyridazin-2-yl, imidazo[1,2-b]pyridazin-2-yl, pyrazolo[1,5-a]pyrazin-2-yl, pyrazolo[1,5-a]pyridin-2-yl, pyrazolo[1,5-a]pyrimidin-6-yl, pyrazolo[5,1-c][1,2,4]triazin-3-yl, pyrido[1,2-a]pyrimidin-4-one-2-yl, pyrido[1,2- , a]pynmidin-4-one-3-yl, quinazolin-2-yl, quinoxalin-6-yl, thiazolo[3,2-a]pyrimidin-5-one-7-yl, thiazolo[5,4-b]pyridin-2-yl, thieno[3,2-b]pyridin-6-yl, thiazolo[5,4-b]pyndin-6-yl, 4-oxo-4H-pyrido[1,2-a]pyrimidin-2-yl, 1-oxo-1,2-dihydro-isoquinolin-3-yl, thiazolo[4,5-b]pyridin-5-yl, [1,2,3]thiadiazolo[5,4-b]pyridin-6-yl, 2H-isoquinolin-1-one-3-yl

### (a) is non aromatic

(2S)-2,3-dihydro-1H-indol-2-yl, (2S)-2,3-dihydro-benzo[1,4]dioxine-2-yl, 3-(R,S)-3,4-dihydro-2H-benzo[1,4]thiazin-3-yl, 3-(R)-2,3-dihydro-[1,4]dioxino[2,3-b]pyridin-3-yl, 3-(S)-(2,3-dihydro-[1,4]dioxino[2,3-b]pyridin-3-yl, 3-substituted-3H-quinazolin-4-one-2-yl, 2,3-dihydro-benzo[1,4]dioxan-2-yl, 1-oxo-1,3,4,5-tetrahydrobenzo[c]azepin-2-yl.

### (b) is non aromatic

1,1,3-trioxo-1,2,3,4-tetrahydro-1 *l*⁶-benzo[1,4] thiazin-6-yl, benzo[1,3]dioxol-5-yl, 2,3-dihydro-benzo[1,4]dioxin-6-yl, 2-oxo-2,3-dihydro-benzooxazol-6-yl, 4H-benzo[1,4]oxazin-3-one-6-yl (3-oxo-3,4-dihydro-2H-benzo[1,4]oxazin-6-yl), 4H-benzo[1,4]thiazin-3-one-6-yl (3-oxo-3,4-dihydro-2H-benzo[1,4]thiazin-6-yl), 4H-benzo[1,4]oxazin-3-one-7-yl, 4-oxo-2,3,4,5-tetrahydro-benzo[b][1,4]thiazepine-7-yl, 5-oxo-2,3-dihydro-5H-thiazolo[3,2-a]pyrimidin-6-yl, benzo[1,3]dioxol-5-yl, 2-oxo-2,3-dihydro-1H-pyrido[2,3-b][1,4]thiazin-7-yl, 2,3-dihydro-[1,4]dioxino[2,3-b]pyridin-6-yl, 2,3-dihydro-[1,4]dioxino[2,3-c]pyridin-7-yl, 2,3-dihydro-[1,4]dioxino[2,3-b]pyridin-7-yl, 3,4-dihydro-2H-benzo[1,4]oxazin-6-yl, 2-oxo-2,3-dihydro-1H-pyrido[3,4-b][1,4]thiazin-7-yl, 3-oxo-3,4-dihydro-2H-pyrido[3,2-b][1,4]thiazin-6-yl, 6,7-dihydro-[1,4]dioxino[2,3-d]pyrimidin-2-yl, 3-oxo-3,4-dihydro-2H-pyrido[3,2-b][1,4]oxazin-6-yl, 2-oxo-2,3-dihydro-1H-pyrido[3,4-b][1,4]oxazin-7-yl, 2-oxo-2,3-dihydro-1H-pyrido[2,3-b][1,4]oxazin-7-yl, 6-oxo-6,7-dihydro-5H-8-thia-1,2,5-triaza-naphthalen-3-yl, 3,4-dihydro-2H-benzo[1,4]thiazin-6-yl, 3-substituted-3H-benzooxazol-2-one-6-yl, 3-substituted-3H-benzooxazol-2-thione-6-yl, 3-substituted-3H-benzothiazol-2-one-6-yl, 2,3-dihydro-1H-pyrido[2,3-b][1,4]thiazin-7-yl, 3,4-dihydro-1H-quinolin-2-one-7-yl, 3,4-dihydro-1H-quinoxalin-2-one-7-yl, 6,7-dihydro-4H-pyrazolo[1,5-a]pyrimidin-5-one-2-yl, 5,6,7,8-tetrahydro-[1,8]naphthyridin-2-yl, 2-oxo-3,4-dihydro-1*H*-[1,8]naphthyridin-6-yl, 3,4-dihydro-2H-pyrido[3,2-b][1,4]thiazin-6-yl.

R¹³ is preferably H if in ring (a) or in addition (C₁₋₄)alkyl such as methyl or isopropyl when in ring (b). More preferably, in ring (b) R¹³ is H when NR¹³ is bonded to X³ and (C ₁₋₄)alkyl when NR¹³ is bonded to X⁵.

R¹⁴ and R¹⁵ are preferably independently selected from hydrogen, halo, hydroxy, (C₁₋₄) alkyl, (C₁₋₄)alkoxy, trifluoromethoxy, nitro, cyano, aryl(C₁₋₄)alkoxy and (C₁₋₄)alkylsulphonyl.

More preferably R¹⁵ is hydrogen.

More preferably each R¹⁴ is selected from hydrogen, chloro, fluoro, hydroxy, methyl, methoxy, trifluoromethoxy, benzyloxy, nitro, cyano and methylsulphonyl. Most preferably R¹⁴ is selected from hydrogen, hydroxy, fluorine or nitro. Preferably 0-3 groups R¹⁴ are substituents other than hydrogen.

Most preferably CR¹⁴R¹⁵ is CH₂.

Most preferred groups R⁵₂ include:
[1,2,3]thiadiazolo[5,4-b]pyridin-6-yl
1H-Pyrrolo[2,3-b]pyridin-2-yl
2,3-Dihydro-[1,4]dioxino[2,3-b]pyridin-6-yl
2,3-Dihydro-[1,4]dioxino[2,3-b]pyridin-7-yl
2,3-Dihydro-[1,4]dioxino[2,3-c]pyridin-7-yl
2,3-dihydro-benzo[1,4]dioxin-6-yl
2-oxo-2,3-dihydro-1H-pyrido[2,3-b][1,4]oxazin-7-yl
2-oxo-2,3-dihydro-1H-pyrido[2,3-b]thiazin-7-yl
3,4-dihydro-2H-benzo[1,4]oxazin-6-yl
3-Methyl-2-oxo-2,3-dihydro-benzooxazol-6-yl
3-oxo-3,4-dihydro-2H-benzo[1,4]oxazin-6-yl
3-oxo-3,4-dihydro-2H-pyrido[2,3-b]oxazin-6-yl
3-oxo-3,4-dihydro-2H-benzo[1,4]thiazin-6-yl (4H-benzo[1,4] thiazin-3-one-6-yl)
4-oxo-4H-pyrido[1,2-a]pyrimidin-2-yl
6-nitro-benzo[1,3]dioxol-5-yl
7-fluoro-3-oxo-3,4-dihydro-2H-benzo[1,4] oxazin-6-yl
8-Hydroxy-1-oxo-1,2-dihydro-isoquinolin-3-yl
8-hydroxyquinolin-2-yl
benzo[1,2,3]thiadiazol-5-yl
benzo[1,2,5]thiadiazol-5-yl
benzothiazol-5-yl
thiazolo-[5,4-b]pyridin-6-yl
3,4-dihydro-2H-benzo[1,4]oxazin-6-yl
3-oxo-3,4-dihydro-2*H*-pyrido[3,2-*b*][1,4]thiazin-6-yl
7-chloro-3-oxo-3,4-dihydro-2*H*-pyrido[3,2-*b*][1,4]thiazin-6-yl
7-fluoro-3-oxo-3,4-dihydro-2*H*-pyrido[3,2-*b*][1,4]thiazin-6-yl
2-oxo-2,3-dihydro-1*H*-pyrido[3,4-*b*][1,4]thiazin-7-yl
especially
3-oxo-3,4-dihydro-2H-benzo[1,4]thiazin-6-yl (4H-benzo[1,4]thiazin-3-one-6-yl)
3,4-dihydro-2H-benzo[1,4]oxazin-6-yl
3-oxo-3,4-dihydro-2*H*-pyrido[3,2-*b*][1,4]thiazin-6-yl.

When used herein, the term "alkyl" includes groups having straight and branched chains, for instance, methyl, ethyl, n-propyl, iso-propyl, n-butyl, iso-butyl, sec-butyl, t-butyl, pentyl and hexyl. The term 'alkenyl' should be interpreted accordingly.

Halo or halogen includes fluoro, chloro, bromo and iodo.

Haloalkyl moieties include 1-3 halogen atoms.

Unless otherwise defined, the term 'heterocyclic' as used herein includes aromatic and non-aromatic, single and fused, rings suitably containing up to four hetero-atoms in each ring selected from oxygen, nitrogen and sulphur, which rings may be unsubstituted or C-substituted by, for example, up to three groups selected from (C₁₋₄)alkylthio; halo; carboxy(C ₁₋₄)alkyl; halo(C ₁₋₄)alkoxy; halo(C ₁₋₄)alkyl; (C ₁₋₄)alkyl; (C₂₋₄)alkenyl; (C₁₋₄)alkoxycarbonyl; formyl; (C ₁₋₄)alkylcarbonyl; (C₂₋₄)alkenyloxycarbonyl; (C₂₋₄)alkenylcarbonyl; (C ₁₋₄)alkylcarbonyloxy; (C₁₋₄)alkoxycarbonyl(C₁₋₄)alkyl; hydroxy; hydroxy(C ₁₋₄)alkyl; mercapto(C ₁₋₄)alkyl; (C ₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C ₁₋₄)alkyl or (C₂₋₄)alkenyl; optionally substituted aryl, aryl(C ₁₋₄)alkyl or aryl(C ₁₋₄)alkoxy and oxo groups. Each heterocyclic ring suitably has from 4 to 7, preferably 5 or 6, ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

Where an amino group forms part of a single or fused non-aromatic heterocyclic ring as defined above suitable optional substituents in such substituted amino groups include H; trifluoromethyl; (C ₁₋₄)alkyl optionally substituted by hydroxy, (C₁₋₆)alkoxy, (C ₁₋₆)alkylthio, halo or trifluoromethyl; (C₂₋₄)alkenyl; aryl; aryl (C ₁₋₄)alkyl; (C₁₋₄)alkoxycarbonyl; (C ₁₋₄)alkylcarbonyl; formyl; (C ₁₋₆)alkylsulphonyl; or aminocarbonyl wherein the amino group is optionally substituted by (C ₁₋₄)alkoxycarbonyl, (C₁₋₄)alkylcarbonyl, (C₂₋₄)alkenyloxycarbonyl, (C₂₋₄)alkenylcarbonyl, (C ₁₋₄)alkyl or (C₂₋₄)alkenyl and optionally further substituted by (C ₁₋₄)alkyl or (C₂₋₄)alkenyl.

When used herein the term 'aryl', includes phenyl and naphthyl, each optionally substituted with up to five, preferably up to three, groups selected from(C ₁₋₄)alkylthio; halo; carboxy(C ₁₋₄)alkyl; halo(C ₁₋₄)alkoxy; halo(C ₁₋₄)alkyl; (C ₁₋₄)alkyl; (C₂₋₄)alkenyl; (C ₁₋₄)alkoxycarbonyl; formyl; (C ₁₋₄)alkylcarbonyl; (C₂₋₄)alkenyloxycarbonyl; (C₂₋₄)alkenylearbonyl; (C ₁₋₄)alkylcarbonyloxy; (C ₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano, carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; phenyl, phenyl(C₁₋₄)alkyl or phenyl(C₁₋₄)alkoxy.

The term 'acyl' includes (C ₁₋₆)alkoxycarbonyl, formyl or (C ₁₋₆) alkylcarbonyl groups.

Some of the compounds of this invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Since the compounds of formula (I) are intended for use in pharmaceutical compositions it will readily be understood that they are each provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 59% of a compound of the formula (I) or pharmaceutically acceptable derivative thereof.

Pharmaceutically acceptable derivatives of the above-mentioned compounds of formula (I) include the free base form or their acid addition or quaternary ammonium salts, for example their salts with mineral acids e.g. hydrochloric, hydrobromic, sulphuric, nitric or phosphoric acids, or organic acids, e.g. acetic, fumaric, succinic, maleic, citric, benzoic, p-toluenesulphonic, methanesulphonic, naphthalenesulphonic acid or tartaric acids. Compounds of formula (I) may also be prepared as the N-oxide. Compounds of formula (I) having a free carboxy group may also be prepared as an *in vivo* hydrolysable ester. The invention extends to all such derivatives.

Examples of suitable pharmaceutically acceptable *in vivo* hydrolysable ester-forming groups include those forming esters which break down readily in the human body to leave the parent acid or its salt. Suitable groups of this type include those of part formulae (i), (ii), (iii), (iv) and (v):

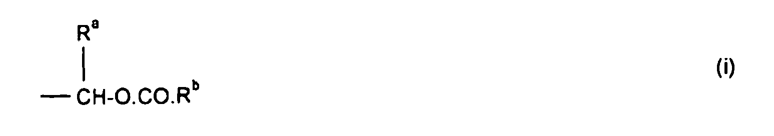

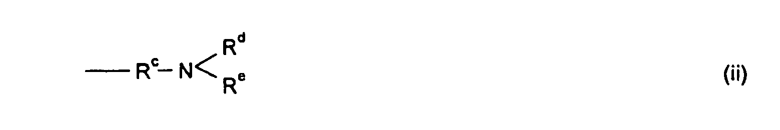

-CH₂-OR^{f} (iii)

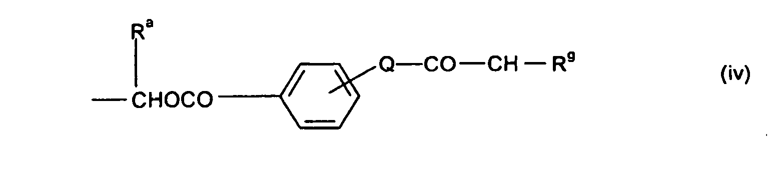

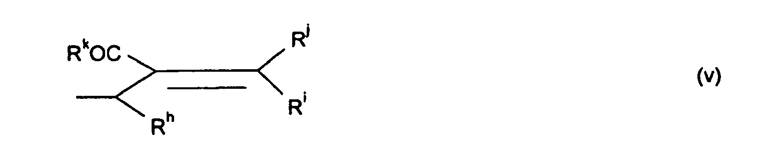

wherein R^{a} is hydrogen, (C₁₋₆) alkyl, (C₃₋₇) cycloalkyl, methyl, or phenyl, R^{b} is (C₁₋₆) alkyl, (C₁₋₆) alkoxy, phenyl, benzyl, (C₃₋₇) cycloalkyl, (C₃₋₇) cycloalkyloxy, (C₁₋₆) alkyl (C₃₋₇) cycloalkyl, 1-amino (C₁₋₆) alkyl, or 1-(C₁₋₆ alkyl)amino (C₁₋₆) alkyl; or R^{a} and R^{b} together form a 1,2-phenylene group optionally substituted by one or two methoxy groups; R^{c} represents (C₁₋₆) alkylene optionally substituted with a methyl or ethyl group and R^{d} and R^{e} independently represent (C₁₋₆) alkyl; R^{f} represents (C₁₋₆) alkyl; R^{g} represents hydrogen or phenyl optionally substituted by up to three groups selected from halogen, (C₁₋₆) alkyl, or (C₁₋₆) alkoxy; Q is oxygen or NH; R^{h} is hydrogen or (C₁₋₆) alkyl; Rⁱ is hydrogen, (C₁₋₆) alkyl optionally substituted by halogen, (C₂₋₆) alkenyl, (C₁₋₆) alkoxycarbonyl, aryl or heteroaryl; or R^{h} and Rⁱ together form (C₁₋₆) alkylene; R^{j} represents hydrogen, (C₁₋₆) alkyl or (C₁₋₆) alkoxycarbonyl; and R^{k} represents (C₁₋₈) alkyl, (C₁₋₈) alkoxy, (C₁₋₆) alkoxy(C₁₋₆)alkoxy or aryl.

Examples of suitable *in vivo* hydrolysable ester groups include, for example, acyloxy(C ₁₋₆)alkyl groups such as acetoxymethyl, pivaloyloxymethyl, α-acetoxyethyl, α-pivaloyloxyethyl, 1-(cyclohexylcarbonyloxy)prop-1-yl, and
(1-aminoethyl)carbonyloxymethyl; (C ₁₋₆)alkoxycarbonyloxy(C₁₋₆)alkyl groups, such as ethoxycarbonyloxymethyl, α-ethoxycarbonyloxyethyl and propoxycarbonyloxyethyl; di(C ₁₋₆)alkylamino(C ₁₋₆)alkyl especially di(C ₁₋₄)alkylamino(C₁₋₄)alkyl groups such as dimethylaminomethyl, dimethylaminoethyl, diethylaminomethyl or diethylaminoethyl;
2-((C₁₋₆)alkoxycarbonyl)-2-(C₂₋₆)alkenyl groups such as
2-(isobutoxycarbonyl)pent-2-enyl and 2-(ethoxycarbonyl)but-2-enyl; lactone groups such as phthalidyl and dimethoxyphthalidyl.

A further suitable pharmaceutically acceptable *in vivo* hydrolysable ester-forming group is that of the formula: 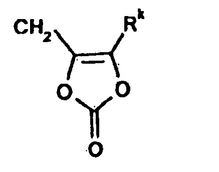 wherein R^{k} is hydrogen, C₁₋₆ alkyl or phenyl.

R is preferably hydrogen.

Certain of the above-mentioned compounds of formula (I) may exist in the form of optical isomers, e.g. diastereoisomers and mixtures of isomers in all ratios, e.g. racemic mixtures. The invention includes all such forms, in particular the pure isomeric forms. For examples the invention includes compound in which an A-B group CH(OH)-CH₂ is in either isomeric configuration the *R*-isomer is preferred. The different isomeric forms may be separated or resolved one from the other by conventional methods, or any given isomer may be obtained by conventional synthetic methods or by stereospecific or asymmetric syntheses.

In a further aspect of the invention there is provided a process for preparing compounds of formula (I), or a pharmaceutically acceptable derivative thereof, which process comprises reacting a compound of formula (IV) with a compound of formula (V): 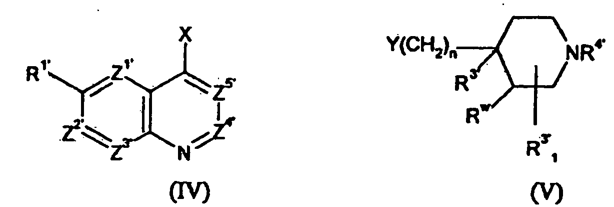 wherein n is as defined in formula (I); Z^{1'}, Z^{2'}, Z^{3'}, Z^{4'}, Z^{5'}, R^{1'}, R^{3'}₁ and R^{3'} are Z¹, Z², Z³, Z⁴, Z⁵, R¹, R³₁ and R³ as defined in formula (I) or groups convertible thereto and R^{W} is hydrogen or R^{W} and R^{3'} represent a bond;
and X and Y may be the following combinations:
(i) one of X and Y is CO₂R^{y} and the other is CH₂CO₂R^{x};
(ii) X is CHR⁶R⁷ and Y is C(=O)R⁹;
(iii) X is CR⁷=PR^{z}₃ and Y is C(=O)R⁹;
(iv) X is C(=O)R⁷ and Y is CR⁹=PR^{z}₃;
(v) one of Y and X is COW and the other is NHR ^{11'} or NR^{11'} COW;
(vi) X is NHR^{11'} and Y is C(=O)R⁸ or X is C(=O)R⁶ and Y is NHR^{11'};
(vii) X is NHR^{11'} and Y is CR⁸R⁹W;
(viii) X is W or OH and Y is CH₂OH;
(ix) X is NHR^{11'} and Y is SO₂W;
(x) one of X and Y is (CH₂)ₚ-W and the other is (CH₂)_{q}NHR^{11'}, (CH₂)_{q}OH, (CH₂)_{q}SH or (CH₂)_{q}SCOR^{x} where p+q=1;
(xi) one of X and Y is OH and the other is -CH=N₂;
(xii) X is NCO and Y is OH or NH₂;
(xiii) X is CR⁶R⁷SO₂W, A'COW, CR⁶=CH₂ or oxirane and Y is NHR^{11'};
(xiv) X is W and Y is CONHR ^{11'} or OCONH₂;
(xv) X is W and Y is -CH=CH₂;
(xvi) X is W and Y is -C≡CH (followed by hydrogenation of the intermediate -C≡C-group);
(xvii) X is NHR^{11'} and together Y and R³ are O and n = 1;
in which W is a leaving group, e.g. halo, methanesulphonyloxy, trifluoromethanesulphonyloxy or imidazolyl; R^{x} and R^{y} are (C₁₋₆)alkyl; R^{z} is aryl or (C₁₋₆)alkyl; A' and NR^{11'} are A and NR¹¹ as defined in formula (I), or groups convertible thereto; and oxirane is: 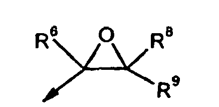 wherein R⁶, R⁸ and R⁹ are as defined in formula (I);
and thereafter optionally or as necessary converting A', Z^{1'}, Z^{2'}, Z^{3'}, Z^{4'}, Z^{5'}, R^{1'}, R^{3'}, R^{3'}₁, R^{4'} and NR^{11'} to A, Z¹, Z², Z³, Z⁴, Z⁵, R¹, R³, R³₁, R⁴ and NR^{11'}; converting R^{3'} and R^{w} when a bond into R³ and hydrogen or R³₁; converting A-B to other A-B, interconverting R¹, R³, R³₁ and/or R⁴, and/or forming a pharmaceutically acceptable derivative thereof.
Process variant (i) initially produces compounds of formula (I) wherein A-B is CO-CH₂ or CH₂-CO.
Process variant (ii) initially produces compounds of formula (I) wherein A-B is CR⁶R⁷-CR⁹OH.
Process variant (iii) and (iv) initially produce compounds of formula (I) wherein A-B is CR⁷=CR⁹.
Process variant (v) initially produces compounds of formula (I) where A-B is CO-NR¹¹ or NR¹¹-CO.
Process variant (vi) initially produces compounds of formula (I) wherein A-B is NR ¹¹-CHR⁸. or CHR⁶-NHR¹¹.
Process variant (vii) initially produces compounds of formula (I) wherein A-B is NR^{11'}-CR⁸R⁹.
Process variant (viii) initially produces compounds of formula (I) wherein A-B is O-CH₂.
Process variant (ix) initially produces compounds where AB is NR¹¹SO₂.
Process variant (x) initially produces compounds of formula (I) wherein one of A and B is CH₂ and the other is NHR¹¹, O or S.
Process variant (xi) initially produces compounds of formula (I) wherein A-B is OCH₂ or CH₂O.
Process variant (xii) initially produces compounds where AB is NH-CO-NH or NH-CO-O.
Process variant (xiii) initially produces compounds where n is 0 and AB is CR⁶R⁷SO₂NR¹¹, A'-CONR¹¹ or CR⁶R⁷CR⁸R⁹NR¹¹.
Process variant (xiv) initially produces compounds of formula (I) where A-B is NR^{11'}-CO or NH-CO-O.
Process variants (xv) and (xvi) initially produce compounds of formula (I) where A-B is -CH=CH- or -C≡C-, which may be hydrogenated to -CH=CH- or -CH₂CH₂-.
Process variant (xvii) initially produces compounds of formula (I) where A-B is NR^{11'}-CH₂.

In process variants (v) and (xiii) (second variant) the reaction is a standard amide or urea formation reaction involving e.g.:
1. Activation of a carboxylic acid (e.g. to an acid chloride, mixed anhydride, active ester, O-acyl-isourea or other species), and treatment with an amine (Ogliaruso, M.A.; Wolfe, J.F. in *The Chemistry of Functional Groups (Ed. Patai, S.) Suppl. B: The Chemistry of Acid Derivatives, Pt. 1* (John Wiley and Sons, 1979), pp 442-8; Beckwith, A.L.J. in *The Chemistry of Functional Groups (Ed. Patai, S.) Suppl. B: The Chemistry of Amides (Ed. Zabricky, J.)* (John Wiley and Sons, 1970), p 73 ff. The acid and amine are preferably reacted in the presence of an activating agent such as 1-(dimethylaminopropyl)-3-ethylcarbodiimide hydrochloride (EDC) or 1-hydroxybenzotriazole (HOBT) or O-(7-azabenzotriazol-1-yl)-*N,N,N',N'*-tetramethyluronium hexafluorophosphate (HATU); or
2. The specific methods of:
   a. *in situ* conversion of an acid into the amine component by a modified Curtius reaction procedure (Shioiri, T., Murata, M., Hamada, Y., *Chem. Pharm. Bull.* 1987, 35, 2698)
   b. *in situ* conversion of the acid component into the acid chloride under neutral conditions (Villeneuve, G. B.; Chan, T. H., *Tetrahedron. Lett.* 1997, 38, 6489).

A' may be, for example. protected hydroxymethylene.

The process variant (xiii) (third variant) is a standard addition reaction using methods well known to those skilled in the art. The process is preferably carried out in a polar organic solvent e.g. acetonitrile in the presence of an organic base e.g. triethylamine.

In process variant (xiii) (fourth variant) the coupling may be effected in acetonitrile at room temperature in the presence of one equivalent of lithium perchlorate as catalyst (general method of J.E. Chateauneuf *et al, J Org. Chem.,* 56, 5939-5942, 1991) or more preferably with ytterbium triflate in dichloromethane. In some cases an elevated temperature such as 40-70°C may be beneficial. Alternatively, the compound of formula (V) may be treated with a base, such as one equivalent of butyl lithium, and the resulting salt reacted with the oxirane in an inert solvent such as tetrahydrofuran, preferably at an elevated temperature such as 80°C. Use of a chiral epoxide will afford single diastereomers. Alternatively, mixtures of diastereomers may be separated by preparative HPLC or by conventional resolution through crystallisation of salts formed from chiral acids.

The process variant (xii) is a standard urea or carbamate formation reaction from the reaction of an isocyanate with an amine or alcohol and is conducted by methods well known to those skilled in the art (for example see March, J; *Advanced Organic Chemistry, Edition 3* (John Wiley and Sons, 1985), p802-3). The process is preferably carried out in a polar solvent such as N,N-dimethylformamide.

In process variant (i) the process is two step: firstly a condensation using a base, preferably sodium hydride or alkoxide, sodamide, alkyl lithium or lithium dialkylamide, preferably in an aprotic solvent e.g. ether, THF or benzene; secondly, hydrolysis using an inorganic acid, preferably HCl in aqueous organic solvent at 0-100°C. Analogous routes are described in DE330945, EP31753, EP53964 and H. Sargent, J. Am. Chem. Soc. **68**, 2688-2692 (1946). Similar Claisen methodology is described in Soszko et. al., Pr.Kom.Mat. Przyr.Poznan.Tow.Przyj.Nauk., (1962), 10, 15.

In process variant (ii) the reaction is carried out in the presence of a base, preferably organometallic or metal hydride e.g. NaH, lithium diisopropylamide or NaOEt, preferably in an aprotic solvent, preferably THF, ether or benzene at -78 to 25°C (analogous process in Gutswiller et al. (1978) J. Am. Chem. Soc. 100, 576).

In process variants (iii) and (iv) if a base is used it is preferably NaH, KH, an alkyl lithium e.g. BuLi, a metal alkoxide e.g. NaOEt, sodamide or lithium dialkylamide e.g.diisopropylamide. An analogous method is described in US 3989691 and M.Gates et. al. (1970) J. Amer.Chem.Soc., 92, 205, as well as Taylor et al. (1972) JACS 94, 6218.

In process variant (vi) the reaction is a standard reductive alkylation using, e.g., sodium borohydride or sodium triacetoxyborohydride (Gribble, G. W. in *Encyclopedia of Reagents for Organic Synthesis (Ed Paquette, L. A.)* (John Wiley and Sons, 1995), p 4649).

The process variant (vii) is a standard alkylation reaction well known to those skilled in the art, for example where an alcohol or amine is treated with an alkyl halide in the presence of a base (for example see March, J; *Advanced Organic Chemistry, Edition 3* (John Wiley and Sons, 1985), p364-366 and p342-343). The process is preferably carried out in a polar solvent such as N,N-dimethylformamide

In process variant (xiii) (first variant) the reaction is a standard sulphonamide formation reaction well known to those skilled in the art This may be e.g. the reaction of a sulphonyl halide with an amine.

In process variant (viii) where X is W such as halogen, methanesulphonyloxy or trifluoromethanesulphonyloxy, the hydroxy group in Y is preferably converted to an OM group where M is an alkali metal by treatment of an alcohol with a base. The base is preferably inorganic such as NaH, lithium diisopropylamide or sodium. Where X is OH, the hydroxy group in Y is activated under Mitsunobu conditions (Fletcher et.al. *J* Chem Soc. (1995), 623). Alternatively the X=O and Y=CH₂OH groups can be reacted directly by activation with 1,3-dicyclohexylcarbodiimide (DCC) (Chem. Berichte 1962, 95, 2997 or Angewandte Chemie 1963 75,377).

In process variant (ix) the reaction is conducted in the presence of an organic base such as triethylamine or pyridine such as described by Fuhrman et.al., J. Amer. Chem. Soc.; **67**, 1245,1945. The X=NR¹¹' or Y=SO₂W intermediates can be formed from the requisite amine e.g. by reaction with SO₂Cl₂ analogously to the procedure described by the same authors Fuhrman et.al., J. Amer. Chem. Soc.; **67**, 1245, 1945.

In process variant (x) where one of X and Y contains NHR¹¹ the leaving group W is halogen and the reaction is a standard amine formation reaction such as direct alkylation described in (Malpass, J. R., in *Comprehensive Organic Chemistry,* Vol. 2 (Ed. Sutherland, I. O.), p 4 ff.) or aromatic nucleophilic displacement reactions (see references cited in *Comprehensive Organic Chemistry,* Vol. 6, p 946-947 (reaction index); Smith, D. M. in *Comprehensive Organic Chemistry,* Vol. 4 (Ed. Sammes, P. G.) p 20 ff.). This is analogous to the methods described in GB 1177849.

In process variant (x) where one of X and Y contains OH or SH, this is preferably converted to an OM or SM group where M is an alkali metal by treatment of an alcohol, thiol or thioacetate with a base. The base is preferably inorganic such as NaH, lithium diisopropylamide or sodium, or, for SH, metal alkoxide such as sodium methoxide. The X/Y group containing the thioacetate SCOR^{x} is prepared by treatment of an alcohol or alkyl halide with thioacetic acid or a salt thereof under Mitsunobu conditions. The leaving group V is a halogen. The reaction may be carried out as described in Chapman et.al., J. Chem Soc., (1956),1563, Gilligan et. al., J. Med. Chem., (1992), **35**, 4344, Aloup et. al., J. Med. Chem. (1987), **30**, 24, Gilman et al., J.A.C.S. (1949), 71, 3667 and Clinton et al., J.A.C.S. (1948), **70**, 491, Barluenga et al., J. Org. Chem. (1987) **52**, 5190. Alternatively where X is OH and Y is CH₂W, W is a hydroxy group activated under Mitsunobu conditions (Fletcher et.al. J Chem Soc. (1995), 623).

In process variant (xi) the reaction is as described in den Hertzog et. al., recl.Trav. Chim. Pays-Bas, (1950),**69**, 700.

In process variant (xiv) the leaving group W is preferably chloro or trifluoromethylsulphonyl and the reaction is the palladium catalysed process known as the "Buchwald" reaction (J. Yin and S. L. Buchwald, Org.Lett., 2000, 2, 1101).

In process variant (xv) the leaving group W is preferably trifluoromethylsulphonyl and the reaction is known as the "Heck" reaction (Heck, *Comp. Org. Syn.* Vol 4 Ch4.3 p.833).

In process variant (xvi) coupling of the acetylene compound (V) with the compound (IV), where the leaving group X is preferably trifluoromethylsulphonyl or halide, is accomplished using standard Pd-mediated chemistry, for example using Pd(Ph₃P)₂Cl₂ as the catalyst along with the addition of CuI in a mixture of triethylamine and dimethylformamide. Hydrogenation of the intermediate -C≡C- group is carried out conventionally over a suitable catalyst eg Pd/C, either partially to -CH=CH- or fully to - CH₂-CH₂-.

In process variant (xvii) the amine X is NHR^{11'} isdeprotonated with a strong base such as sodium hydride or potassium tert-butoxide followed by reaction with an oxirane (V), where Y and R³ together are oxygen and n = 1.

Reduction of a carbonyl group A or B to CHOH can be readily accomplished using reducing agents well known to those skilled in the art, e.g. sodium borohydride in aqueous ethanol or lithium aluminium hydride in ethereal solution. This is analogous to methods described in EP53964, US384556 and J. Gutzwiller *et al, J. Amer. Chem. Soc.,* 1978, 100, 576.

The carbonyl group A or B may be reduced to CH₂ by treatment with a reducing agent such as hydrazine in ethylene glycol, at e.g. 130-160°C, in the presence of potassium hydroxide.

Reaction of a carbonyl group A or B with an organometallic reagent yields a group where R⁶ or R⁸ is OH and R⁷ or R⁹ is alkyl.

A hydroxy group on A or B may be oxidised to a carbonyl group by oxidants well known to those skilled in the art, for example, manganese dioxide, pyridinium chlorochromate or pyridinium dichromate.

A hydroxyalkyl A-B group CHR⁷CR⁹OH or CR⁷(OH)CHR⁹ may be dehydrated to give the group CR⁷=CR⁹ by treatment with an acid anhydride such as acetic anhydride.

Methods for conversion of CR⁷=CR⁹ by reduction to CHR⁷CHR⁹ are well known to those skilled in the art, for example using hydrogenation over palladium on carbon as catalyst. Methods for conversion of CR⁷=CR⁹ to give the A-B group CR⁷(OH)CHR⁹ or CHR⁷CR⁹OH are well known to those skilled in the art for example by epoxidation and subsequent reduction by metal hydrides, hydration, hydroboration or oxymercuration.

An amide carbonyl group may be reduced to the corresponding amine using a reducing agent such as lithium aluminium hydride.

A hydroxy group in A or B may be converted to azido by activation and displacement e.g. under Mitsunobu conditions using hydrazoic acid or by treatment with diphenylphosphorylazide and base, and the azido group in turn may be reduced to amino by hydrogenation.

Examples of groups Z^{1'}, Z^{2'}, Z^{3'}, Z^{4'}, Z^{5'}, are CR^{1a'} where R^{1a'} is a group convertible to R^{1a}. Z^{1'}, Z^{2'}, Z^{3'}, Z^{4'} and Z^{5'} are preferably Z¹, Z², Z³, Z⁴ and Z⁵.

R^{1a'} and R^{1'} are preferably R^{1a} and R¹. R^{1'} is preferably methoxy. R^{3'} is preferably R³ or with R^{w} is a bond, R^{3'}₁ is R³₁ or more preferably hydrogen, vinyl, alkoxycarbonyl or carboxy. R^{4'} is R⁴ or more preferably H or an N-protecting group such as t-butoxycarbonyl, benzyloxycarbonyl or 9-fluorenylmethyloxycarbonyl.

Conversions of R^{1a'}, R^{1'}, R^{3'} and R^{4'} and interconversions of R^{1a}, R¹, R³₁, R³ and R⁴ are conventional. In compounds which contain an optionally substituted hydroxy group, suitable conventional hydroxy protecting groups which may be removed without disrupting the remainder of the molecule include acyl and alkylsilyl groups. N protecting groups are removed by conventional methods.

For example R^{1'} methoxy is convertible to R^{1'} hydroxy by treatment with lithium and diphenylphosphine (general method described in Ireland et. al. (1973) J.Amer.Chem.Soc.,7829) or HBr. Alkylation of the hydroxy group with a suitable alkyl derivative bearing a leaving group such as halide and a protected amino, piperidyl, amidino or guanidino group or group convertible thereto, yields, after conversion/deprotection, R¹ alkoxy substituted by optionally N-substituted amino, piperidyl, guanidino or amidino.

R³ or R³₁ alkenyl is convertible to hydroxyalkyl by hydroboration using a suitable reagent such as 9-borabicyclo[3.3.1]nonane, epoxidation and reduction or oxymercuration.

R³ or R³₁ 1,2-dihydroxyalkyl can be prepared from R^{3'} alkenyl using osmium tetroxide or other reagents well known to those skilled in the art (see Advanced Organic Chemistry *(Ed. March, J)* (John Wiley and Sons, 1985), p 732-737 and refs. cited therein) or epoxidation followed by hydrolysis (see Advanced Organic Chemistry *(Ed. March, J.)* (John Wiley and Sons, 1985), p 332,333 and refs. cited therein).

R³ or R³₁ vinyl can be chain extended by standard homologation e.g by conversion to hydroxyethyl followed by oxidation to the aldehyde which is then subjected to a Wittig reaction.

Opening an epoxide R^{3'} or R^{3'}₁ group with cyanide anion yields a CH(OH)-CH₂CN group.

Opening an epoxide-containing R^{3'} or R^{3'}₁ group with azide anion yields an α-hydroxy azide derivative which can be reduced to the α-hydroxy amine. Conversion of the α-hydroxy amine to a carbamate is followed by ring closure with base to give the 2-oxo-oxazolidinyl containing R³ or R³₁ group.

Substituents on R³ or R³₁ alkyl or alkenyl may be interconverted by conventional methods, for example hydroxy may be derivatised by esterification, acylation or etherification. Hydroxy groups may be converted to halogen, thiol, alkylthio, azido, alkylcarbonyl, amino, aminocarbonyl, oxo, alkylsulphonyl, alkenylsulphonyl or aminosulphonyl by conversion to a leaving group and substitution by the required group, hydrolysis or oxidation as appropriate or reaction with an activated acid, isocyanate or alkoxyisocyanate. Primary and secondary hydroxy groups can be oxidised to an aldehyde or ketone respectively and alkyated with a suitable agent such as an organometallic reagent to give a secondary or tertiary alcohol as appropriate. A carboxylate group may be converted to an hydroxymethyl group by reduction of an ester of this acid with a suitable reducing agent such as lithium aluminium hydride.

Substituted 2-oxo-oxazolidinyl containing R³ or R³₁ groups may be prepared from the corresponding aldehyde by conventional reaction with a glycine anion equivalent, followed by cyclisation of the resulting amino alcohol (M Grauert et al, Ann Chem (1985) 1817, Rozenberg et al, Angew Chem Int Ed Engl (1994) 33(1) 91). The resulting 2-oxo-oxazolidinyl group contains a carboxy group which can be converted to other R¹⁰ groups by standard procedures.

Carboxy groups within R³ or R³₁ may be prepared by Jones' oxidation of the corresponding alcohols CH₂OH using chromic acid and sulphuric acid in water/methanol (E.R.H. Jones et al, J.C.S. 1946,39). Other oxidising agents may be used for this transformation such as sodium periodate catalysed by ruthenium trichloride (G.F.Tutwiler *et al,* J.Med.Chem., 1987, *30*(6), 1094), chromium trioxide-pyridine (G. Just *et al,* Synth. Commun. 1979, *9*(7), 613), potassium permanganate (D.E.Reedich *et al,* J. Org. Chem.,1985,*50*(19),3535, and pyridinium chlorochromate (D. Askin *et al,* Tetrahedron Letters, 1988,29(3), 277.

The carboxy group may alternatively be formed in a two stage process, with an initial oxidation of the alcohol to the corresponding aldehyde using for instance dimethyl sulphoxide activated with oxalyl chloride (N.Cohen *et al,* J. Am. Chem. Soc., 1983, 105, 3661) or dicyclohexylcarbodiimide (R.M.Wengler, Angew. Chem. Int. Ed. Eng., 1985, 24(2), 77), or oxidation with tetrapropylammonium perruthenate (Ley *et al,* J. Chem.Soc. Chem Commun., 1987, 1625). The aldehyde may then be separately oxidised to the corresponding acid using oxidising agents such as silver (II) oxide (R.Grigg *et al,* J. Chem. Soc. Perkinl, 1983, 1929), potassium permanganate (A.Zurcher, Helv. Chim. Acta., 1987, 70 (7), 1937), sodium periodate catalysed by ruthenium trichloride (T.Sakata *et al,* Bull. Chem. Soc. Jpn., 1988,61(6), 2025), pyridinium chlorochromate (R.S.Reddy *et al,* Synth. Commun., 1988, 18(51), 545) or chromium trioxide (R.M.Coates *et al,* J. Am. Chem. Soc.,1982,104, 2198).

An R³ or R³₁ CO₂H group may also be prepared from oxidative cleavage of the corresponding diol, CH(OH)CH₂OH, using sodium periodate catalysed by ruthenium trichloride with an acetonitrile-carbontetrachloride-water solvent system (V.S.Martin *et al,* Tetrahedron Letters, 1988, *29*(22), 2701).

R³ or R³_{1.} groups containing a cyano or carboxy group may also be prepared by conversion of an alcohol to a suitable leaving group such as the corresponding tosylate by reaction with para-toluenesulphonyl chloride (M.R.Bell, J. Med. Chem., 1970, 13, 389), or the iodide using triphenylphosphine, iodine, and imidazole (G. Lange, Synth. Commun., 1990, 20, 1473). The second stage is the displacement of the leaving group with cyanide anion (LA.Paquette et al, J. Org. Chem.,1979, 44 (25), 4603; P.A.Grieco et al, J. Org. Chem.,1988, 53 (16), 3658). Finally acidic hydrolysis of the nitrile group gives the desired acids (H.Rosemeyer et al, Heterocycles, 1985, 23 (10), 2669). The hydrolysis may also be carried out with base e.g. potassium hydroxide (H.Rapoport, J. Org. Chem.,1958, 23, 248) or enzymatically (T. Beard et al, Tetrahedron Asymmetry, 1993, 4 (6), 1085).

R³ fluoro groups may be prepared from the compound where R³ is hydroxy by reaction with a fluorodeoxygenating reagent such as DAST (diethylaminosulfur trifluoride).

Other functional groups in R³ or R³₁ may be obtained by conventional conversions of carboxy or cyano groups.

Tetrazoles are conveniently prepared by reaction of sodium azide with the cyano group (e.g. F. Thomas et al, Bioorg. Med. Chem. Lett., 1996, 6 (6), 631; K.Kubo et al, J. Med. Chem., 1993, 36,2182) or by reaction of azidotri-n-butyl stannane with the cyano group followed by acidic hydrolysis ( P.L.Ornstein, J. Org. Chem., 1994, *59,* 7682 and J. Med. Chem, 1996, *39* (11), 2219).

The 3-hydroxy-3-cyclobutene-1,2-dion-4-yl group (e.g. R.M.Soll, Bioorg. Med. Chem. Lett., 1993,3 (4), 757 and W. A. Kinney, J. Med. Chem., 1992, 35 (25), 4720) can be prepared by the following sequence: (1) a compound where R³ is (CH₂)ₙCHO (n = 0,1,2) is treated with triethylamine, carbon tetrabromide/triphenylphosphine to give initially (CH₂)ₙCH=CBr₂; (2) dehydrobromination of this intermediate to give the corresponding bromoethyne derivative (CH₂)ₙC≡CBr (for this 2 stage sequence see D. Grandjean et al, Tetrahedron Letters, 1994, *35* (21), 3529); (3) palladium-catalysed coupling of the bromoethyne with 4-(1-methylethoxy)3-(tri-n-butylstannyl)cyclobut-3-ene-1,2-dione (Liebeskind et al, J. Org. Chem., 1990, *55,* 5359); (4) reduction of the ethyne moity to -CH2CH2- under standard conditions of hydrogen and palladium on charcoal catalysis(see Howard et al, Tetrahedron, 1980, *36,* 171); and finally (5) acidic hydrolysis of the methylethoxyester to generate the corresponding 3-hydroxy-3-cyclobutene-1,2-dione group (R.M.Soll, Bioorg. Med. Chem. Lett., 1993, *3* (4), 757).

The Letzazol-5-ylaminocarbonyl group may be prepared from the corresponding carboxylic acid and 2-aminotetrazole by dehydration with standard peptide coupling agents such as 1,1'-carbonyldiimidazole (P. L. Ornstein et al, J. Med Chem, 1996, *39* (11), 2232).

The alkyl- and alkenyl-sulphonylcarboxamides are similarly prepared from the corresponding carboxylic acid and the alkyl- or alkenyl-sulphonamide by dehydration with standard peptide coupling agents such as 1,1'-carbonyldiimidazole (P. L. Ornstein et al, J.Med.Chem., 1996,39 (11), 2232).

The hydroxamic acid groups are prepared from the corresponding acids by standard amide coupling reactions eg N. R. Patel et al, Tetrahedron, 1987, *43* (22), 5375

2,4-Thiazolidinedione groups may prepared from the aldehydes by condensation with 2,4-thiazolidinedione and subsequent removal of the olefinic double bond by hydrogenation.

The preparation of 5-oxo-1,2,4-oxadiazoles from nitriles is decribed by Y.Kohara et al, Bioorg. Med. Chem. Lett., 1995, *5*(17), 1903.

1,2,4-Trizol-5-yl groups may be prepared from the corresponding nitrile by reaction with an alcohol under acid conditions followed by reaction with hydrazine and then an R¹⁰-substituted activated carboxylic acid (see JB Polya in 'Comprehensive Heterocyclic Chemistry' Edition 1 p762, Ed AR Katritzky and CW Rees, Pergamon Press, Oxford 1984 and J.J. Ares et al, J. Heterocyclic Chem., 1991, 28(5), 1197).

Novel intermediates of formula (VI): 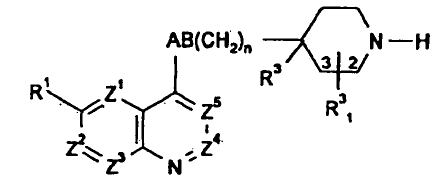 where the variables are as defined in formula (I), also form part of the invention.

The piperidine NH is converted to NR⁴ by conventional means such as amide or sulphonamide formation, or by alkylation, for example by reaction with a vinyl derivative and heating in an alcohol such as ethanol containing an acid such as acetic acid, with an alkyl halide or other reactive alkyl derivative in the presence of base, acylation/reduction or reductive alkylation with an aldehyde. For example where R⁴ is a group ―U-V-R⁵₂ an acyl derivative R⁵₂V'COW or R⁵₂V'SO₂W may be used for compounds where U is CO or SO₂ or, where U and V are CH₂, by reaction with a vinyl derivative R⁵₂-CH=CH₂, for example by heating in an alcohol such as ethanol containing an acid such as acetic acid, alkylation with an alkyl halide R⁵₂-V'-CH₂-halide or alkyl derivative R⁵₂-V'-CH₂-W in the presence of base, acylation/reduction or reductive alkylation with an aldehyde R⁵-V'-CHO where V' is V or a group convertible thereto such as a dimethyl acetal or 1,3-dithiane. Such groups are deprotected by conventional means eg dimethyl acetal by hydrolysis using dilute acid in tetrahydrofuran, and 1,3-dithiane using HgCl₂ in aqueous acetonitrile (Marshall J. A. et al., J. Org. Chem. 34, 4188 (1969)) or AgNO₂ and I₂ in tetrahydrofuran (Nishide K. et al., Heterocycles 44(1) 393 (1997)).

Where V is a group CR¹⁷R¹⁸ and R¹⁸ is H this may be obtained by reduction of a V = CO group followed by derivatisation of the resulting R¹⁷ = OH group as necessary.

A group V' is CO may be converted to an oxime or oxime derivative C=NOR¹⁹ by reaction with hydroxylamine or substituted hydroxylamine NH₂OR¹⁹ by heating in the presence of sodium acetate, for instance in an alcohol solvent such as methanol. The E- and Z-oximes may be separated by crystallisation or by conventional chromatography.

Where one of R³ and R⁶, R⁷, R⁸ or R⁹ contains a carboxy group and the other contains a hydroxy or amino group they may together form a cyclic ester or amide linkage. This linkage may form spontaneously during coupling of the compounds of formulae (IV) and (V) or in the presence of standard peptide coupling agents.

It will be appreciated that under certain circumstances interconvertions may interfere, for example, hydroxy groups in A or B and the piperidine NH will require protection e.g. as a carboxy- or silyl-ester group for hydroxy and as an acyl derivative for nitrogen, during conversion of R^{1a'}, R^{1'}, R^{2'}, R^{3'}, R^{3'}₁ or R^{4'}, or during the coupling of the compounds of formulae (IV) and (V).

Compounds of formulae (IV) and (V) are known compounds, (see for example Smith *et al, J. Amer. Chem. Soc.,* 1946, 68,1301) or prepared analogously, see for example the references cited above.

Compounds of formula (IV) where X is CR⁶R⁷SO₂W may be prepared by a route analogous to that of Ahmed E1 Hadri *et al, J. Heterocyclic Chem.,* 1993, 30(3), 631. Thus compounds of formula (IV) where X is CH₂SO₂OH may be prepared by reacting the corresponding 4-methyl compound with N-bromosuccinimide, followed by treatment with sodium sulfite. The leaving group W may be converted to another leaving group W, e.g. a halogen group, by conventional methods.

The isocyanate of formula (IV) may be prepared conventionally from a 4-amino derivative such as 4-amino-quinoline, and phosgene, or phosgene equivalent (eg triphosgene) or it may be prepared more conveniently from a 4-carboxylic acid by a "one-pot" Curtius Reaction with diphenyl phosphoryl azide (DPPA) [see T. Shiori et al. *Chem. Pharm. Bull.* **35**, 2698-2704 (1987)].

The 4-amino derivatives are commercially available or may be prepared by conventional procedures from a corresponding 4-chloro or 4-trifluoromethanesulphonate derivative by treatment with ammonia (O.G. Backeberg et. al., J. Chem Soc., 381, 1942) or propylamine hydrochloride (R, Radinov et. al., Synthesis, 886, 1986).

4-Alkenyl compounds of formula (IV) may be prepared by conventional procedures from a corresponding 4-halogeno-derivative by e.g. a Heck synthesis as described in e.g. *Organic Reactions,* 1982, 27, 345.

4-Halogeno derivatives of compounds of formula (IV) are commercially available, or may be prepared by methods known to those skilled in the art. A 4-chloroquinoline is prepared from the corresponding quinolin-4-one by reaction with phosphorus oxychloride (POCl₃) or phosphorus pentachloride, PCl_{5.} A 4-chloroquiuazoline is prepared from the corresponding quinazolin-4-one by reaction with phosphorus oxychloride (POCl₃) or phosphorus pentachloride, PCl₅. A quinazolinone and quinazolines may be prepared by standard routes as described by T.A. Williamson in *Heterocyclic Compounds,* 6, 324 (1957) Ed. R.C. Elderfield.

Activated carboxy derivatives X=A'COW of formula (IV) may be prepared from X=A'CO₂H derivatives in turn prepared from CO₂H derivatives by conventional methods such as homologation.

4-Carboxy derivatives of compounds of formula (TV) are commercially available or may be prepared by conventional procedures for preparation of carboxy heteroaromatics well known to those skilled in the art. For example, quinazolines may be prepared by standard routes as described by T.A. Williamson in *Heterocyclic Compounds,* 6, 324 (1957) Ed. R.C. Elderfield. These 4-carboxy derivatives may be activated by conventional means, e.g. by conversion to an acyl halide or anhydride.

4-Carboxy derivatives such as esters may be reduced to hydroxymethyl derivatives with for example lithium aluminium hydride. Reaction with mesyl chloride and triethylamine would give the mesylate derivative. A diazo compound (X is -CH=N₂) may be prepared from the 4-carboxaldehyde via the tosyl hydrazone. The 4-carboxaldehyde may be obtained from the acid by standard procedures well known to those skilled in the art.

A 4-oxirane derivative of compounds of formula (IV) is conveniently prepared from the 4-carboxylic acid by first conversion to the acid chloride with oxalyl chloride and then reaction with trimethylsilyldiazomethane to give the diazoketone derivative. Subsequent reaction with 5M hydrochloric acid gives the chloromethylketone. Reduction with sodium borohydride in aqueous methanol gives the chlorohydrin which undergoes ring closure to afford the epoxide on treatment with base, e.g. potassium hydroxide in ethanol-tetrahydrofiuan.

Alternatively and preferably, 4-oxirane derivatives can be prepared from bromomethyl ketones which can be obtained from 4-hydroxy compounds by other routes well known to those skilled in the art. For example, hydroxy compounds can be converted to the corresponding 4-trifluoromethanesulphonates by reaction with trifluoromethanesulphonic anhydride under standard conditions (see K. Ritter, Synthesis, 1993, 735). Conversion into the corresponding butyloxyvinyl ethers can be achieved by a Heck reaction with butyl vinyl ether under palladium catalysis according to the procedure of W. Cabri *et al,* J. Org. Chem, 1992, 57 (5), 1481. (Alternatively, the equivalent intermediates can be attained by Stille coupling of the trifluoromethanesulphonates or the analogous chloro derivatives with (1-ethoxyvinyl)tributyl tin (T. R. Kelly, J. Org. Chem., 1996, **61,** 4623).) The alkyloxyvinyl ethers are then converted into the corresponding bromomethylketones by treatment with N-bromosuccinimide in aqueous tetrahydrofuran in a similar manner to the procedures of J. F. W. Keana, J. Org. Chem., 1983, **48,** 3621 and T. R. Kelly, J. Org. Chem., 1996, **61,** 4623.

The 4-hydroxyderivatives can be prepared from an aminoaromatic by reaction with methylpropiolate and subsequent cyclisation, analogous to the method described in N. E. Heindel et al, J. Het. Chem., 1969, **6,** 77. For example, 5-amino-2-methoxy pyridine can be converted to 4-hydroxy-6-methoxy-[1,5]naphthyridine using this method.

If a chiral reducing agent such as (+) or (-)-B-chlorodiisopinocamphenylborane ['DIP-cMoride'] is substituted for sodium borohydride, the prochiral chloromethylketone is converted into the chiral chlorohydrin with ee values generally 85-95% [see C. Bolm et *al, Chem. Ber.* 125, 1169-1190, (1992)]. Recrystallisation of the chiral epoxide gives material in the mother liquor with enhanced optical purity (typically ee 95%).

The (*R)*-epoxide, when reacted with an amine derivative gives ethanolamine compounds as single diastereomers with (*R*)-stereochemistry at the benzylic position.

Alternatively, the epoxide may be prepared from the 4-carboxaldehyde by a Wittig approach using trimethylsulfonium iodide [see G.A. Epling and K-Y Lin, *J. Het. Chem.,* 1987, 24, 853-857], or by epoxidation of a 4-vinyl derivative.

Pyridazines may be prepared by routes analogous to those described in Comprehensive Heterocyclic Chemistry, Volume 3, Ed A.J. Boulton and A. McKillop and napthyridines may be prepared by routes analogous to those described in Comprehensive Heterocyclic Chemistry, Volume 2, Ed A.J. Boulton and A. McKillop.

4-Hydroxy-1,5-naphthyridines can be prepared from 3-aminopyridine derivatives by reaction with diethyl ethoxymethylene malonate to produce the 4-hydroxy-3-carboxylic acid ester derivative with subsequent hydrolysis to the acid, followed by thermal decarboxylation in quinoline (as for example described for 4-Hydroxy-[1,5]naphthyridine-3-carboxylic acid, J. T. Adams *et al., J.Amer. Chem.Soc.,* 1946, **68**, 1317). A 4-hydroxy-[1,5]naphthyridine can be converted to the 4-chloro derivative by heating in phosphorus oxychloride, or to the 4-methanesulphonyloxy or 4-trifluoromethanesulphonyloxy derivative by reaction with methanesulphonyl chloride or trifluoromethanesulphonic anhydride, respectively, in the presence of an organic base. A 4-amino 1,5-naphthyridine can be obtained from the 4-chloro, 4-methanesulphonyloxy or 4-trifluoromethanesulphonyloxy derivative by reaction with n-propylamine in pyridine.

Similarly, 6-methoxy-1,5-naphthyridine derivatives can be prepared from 3-amino-6-methoxypyridine.

1,5-Naphthyridines may be prepared by other methods well known to those skilled in the art (for examples see P.A. Lowe in "Comprehensive Heterocyclic Chemistry" Volume 2, p581-627, Ed A.R. Katritzky and C.W. Rees, Pergamon Press, Oxford, 1984).

The 4-hydroxy and 4-amino-cinnolines may be prepared following methods well known to those skilled in the art [see A.R. Osborn and K. Schofield, *J. Chem. Soc.* 2100 (1955)]. For example, a 2-aminoacetophenone is diazotised with sodium nitrite and acid to produce the 4-hydroxycinnoline with conversion to chloro and amino derivatives as described for 1,5-naphthyridines.

The compounds of formula (V) are either commercially available or may be prepared by conventional methods.

For compounds of formula (V), where Y is NHR^{11'}suitable amines may be prepared from a 4-substituted piperidine or tetrahydropyridine acid or alcohol. In a first instance, an N-protected piperidine or tetrahydropyridine containing an acid bearing substituent, can undergo a Curtius rearrangement and the intermediate isocyanate can be converted to a carbamate by reaction with an alcohol. Conversion to the amine may be achieved by standard methods well known to those skilled in the art used for amine protecting group removal. For example, an acid substituted N-protected piperidine or tetrahydropyridine can undergo a Curtius rearrangement e.g. on treatment with diphenylphosphoryl azide and heating, and the intermediate isocyanate reacts in the presence of 2-trimethylsilylethanol to give the trimethylsilylethylcarbamate (T.L. Capson & C.D. Poulter, *Tetrahedron Lett.,* 1984, 25, 3515). This undergoes cleavage on treatment with tetrabutylammonium fluoride to give the 4-amine substituted N-protected compound of formula (V). Alternatively, an acid group (CH₂)ₙ₋₁CO₂H may be converted to (CH2)ₙNHR¹¹ by reaction with an activating agent such as isobutyl chloroformate followed by an amine R^{11'}NH₂ and the resulting amide reduced with a reducing agent such as LiAlH₄.

In a second instance, an N-protected piperidine or tetrahydropyridine containing an alcohol bearing substituent undergoes a Mitsunobu reaction (for example as reviewed in Mitsunobu, *Synthesis,* (1981), 1), for example with succinimide in the presence of diethyl azodicarboxylate and triphenylphosphine to give the phthalimidoethylpiperidyl- or tetrahydropiperidyl amine. Removal of the phthaloyl group, for example by treatment with methylhydrazine, gives the amine of formula (V).

Compounds of formula (V) where n=1 may be prepared from the compound where n=0 by homologation eg starting from a compound of formula (V) where Y=CO₂H.

Compounds of formula (V) where Y is a carboxylic acid or derivative, substituted by R³ at the 4-position may be prepared from a 4-keto derivative via a cyanohydrin reaction with sodium cyanide/hydrochloric acid, or more conveniently using trimethylsilylcyanide in dichloromethane, followed by hydrolysis by heating in conc. hydrochloric acid to give the α-hydroxy acid (Compound(V), Y=CO₂H, n=0, R^{3'} =OH).

The same keto-derivative could undergo a Wittig reaction with Ph₃PCH=CO₂Me to give the αβ-unsaturated carboxylic ester MeO₂C-CH=C<Ring, which could be epoxidised (eg meta-chloroperbenzoic acid) to give the α,β-epoxy-ester. Alternatively this could be formed directly from the keto-derivative via a glycidic ester condensation with an α-halogeno-ester. Base hydrolysis would afford the α,β-epoxy-carboxylic acid, which on reduction (eg lithium triethylborohydride - see J. Miklefield et al J. Amer. Chem. Soc. 117, 1153-1154 (1995) or hydrogenation over platinum oxide (see Artamonow Zh.Obshch.Khim. 28 1355-1359 (1958)) would afford the β-hydroxy acid (Compound (V) Y=CO₂H, n=1, R^{3'} =OH). Alternatively a Reformatsky reaction with the keto-derivative and an α-bromocarboxylic acid ester and zinc, followed by acid hydrolysis would afford the β-hydroxycarboxylic acid directly.

The keto-derivative could also undergo a Strecker type synthesis via a Bucherer-Bergs procedure (potassium cyanide/ammonium carbonate) [see T. Scott Yokum et al. Tetrahedron Letters, 38, 4013-4016 (1997)] to give the α-amino-carboxylic acid (Compound (V) Y=CO₂H, n=0, R^{3'} =NH₂).

The keto derivative (R^{4'} is a protecting group) can be reacted with the lithium anion derived from trimethylsilyl acetylene and n-butyl lithium followed by removal of the trimethylsilyl group by conventional methods eg using K₂CO₃ and MeOH, to give the acetylenic alcohol (Compound (V) Y = C≡CH, n=0, R^{3'} = OH).

Compounds of formula (V) with Y = C≡CH, n=0, R^{3'} = F) may be prepared from the protected keto derivative using the general method of van Niel and Collins, *J. Med.* Chem., 1999, 42, 2087-2104.

Compounds of formula (V) with a Y = -CONHR^{11'} group may be prepared from the corresponding nitrile by partial hydrolysis with with concentrated mineral acid at ambient temperature, such as concentrated hydrochloric acid (M. Brown *et al,* J. Med. Chem., 1999,**42**, (9), 1537) or with concentrated sulphuric acid (F. Macias *et al* Tetrahedron, 2000, **56**, (21), 3409).

Compounds of formula (V) with a Y = -OCONH₂ group may be prepared from the corresponding alcohol by reaction with phosgene followed by ammonia.

Compounds with Y = CO₂H may be converted to a -CONH₂ group by conversion to an 'active ester' for example by reaction with N-hydroxysuccinimide to give the succinimide ester, which on reaction with ammonia gives the amide -CONH₂.

Compounds of formula (V) with a Y = -CH=CH₂ group and n=0 may be prepared by reaction of vinyl lithium or vinyl Grignard with a ketone ((V) where Y(CH₂)ₙ- and R^{3'} are together =O).

Compounds of formula (V) where R³ and R³₁ are both hydroxy and oriented trans may be prepared from a tetrahydropyridine by conversion to an epoxide (for example reaction with meta-chloroperbenzoic acid) followed by hydrolysis. The corresponding cis-diol may be prepared from the tetrahydropyridine by reaction with osmium tetroxide, preferably with a co-oxidant such as N-methyl morpholine oxide. The above epoxide may be opened with an amine or azide (followed by conversion of the azide to amino by, for example, hydrogenation) to introduce a nitrogen-substituent at position-3.

Compounds of formula (V) where R³₁ is fluoro may be prepared by reaction of the epoxide with HF/pyridine [see F. Pasqui et al *Tetrahedron,* **52**, 185-198 (1996)]. Compounds where R³ is fluoro may be prepared from the compound where R³ is hydroxy by reaction with a fluorodeoxygenating reagent such as DAST (diethylaminosulfur trifluoride).

Compounds where R³ is a carboxylic acid or derivative may be prepared from a N-protected piperidine-4,4-dicarboxylic acid diester, for example a tert-butyl, methyl diester by base hydrolysis of the methyl ester. Compounds where R³ is carboxamide may be prepared from a diester by reaction with ammonia. Compounds where R³ is a tert-butyl ester may be converted later in the synthesis to compounds where R³ is carboxylic acid , which may be further modified, eg by conversion to carboxamide, or by reduction to hydroxymethyl. N-protected piperidine-4,4-dicarboxylic diesters or 4-cyano-4-carboxylic esters may be prepared by reaction of a malonic acid diester or cyanoacetic acid ester with an intermediate (WCH₂CH₂)₂NR^{4'} where W is a leaving group eg Cl or Br, in the presence of a base eg K₂CO₃ or NaH (S. Huybrechts and GJ. Hoornaert, Synth. Commun., 1981, 11,17).

Compounds where R³ is alkyl may be prepared from compounds where R³ is hydrogen by treatment with a strong base, such as lithium diisopropylamide, and quenching with an alkyl halide, such as methyl iodide. Compounds where R³ is CF₃ may be prepared by quenching with 5-(trifluoromethyl)dibenzothiophenium trifluoromethanesulfonate. Alternatively, quenching the anion with an electrophilic fluorinating reagent such as 1-chloromethyl-4-fluoro-1,4-diazoniabicyclo[2.2,2]octane bis(tetrafluoroborate) will give compounds where R³ is fluoro.

Compounds where R³ is hydroxy or amino may be alkylated (eg with an alkyl halide such as methyl iodide) or acylated (for example with acetyl chloride) to give compounds where R³ is alkoxy, acyloxy, alkylamino or acylamino.

Compounds of formula (V) where R³₁ is in the 2-position can be prepared from 4-oxopiperidine 2-carboxylic acid by converting the 2-carboxylic acid to R³₁ and then transforming the 4-ketone to an appropriate Y(CH₂)ₙ/R³ moiety such as a hydroxycarboxylic acid (via the cyanohydrin) or aminocarboxylic acid (via a Strecker type synthesis).

Compounds of formula (V) with a 3-hydroxyl group may be prepared from a 3,4 oxirane-piperidine carboxylic acid by reaction with an amine NHR² or azide (followed by conversion of the azide to amino). [See for example K. Krajewski et al. Tetrahedron Asymmetry 10, 4591-4598 (1999)].

R⁵₂-V'-CH₂-halides and R⁵₂-V'-CH₂-W derivatives, acyl derivatives R⁵₂V'COW and R⁵₂V'SO₂W, vinyl derivatives R⁵₂-CH=CH2, alkyl derivatives R⁵-V'-CH₂-W or aldehydes R⁵₂-V'-CHO are commercially available or are prepared conventionally. The aldehydes may be prepared by partial reduction of the R⁵₂-V'-ester with lithium aluminium hydride or di-isobutylaluminium hydride or more preferably by reduction to the alcohol, with lithium aluminium hydride or sodium borohydride (see *Reductions by the Alumino- and Borohydrides in Organic Synthesis,* 2nd ed., Wiley, N.Y., 1997; *JOC,* 3197, *1984; Org. Synth. Coll.,* 102, 1990; 136, 1998; *JOC,* 4260, 1990; *TL,* 995, 1988; *JOC,* 1721, 1999; *Liebigs Ann.*/*Recl.,* 2385, 1997; *JOC,* 5486, 1987), followed by oxidation to the aldehyde with manganese (II) dioxide, or by a 'Swern' procedure (oxalyl chloride/DMSO), or by using potassium dichromate (PDC). The aldehydes may also be prepared from carboxylic acids in two stages by conversion to a mixed anhydride for example by reaction with isobutyl chloroformate followed by reduction with sodium borohydride (R. J. Alabaster et al., Synthesis, 598, 1989) to give the alcohols and then oxidation with a standard oxidising agent such as pyridinium dichromate or by homologation of the R⁵₂CHO intermediate. Acyl derivatives R⁵₂V'COW may be prepared by activation of the R⁵₂- V'-acid. R⁵₂-V'-CH₂-halides such as bromides may be prepared from the alcohol R⁵₂-V'-CH₂OH by reaction with phosphorus tribromide in DCM/triethylamine, DMF or pyridine. R⁵₂-V'-CH₂-W derivatives such as methanesulphonyl derivatives may be prepared from the alcohol R⁵₂₋V'-CH₂OH by reaction with methane sulphonyl chloride in DCM/triethylamine. R⁵₂ V'SO₂W derivatives may be prepared by a route analogous to that of Ahmed El Hadri *et al, J. Heterocyclic Chem.,* 1993, 30(3), 631. Thus compounds of formula R⁵₂CH₂SO₂OH may be prepared by reacting the corresponding R⁵₂CH₃ compound with N-bromosuccinimide, followed by treatment with sodium sulfite. The leaving group W may be converted to another leaving group W, e.g. a halogen group, by conventional methods. The R⁵₂-V'-U- derivatives may be prepared by various conventional strategies. For example the homologous aldehyde R⁵₂-CHO may be treated with trimethylsulfonium methylsulfate in basic conditions, to give an epoxide intermediate (see *Synth. Commun.,* 749, 1985) which is then treated with lewis acid, such as trifluoroboron etherate or diethyl etherate, to provide the desired aldehyde (see *JOC,* 1720, 1999). The aldehyde R⁵₂-CHO could also be treated with an appropriate phosphonium salt, such as (methoxymethyl)triphenylphosphonium chloride, in a Wittig fashion reaction. The resulting enol ether can readily be hydrolysed to homologous aldehydes (*Chem. Ber.,* 2514, 1962). R⁵₂-COW derivatives can be converted to the aldehyde R⁵₂-V'-CHO in several steps (see *JACS,* 1325, 1986). The R⁵₂COCH₂-halide derivatives may be prepared by standard methods from the R⁵₂CO₂H derivative, for example, by acid chloride formation, conversion to the alpha-diazoketone with diazomethane and reaction with a halogen acid to provide the halomethylketone. Vinyl derivatives R⁵-CH=CH₂ may be prepared from the corresponding diethylaminoethyl derivative by quaternisation with dimethyl sulphate and heating, resulting in elimination of the charged amino group. The diethylaminoethyl derivative may be prepared from another ethyl derivative eg the hydroxyethyl by conventional amine formation. Alternatively, it may be prepared from a methyl derivative by condensation with diethylamine and formaldehyde.

Vinyl compounds R⁵₂-CH=CH₂ may be prepared by olefination of an aldehyde by a Peterson-type olefination, or by a Wittig reaction, or by reaction of a hate-compound with tributyl vinyl tin in the presence of a palladium catalyst eg Pd(Ph₃P)₄.

Where R⁵₂ is an optionally substituted benzoimidazol-2-yl group, the compound of formula (V) where R^{4'} is R⁴ may be obtained by converting a R^{4'} cyanomethyl group with partial hydrolysis to give the 2-ethoxycarbonimidoylethyl group which can then be condensed with an appropriately substituted 1,2-diaminobenzene to give the required benzoimidazol-2-yl group.

R⁵₂H heterocycles are commercially available or may be prepared by conventional methods.

For example where a benzoxazinone is required, a nitrophenol may be alkylated with for example ethyl bromoacetate and the resulting nitro ester reduced with Fe in acetic acid (alternatively Zn/AcOH/HCl or H₂/Pd/C or H₂/Raney Ni). The resulting amine may undergo spontaneous cyclisation to the required benzoxazinone, or cyclisation may be induced by heating in acetic acid. Alternatively a nitrophenol may be reduced to the aminophenol, which is reacted with chloroacetyl chloride [method of X. Huang and C. Chan, *Synthesis* 851 (1994)] or ethyl bromoacetate in DMSO [method of Z. Moussavi et al. *Eur. J. Med. Chim. Ther.* **24**, 55-60 (1989)]. The same general routes can be applied to prepare benzothiazinones [See for example F. Eiden and F. Meinel, Arch. Pharm. 312, 302-312 (1979), H. Fenner and R Grauert *Liebigs. Ann. Chem.* 193-313 (1978)]. A variety of routes are available to prepare aza analogues of benzothiazinones via the key corresponding aldehydes. For instance, 2-oxo-2,3-dihydro-1*H*-pyrido[3,4-b][1,4]thiazine-7-carbaldehyde may be accessed from 5-fluoro-2-picoline (E. J. Blanz, F. A. French, J. R. DoAmaral and D. A. French, J. Med. Chem. **1970,** *13,* 1124-1130) by constructing the thiazinone ring onto the pyridyl ring then functionalising the methyl substituent. The dioxin analogue of this aza substitution pattern, 2,3-dihydm-[1,4]dioxino[2,3-c]pyridine-7-carbaldehyde is accessible from Kojic acid by aminolysis from pyrone to pyridone then annelating the dioxin ring. Other aza substitution patterns with pyridothiazin-3-one, pyridooxazin-3-one, and pyridodioxin ring systems are also accessible. Ortho-aminothiophenols may be conveniently prepared and reacted as their zinc complexes [see for example V. Taneja et al *Chem. Ind* 187 (1984)]. Benzoxazolones may be prepared from the corresponding aminophenol by reaction with carbonyl diimidazole, phosgene or triphosgene. Reaction of benzoxazolones with diphosphorus pentasulfide affords the corresponding 2-thione. Thiazines and oxazines can be prepared by reduction of the corresponding thiazinone or oxazinone with a reducing agent such as lithium aluminium hydride.

Conversions of R^{w}, R^{1a'}, R^{1'}, R^{3'}, R^{3'}₁ and R^{4'} may be carried out on the intermediates of formulae (IV) and (V) prior to their reaction to produce compounds of formula (I) in the same way as described above for conversions after their reaction.

The pharmaceutical compositions of the invention include those in a form adapted for oral, topical or parenteral use and may be used for the treatment of bacterial infection in mammals including humans.

The antibiotic compounds according to the invention may be formulated for administration in any convenient way for use in human or veterinary medicine, by analogy with other antibiotics.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) or a pharmaceutically acceptable salt or *in vivo* hydrolysable ester thereof is administered in the above-mentioned dosage range.

The compound of formula (I) may be the sole therapeutic agent in the compositions of the invention or a combination with other antibiotics or with a β-lactamase inhibitor may be employed.

Compounds of formula (I) are active against a wide range of organisms including both Gram-negative and Gram-positive organisms.

The following examples illustrate the preparation of certain compounds of formula (I) and the activity of certain compounds of formula (I) against various bacterial organisms.

### Example 1 4-Methyl-1-[2-(3-oxo-3,4-dihydro-2H-benzo[1,4]thiazin-6-yl)-ethyl]-piperidine-4-carboxylic acid (6-methoxy-[1,5]naphthyridin-4-yl)-amide oxalate

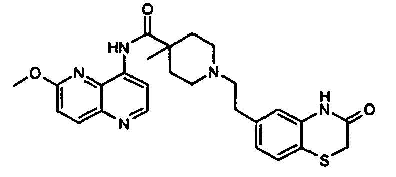

### (a) 6-Methoxy-1H-[1,5]naphthyridin-4-one

A solution of 5-amino-2-methoxypyridine (50g, 0.4 mol) in ethanol (300 ml) was treated with 2,2-dimethyl-[1,3]dioxane-4,6-dione (Meldrum's acid) (68g) and triethylorthoformate (66 ml). The mixture was heated to reflux for 2 hours, then allowed to cool. Filtration afforded the intermediate 5-[(6-methoxy-pyridin-3-ylamino)-methylene]-2,2-dimethyl-[1,3]dioxane-4,6-dione as a white solid (101.2g). A portion of this material (50g) was added to boiling Dowtherm A (300 ml) over 3 minutes under a stream of argon. The mixture was refluxed for a further 5 minutes then allowed to cool before adding to ether. Filtration and drying afforded 6-methoxy-1*H*-[1,5]naphthyridin-4-one as a white solid (24.7 g, 70%).
MS (+ve ion electrospray) m/z 177 (MH+).

### (b) 1,1,1-Trifluoro-methanesulfonic acid 6-methoxy-[1,5]naphthyridin-4-yl ester

A suspension of naphthyridone (a) (10g, 0.057 mol) in dichloromethane (200 ml) containing 2,6-lutidine (9.94 ml, 86 mmol) and 4-dimethylaminopyridine (0.07g, 5.7 mmol) was cooled in ice and treated with trifluoromethanesulfonic anhydride (10.5 ml, 63 mmol). After stirring for 2.5 hours the mixture was washed with saturated ammonium chloride solution, dried, evaporated and purified on silica eluting with dichloromethane affording an oil (13.4g, 76%).
MS (+ve ion electrospray) m/z 309 (MH+).

### (c) 6-Methoxy-[1,5]naphthyridin-4-ylamine

A mixture of triflate (b) (25g, 81.2 mmol) and propylamine hydrochloride (47g, 487 mmol) in pyridine (300 ml) was heated to reflux overnight. The mixture was evaporated to dryness, dissolved in 0.5M aqueous hydrochloric acid (400 ml) and extracted three times with dichloromethane. The aqueous phase was then basified with 40% aqueous sodium hydroxide solution and extracted twice with dichloromethane. Drying and evaporation afforded a buff coloured solid (13.0g, 90%).
MS (+ve ion electrospray) m/z 176 (MH+).

### (d) [4-(2-Hydroxy-ethyl)-2-nitro-phenylsulfanyl]-acetic acid.

A mixture of 2-(4-fluoro-3-nitro-phenyl)-ethanol(1.1 g, 6 mmol), mercaptoacetic acid (0.61 g, 6.6 mmol), potassium carbonate (2.8 g, 20 mmol) and N,N-dimethylformamide (12 ml) was heated at 70°C for 4 hours. The mixture was poured onto water (200 ml) and the aqueous phase was washed with ethyl acetate. The aqueous phase was then acidified with 1M aqueous hydrochloric acid and extracted three times with dichloromethane. The extracts were dried and evaporated to give an oil (0.92 g, 60%).
MS (+ve ion electrospray) m/z 226 (MH+).

### (e) 6-(2-Hydroxy-ethyl)-4H-benzo[1,4]thiazin-3-one.

A mixture of iron powder (1.24 g, 22 mmol) and sodium chloride (1.24g, 21 mmol) in 50% aqueous ethanol (25 ml) was heated under reflux for 15 minutes. A solution of crude (d) (0.92 g, 3.6 mmol) in ethanol (5 ml) was then added quickly and the reaction mixture was heated under reflux for 2 hours. The mixture was filtered through Kieselguhr and the solution was evaporated to dryness. The residue was dissolved in water and acidified to pH5 with 1M aqueous hydrochloric acid. The precipitate was collected by filtration, washed with water, then dried to give a white solid (0.35 g, 46%).
MS (+ve ion electrospray) m/z 210 (MH+).

### (f) 2-(3-Oxo-3,4-dihydro-2H-benzo[1,4]thiazin-6-yl)-ethanol, methanesulfonate

A solution of (e) (034g, 1.7 mmol) and triethylamine (0.25 ml, 0.19g, 1.8 mmol) in dichloromethane (5 ml) was cooled to -15°C and treated dropwise with methanesulfonyl chloride (0.14 ml,1.75 mmol). The ice bath was removed and the mixture stirred 15 hours at room temperature. The organic phase was separated, washed with water, dried over magnesium sulfate and evaporated. The crude product was chromatographed on silica eluting with ethyl acetate/heptane (2/1) to give a white solid (036 g, 75%).
MS (+ve ion electrospray) m/z 288 (MH+).

### (g) 4-Metiopiperidine-1,4-dicarboxylic acid, 1-tert-butyl ester 4-ethyl ester

A solution of piperidine-1,4-dicarboxylic acid, 1-tert-butyl ester 4-ethyl ester (3.8g, 14.8 mmol) in dry tetrahydrofuran (10 ml) was added to a solution of lithium diisopropylamide/monotetrahydrofuran complex (17.3 mmol) in tetrahydrofuran (100 ml) at -70°C. After stirring at this temperature for 0.5 hour, N,N,N'N'-tetramethylethyleoediamine (2.5 ml) and iodomethane (1.1 ml, 17.5 mmol) were added. After 2 hours stirring at -70 C, more lithium diisopropylamide/monotetrahydrofuran complex (1.5M, 9.9 ml) was added, followed after 0.5 hour by more iodomethane ( 0.94 ml). The mixture was stirred for 2.7 hours at -70°C then allowed to warm to room temperature overnight. Solvent was evaporated and the residue was partitioned between ether and aqueous ammonium chloride. The aqueous phase was re-extracted with ether, and the organic extracts were washed with aqueous citric acid, water, brine, then dried and evaporated. Chromatography on silica eluying with 20% ether/hexane gave the product (1.14g, 28%).
MS (+ve ion electrospray) m/z 172 (loss of C₅H₈O₂ from MH+).

### (h) 4-Methylpiperidine-1,4-dicarboxylic acid, 1-tert-butyl ester

A solution of the diester (g) (1.1g, 4.2 mmol) in 1,4-dioxane (5 ml) and 2M aqueous sodium hydroxide solution (4.2 ml) was stirred at room temperature for 5 days. The mixture was partially evaporated, diluted with water, acidified to pH2 (dilute hydrochloric acid) and extracted three times with ethyl acetate. The organic extracts were washed with brine, dried and evaporated to give the monoester (0.95g, 93%).
MS (+ve ion electrospray) m/z 144 (loss of C₅H₈O₂ from MH+).

### (i) 4-(6-Methoxy-[1,5]naphthyridin-4-ylcarbamoyl)-4-methylpiperidine-1-carboxylic acid tert-butyl ester.

A solution of the monoacid (h) (0.94g, 3.86 mmol) in dry dichloromethane (10 ml) was treated with N,N'-carbonyldiimidazole (0.81g, 5.0 mmol) at room temperature. After 1.75 hours, the solvent was evaporated and the residue was dissolved in dry N,N-dimethylformamide (10 ml). Amine (c) (0.68g, 3.9 mmol) was added and the mixture was heated at 100°C for 8 hours. After evaporation of solvent, the residue was dissolved in ethyl acetate and water. The aqueous phase was re-extracted twice with ethyl acetate. Organic extracts were washed with water, dried and evaporated. Chromatography on silica eluting with 2% methanol/dichloromethane gave an impure product which was rechromatographed (silica gel, 1:1 ethyl acetate/hexane) to give the amide (0.57g).
MS (+ve ion electrospray) m/z 401(MH+).

### (j) 4-(6-Methoxy-[1,5]naphthyridin-4-ylcarbamoyl)-4-methylpiperidine

The tert-butyl carbamate (i) (0.57g, 1.4 mmol) in dichloromethane (5 ml) was treated with trifluoroacetic acid (5 ml) at room temperature for 1.5 hours. The mixture was evaporated and the residue was basified to pH9 and extracted several times with 10% methanol/dichloromethane. The extract was dried and evaporated to give the amine (0.48g, 100%).
MS (+ve ion electrospray) m/z 301 (MH+).

### (k) Title compound

The piperidine (j) (0.10g, 0.33 mmol) and mesylate (f) (0.095g, 0.33 mmol) were stirred with potassium carbonate (138 mg) in N,N-dimethylformamide (2 ml) at room temperature for 3 days. Solvent was evaporated and the residue was dissolved in dichloromethane/methanol and evaporated onto silica gel, before purifying by chromatography on silica eluting with a 2-10% methanol/dichloromethane gradient to give the free base of the title compound (95 mg, 59%).
δH (CDCl₃, 250MHz): 9.88 (1H, br), 8.72 (1H, d), 8.51 (1H, d), 8.23 (1H, d), 8.17 (1H, br), 7.19 (2H, m), 6.84 (1H, dd), 6.67 (1H,d), 4.09 (3H, s), 2.41 (1H, s), 2.75 (4H, m), 2.57 (2H, m), 2.44 (2H, m), 2.31 (2H, m), 1.78 (2H, m), 1.40 (3H, s).
MS (+ve ion electrospray) m/z 492(MH+).
The free base in dichloromethane was treated with one equivalent of oxalic acid (0.1M in ether) to give the title compound as a white solid.

### Example 2 4-Amino-1-[2-(3-oxo-3,4-dihydro-2H-benzo[1,4]thiazin-6-yl)-ethyl]-piperidine-4-carboxylic acid (6-methoxy-[1,5]naphthyridin-4-yl)-amide dioxalate

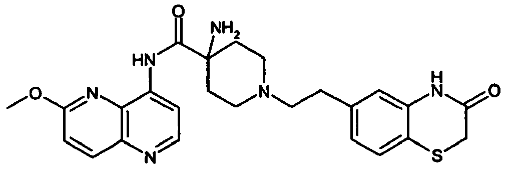

### (a) 4-Allyloxycarbonylamino-piperidine-1,4-dicarboxylic acid mono-tert-butyl ester

A solution of 4-amino-piperidine-1,4-dicarboxylic acid mono-*tert*-butyl ester (2g, 8.2 mmol) in dioxan/water (35 ml/10 ml) was treated with potassium carbonate (3.4g, 24 mmol) and allyl chloroformate (1.7 ml, 16 mmol) and stirred overnight. The mixture was concentrated and the residue partitioned between ethyl acetate and water. The aqueous extract was acidified and extracted with ethyl acetate. The organic phase was dried and evaporated to give the product as an oil (2.51g, 94%).
MS (+ve ion electrospray) m/z 328(MH+) and 228 (loss of C₅H₈O₂ from MH+)

### (b) 4-Allyloxycarbonylamino-4-(6-methoxy-[1,5]naphthyridin-4-ylcarbamoyl)-piperidine-1-carboxylic acid tert-butyl ester

A solution of acid (a) (7.5g, 23 mmol) in dichloromethane (40 ml) was treated with 1,1'-carbonyldiimidazole (4.85g). The mixture was stirred for 3 hours then evaporated to dryness. This was dissolved in N,N-dimethylformamide (22 ml) and treated with amine (1c) (3.5g, 20 mmol). The mixture was heated at 100°C for 5 hours then evaporated to dryness. The residue was partitioned between ethyl acetate and water. The organic extract was dried and evaporated. Chromatography eluting with 0-100% ethyl acetate in dichloromethane afforded the product (2.64g, 27%).
MS (+ve ion electrospray) m/z 486 (MH+) and 386 (loss of C₅H₈O₂ from MH+)

### (c) [4-(6-Methoxy[1,5]naphthyridin-4-ylcarbamoyl)-piperidine-4-ylcarbamic acid allyl ester

A solution of carbamate (b) (2.63g, 5.4 mol) in trifluoroacetic acid (150 ml)was stirred for 16 hours then evaporated to dryness. The residue was partitioned between ethyl acetate and saturated aqueous sodium bicarbonate solution. The organic extract was dried and evaporated affording an oil (2.05g, 98%).
MS (+ve ion electrospray) m/z 386 (MH+)

### (d) 4-Amino-pipendine-4-carboxylic acid (6-methoxy-[1,5]naphthyridin-4-yl)-amide

A solution of carbamate (c) (0.20g, 0.45 mmol) in tetrahydrofuran (15 ml) ws treated with tetrakis(triphenylphosphine)palladium(0) (6 mg) and tri-*n*-butyltin hydride (0.15 ml, 0.57 mmol). After 1 hour the mixture was evaporated and the residue was partitioned between chloroform and water. The aqueous phase was evaporated to give the product (0.10g, 64%).
MS (+ve ion electrospray) m/z 302 (MH+)

### (e) Title compound

A solution of piperidine (d) (0.1g, 03 mmol) in N,N-dimethylformamide (2 ml) was treated with mesylate (1f) (95 mg, 0.3 mmol) and potassium carbonate (50 mg, 0.3 mmol) and stirred at room temperature overnight. The mixture was heated at 100°C for 1 hour then evaporated to dryness. The residue was chromatographed eluting with 0-20% methanol in ethyl acetate affording the free base of the title compound (80 mg).
δH (d6-DMSO, 250MHz): 10.55 (1H, br), 8.70 (1H, d), 8.45 (1H, d), 8.25 (1H, d), 7.42 (1H, d), 7.25 (1H, d), 6.90-6.80(2H, m), 4.10 (3H, s), 3.45 (2H, s), 2.94-2.(6H, m), 2.50 (2H, m), 2.20 (2H, m), 1.78 (2H, m).
MS (+veion electrospray) m/z 493(MH+).
This was converted to the title compound (63 mg) by treatment with 2 equivalents of oxalic acid, in a similar procedure to the salt formation method of Example 1.

### Example 3 1-(2-Benzo[1,3]dioxol-5-yl-ethyl)-4-hydroxy-piperidine-4-carboxylic acid (6-methoxy-[1,5]naphthyridin-4-yl)-amide oxalate

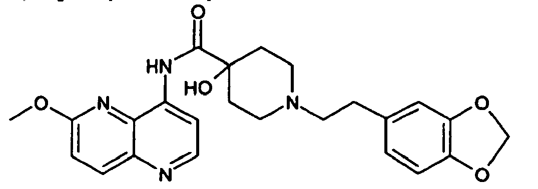

### (a) 4-Hydroxypiperidine-1,4-dicarboxylic acid, 1-tert-butyl ester

4-Hydroxypiperidine-4-carboxylic acid hydrochloride (prepared from 1-benzyl-4-piperidinone cyanohydrin by the method of German patent DE 2748257,11.50g) was dissolved in aqueous sodium hydroxide solution (2.1M, 90 ml) and 1,4-dioxane (100 ml), and treated portionwise with di-tert-butyl dicarbonate (total 30.75g), while maintaining the solution at pH9 by addition of aqueous sodium hydroxide solution.
After stirring for 24 hours, the mixture was extracted with ether, then acidified to pH2-3 and extracted several times with ether. These latter extracts were washed with brine, dried and evaporated to give the tert-butyl carbamate (12.13g, 77.5%).
MS (-ve ion electrospray) m/z 244(M-H⁻).

### (b) 4-Hydroxy-4-(6-methoxy-[1,5]naphthyridin-4-ylcarbamoyl)- piperidine-1-carboxylic acid tert-butyl ester

Method A: To a solution of the hydroxyacid (a) (2.18g, 8.9 mmol) and N-hydroxysuccinimide (1.02g, 8.9 mmol) in dry ethyl acetate (40 ml) was added 1,3-dicyclohexylcarbodiimide (1.83g, 8.9 mmol) in ethyl acetate (5 ml). The mixture was stirred overnight at room temperature, then cooled in ice, filtered and evaporated to give the succinimide ester (3.15g). This was dissolved in N,N-dimethylformamide (15 ml), amine (1c) (1.58g, 8.9 mmol) and 4-dimethylaminopyridine (0.1g) were added and the mixture was heated at 95-100°C overnight. After evaporation of solvent, the residue was dissolved in ethyl acetate and water. The organic extract was washed with water, dried and evaporated. Chromatography on silica eluting with 2% methanol/dichloromethane gave the amide (0.70g, 20%).
   MS (+ve ion electrospray) m/z 403(MH+).
Method B: A solution of the succinimide ester of Method A (15.94g, 46.6 mmol) in tetrahydrofuran (150 ml) was saturated with gaseous ammonia for 15 minutes then stirred for 1 hour at room temperature. After evaporation of solvent, the residue was dissolved in 10% methanol/dichloromethane, washed with water and brine, and evaporated to give 4-hydroxypiperidine-1,4-dicarboxylic acid, 1-tert-butyl ester 4-amide (9.77g, 86%). A mixture of this amide (0.10g, 0.41 mmol), tris(dibenzylidineacetone)dipalladium(0) (8 mg), rac-2,2'-bis(diphenylphosphino)-1,1'-binaphthyl (16 mg, 0.025 mmol) and cesium carbonate (0.17g, 0.53 mmol) in dry 1,4-dioxan (3 ml) was sonicated for 10 minutes, then triflate Example (1b) (0.13g, 0.41 mmol) was added and the mixture was heated at 80-90°C overnight. The solvent was evaporated and the residue was chromatographed on silica eluting with 0-5% methanol/dichloromethane to give the amide (0.10g, 61 %).
   MS (+ve ion electrospray) m/z 403(MH+).

### (c) 4-Hydroxy-4-(6-methoxy-[1,5]naphthyridin-4-ylcarbamoyl)-piperidine.

The tert-butyl carbamate (b) was treated with trifluoroacetic acid in dichloromethane by the method of Example (1j) to give the amine.
MS (+ve ion electrospray) m/z 303(MH+).

### (d) 2-Benzo[1,3]dioxol-5-yl-ethanol

A solution of benzo[1,3]dioxol-5-yl-acetic acid (2.5g, 13.9 mmol) in tetrahydrofuran (15 ml) under argon at -10°C was treated dropwise with borane-dimethylsulfide complex (1.45 ml, 15.3 mmol) (CAUTION - exothermic reaction). The mixture was stirred at ambient temperature for 1.5 hours The orange solution was cooled to - 5°C (ice bath) and methanol (1.4 ml) was added. After 0.5 hour at room temperature, the mixture was partitioned between ethyl acetate and saturated aqueous sodium bicarbonate solution. The organic extract was dried and evaporated to give a yellow oil (1.95g, 84%).
MS (+ve ion electrospray) m/z 167 (MH+).

### (e) 2-Benzo[1,3]dioxol-5-yl-ethanol methanesulfonate

A solution of alcohol (d) (1.90g, 11 mmol) in dichloromethane (25 ml) was treated at 0°C with triethylamine (1.9 ml, 13.7 mmol) and methanesulfonyl chloride (1.1 ml, 13.7 mmol). After 1.5 hours at room temperature the mixture was partitioned between dichloromethane and water. The organic extract was dried and evaporated to give a yellow oil (2.7g, 96%).
MS (+ve ion electrospray) m/z 245 (MH+).

### (f) Title compound.

The piperidine (c) (0.10g) was alkylated with mesylate (e) (0.081g, 0.33 mmol) using the procedure of Example (1k).
Chromatography on silica gel (5% methanol/dichloromethane) gave the title compound as free base (25%).
δH (CDCl₃, 250MHz): 10.87 (1H, br), 8.69 (1H, d), 8.53 (1H, d), 8.20 (1H, d), 7.14 (1H, d), 6.76-6.66 (3H, m), 5.93 (2H, s), 4.09 (3H, s), 2.95 (2H, m), 2.76 (2H, m), 2.64 (2H, m), 2.41 (4H, m), 1.80 (2H, m).
MS (+ve ion electrospray) m/z 451 (MH+).
The free base in dichloromethane was treated with one equivalent of oxalic acid (0.1M in ether) to give the title compound.

### Example 4 1-(2-Benzo[1,3]dioxol-5-yl-ethyl)-4-methylpiperidine-4-carboxylic acid (6-methoxy-[1,5]naphthyridin-4-yl)-amide oxalate

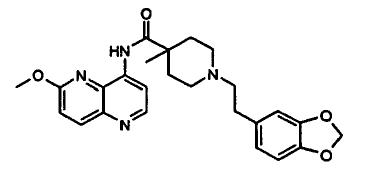

### (a) Title compound

This was prepared by alkylation of piperidine (1j) (0.1g, 0.33 mmol) with mesylate (3e) (0.08g, 0.33 mmol) according the procedure of Example (1k). Chromatography afforded the free base of the title compound (64 mg, 43%). δH (CDCl₃, 250MHz): 9.90 (1H, br), 8.71 (1H, d), 8.51 (1H, d), 8.23 (1H, d), 7.18 (1H, d), 6.72-6.67 (2H, m), 6.62 (1H, d), 5.91 (2H, s), 4.09 (3H, s), 2.72 (4H, m), 2.56 (2H, m), 2.44 (2H, m), 2.31 (2H, m), 1.79 (2H, m), 1.40 (3H, s).
MS (+ve ion electrospray) m/z 449(MH+).
The free base in dichloromethane was treated with one equivalent of oxalic acid (0.1M in ether) to give the title compound as a white solid.

### Example 5 4-Hydroxy-1-[2-(3-oxo-3,4-dihydro-2H-benzo[1,4]thiazin-6-yl)-ethyl]-piperidine-4-carboxylic acid (6-methoxy-[1,5]naphthyridin-4-yl)-amide oxalate

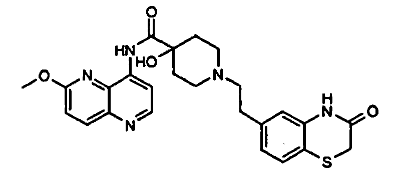

The piperidine Example (3c) (0.16g) was alkylated with mesylate (1f) (0.095g, 0.33 mmol) by the method of Example (1k).
Chromatography on silica eluting with 2-10% methanol/dichloromethane gave the free base of the title compound (34 mg, 21%).
δH (CDCl₃, 250MHz): 8.62 (1H, d), 8.48 (1H, d), 8.16 (1H, d), 7.23 (1H, d), 7.14 (1H, d), 6.87 (1H, dd), 6.78 (1H, d),, 4.11 (3H, s), 3.40 (2H, s), 2.95 (2H, m), 2.79 (2H, m), 2.68 (2H, m), 2.53 (2H, br t), 2.37 (2H, td), 1.80 (2H, br d).
MS (+ve ion electrospray) m/z 494(MH+).
The free base in dichloromethane was treated with one equivalent of oxalic acid (0.1M in ether) to give the title compound as a white solid.

### Example 6 4-(6-Methoxy-[1,5]naphthyridin-4-ylcarbamoyl)-1-12-(3-oxo-3,4-dihydro-2H-benzo[1,4]thiazin-6-yl)-ethyl]-piperidine-4-carboxylic acid

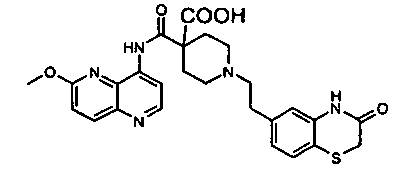

### (a) Bis-(2-chloroethyl)carbamic acid benzyl ester

To a solution of bis-(2-chloroethyl)amine hydrochloride (8.9g, 49.9 mmol) in water (100 ml) at 0°C were added benzyl chloroformate (7.08 ml, 50 mmol) and 8% aqueous sodium hydroxide dropwise to maintain the mixture at pH 8.5-9.5. After addition of chloroformate was complete, further sodium hydroxide was added until the pH remained stable (after 2 hours), and then the pH was raised to 10. The mixture was extracted three times with ether, the extracts were washed with dilute hydrochloric acid, dried and evaporated to give the carbamate (12.93g, 94%).
MS (+ve ion electrospray) m/z 277(MH+).

### (b) Piperidine-1,4,4-tricarboxylic acid 1-benzyl ester 4-tert-butyl ester 4-methyl ester

A mixture of the bis-(chloroethyl) carbamate (a) (8.80g, 32 mmol), malonic acid tert-butyl ester methyl ester (5.44 ml, 32 mmol), tetrabutylammonium iodide (2.37g, 6.4 mmol) and potassium carbonate (13.28g, 96 mmol) in N,N-dimethylformamide (100 ml) was heated at 80°C for 3 days, then evaporated. The residue was dissolved in ether and water. The aqueous phase was extracted twice with ether and the combined organics were washed with water and brine, dried and evaporated. Chromatography on silica eluting with 10-20% ethyl acetate/hexane gave a clear gum (3.30g, 27%).
MS (+ve ion electrospray) m/z 278 (loss of C₅H₈O₂ from MH+).

### (c) Piperidine-1,4,4-tricarboxylic acid 1-benzyl ester 4-tert-butyl ester

A mixture of the triester (b)(3.30g, 8.75 mmol) and 2M aqueous sodium hydroxide (8.75 ml, 17.5 mmol) in tetrahydrofuran (10 ml) was stirred vigorously at room temperature for 3 days. The mixture was partially evaporated, washed with ether, then acidified to pH2 and extracted well with ether. These extracts were dried and evaporated to give a white solid (2.92g, 92%).
MS (+ve ion electrospray) m/z 264 (loss of C₅H₈O₂ from MH+).

### (d) 4-(6-Methoxy-[1,5]naphthyridin-4-ylcarbamoyl)-piperidine-1,4-dicarboxylic acid 1-benzyl ester 4-tert-butyl ester

This was prepared from acid (c) and amine (1c) with 1,1'-carbonyldiimidazole coupling according to the procedure for Example (1i). Chromatography on silica eluting with 2% methanol/dichloromethane gave a yellow gum (2.64g, 63%).
MS (+ve ion electrospray) m/z 521 (MH+).

### (e) 4-(6-Methoxy-[1,5]naphthyridin-4-ylcarbamoyl)-piperidine-4-carboxylic acid 4-tert-butyl ester

A solution of the amide (d) (1.0g, 1.92 mmol) in ethanol (20 ml) was stirred with 10% palladium on charcoal (0.2g) under 1 atmosphere of hydrogen for 12 hours. Filtration and evaporation of solvent gave a white solid (0.77g, 100%).
MS (+ve ion electrospray) m/z 387 (MH+).

### (f) 4-([1,5]Naphthyridin-4-ylcarbamoyl)-1-[2-(3-oxo-3,4-dihydro-2H-benzo[1,4]thiazin-6-yl)-ethyl]-piperidine-4-carboxylic acid 4-tert-butyl ester

The piperidine (e) (0.15g) was alkylated with mesylate (1f) (0.114g, 0.40 mmol) by the method of Example (1k). Chromatography on silica eluting with 2-5% methanol/dichloromethane gave the product (0.049g, 22%).
δH (CDCl₃, 250MHz): 8.70(1H, d), 8.46(1H, d), 8.20(1H, d), 7.20(1H, d), 7.15(1H, d), 6.85(1H, dd), 6.78(1H, d),, 4.17(3H, s), 3.40(2H, s), 2.82(2H, m), 2.74(2H, m), 2.55(2H, m), 2.45-2.20(6H, m), 1.45(9H, s).
MS (+ve ion electrospray) m/z 578(MH+).

### (g) Title compound.

The tert-butyl ester (f) (0.049g) was dissolved in dichloromethane (2 ml) and trifluoroacetic acid (2 ml). The mixture was allowed to stand at room temperature for 5 hours then evaporated. Chromatography on silica gel eluting with 1% conc.aqueous ammonia/ 9% methanol in dichloromethane gave the free base of the title compound (0.019g).
MS (+ve ion electrospray) m/z 522(MH+).

### Example 7 6-{1-R,S-Hydroxy-2-[2-(6-methoxy-[1,5]naphthyridin-4-yl)-1-oxo-2,8-diaza-spiro[4.5]dec-8-yl]-ethyl}-4H-benzo[1,4]oxazin-3-one oxalate

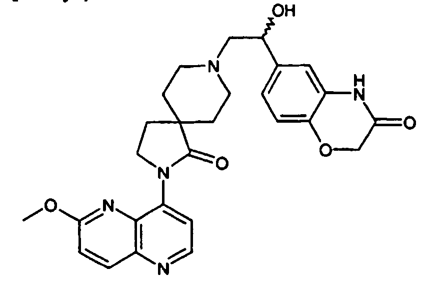

### (a) Piperidine-1,4-dicarboxylic acid 1-tert-butyl ester 4-ethyl ester

Di-tert-butyl dicarbonate (21.12g, 96.88 mmol) was added to piperidine-4-carboxylic acid ethyl ester (15.21g, 96.88 mmol) as a solution in dichloromethane (50 ml). The resulting solution was stirred at room temperature for 1 hour after which time the reaction mixture was partitioned between dichloromethane and a saturated aqueous solution of sodium bicarbonate. The organic phases were combined and dried over magnesium sulphate. The volatiles were removed *in vacuo* and the residue used without further purification.
MS (+ve ion electrospray) m/z 158 (loss of C₅H₈O₂ from MH+).

### (b) 4-Cyanomethyl-piperidine-1,4-dicarboxylic acid 1-tert-butyl ester 4-ethyl ester

Ester (a) (5.20g, 20.23 mmol) was dissolved in tetrahydrofuran (70 ml) and cooled to -78°C. To the solution lithium diisopropylamide (2M, 11.1 ml, 22.26 mmol) was added and stirring was continued for 0.5 hour. Bromoacetonitrile (1.69 ml, 24.28 mmol) was added and the resulting solution allowed to warm to room temperature over 12 hours. The reaction was quenched by the addition of water (2 ml) and the volatiles removed *in vacuo.* The residue was subjected to purification on silica using an ethyl acetate/hexane solvent gradient. This provided a colourless oil (3.17g, 53%).

### (c) 1-Oxo-2,8-diaza-spiro[4.5]decane-8-carboxylic acid-tert-butyl ester

Nitrile (b) (1.50g, 5.07 mmol) was cooled to 0°C and cobalt dichloride hexahydrate (603 mg, 2.53 mmol) was added. To the resulting slurry sodium borohydride (1.93g, 50.7 mmol) was added and stirring continued at room temperature for 48 hours. Concentrated aqueous ammonia (1.5 ml) was added and the mixture filtered through keiselguhr. The filtrate was partitioned between ethyl acetate and water. The organic phases were combined and dried over magnesium sulfate. The volatiles were removed under reduced pressure and the residue purified by chromatography on silica eluting with an ethyl acetate/hexane gradient to give a white solid (680 mg, 53%).
MS (+ve ion electrospray) m/z 155 (loss of C₅H₈O₂ from MH+).

### (d) 2-(6-Methoxy-[1,5]naphthyridin-4-yl)-1-oxo-2,8-diaza-spiro[4.5]decane-8-carboxylic acid tert-butyl ester

Triflate (1b) (629 mg, 2.04 mmol), lactam (c) (432 mg, 1.70 mmol), dipalladium-tris (dibenzylideneacetone) chloroform complex (35 mg, 0.034 mmol), *R*-2,2'-bis(diphenylphosphino)-1,1'-binaphthyl (63mg, 0.102 mmol) and cesium carbonate (776 mg, 2.38 mmol) were all combined and stirred in 1,4-dioxane (5 ml) which had been degassed with argon. The slurry was heated to 90°C for 3 hours. The solvent was then removed under reduced pressure and the residue purified on silica gel using a dichloromethane/methanol solvent gradient. This provided the desired compound as a colourless oil (460mg, 66%).
MS (+ve ion electrospray) m/z 413 (MH⁺), 313(loss of C₅H₈O₂ from MH+).

### (e) 6-{2-[2-(6-Methoxy-[1,5]naphthyridin-4-yl)-1-oxo-2,8-diaza-spiro[4.5]dec-8-yl]-ethanoyl}-4H-benzo[1,4]oxazin-3-one

Amide (d) (156 mg) was dissolved in dichloromethane (5 ml) and treated with trifluoroacetic acid (1 ml). The solution was stirred at room temperature for 2 hours after which time the solvent was removed *in vacuo.* The residue was redissolved in N,N-dimethylformamide (5 ml) then potassium carbonate (156 mg, 1.13 mmol) and 6-(2-chloro-ethanoyl)-4*H*-benzo[1,4]oxazin-3-one (127 mg, 0.564 mmol) were added. Sodium iodide (5mg) was added to the resulting solution and the reaction was stirred at room temperature for 12 hours. The solvent was removed under reduced pressure and the residue purified by chromatography on silica using a methanol and dichloromethane solvent gradient. This provided the desired compound as a colourless oil (90 mg, 48%). MS (+ve ion electrospray) m/z 502 (MH⁺).

### (f) Title compound

Ketone (e) (90mg, 0.179mmol) was dissolved in iso-propyl alcohol (5 ml). The solution was cooled to 0°C and sodium borohydride (14 mg, 0.36 mmol) was added. The reaction was stirred at room temperature for 4 hours and then quenched by the addition of water (1 ml). The volatiles were removed *in vacuo* and the residue purified by chromatography on silica using a dichloromethane/methanol solvent gradient. This provided the free base of the desired compound as a white solid (27 mg, 30%).
δH (CD₃OD, 250MHz): 8.74 (1H, d), 8.25 (1H, d), 7.72 (1H, d), 7.25 (1H, d), 7.00-6.85 (3H, m), 4.54 (2H, s), 4.21 (2H, t), 4.04 (3H, s), 3.32 (2H, s), 3.06 (2H, m), 2.65-2.40 (2H, m), 2.29 (2H, t), 2.14 (2H, m), 1.77 (2H, m).
MS (+ve ion electrospray) m/z 504(MH+).
The free base in dichloromethane was treated with 1 equivalent of oxalic acid (0.1M in ether) to give the title compound as a white solid.

### Example 8 4-Hydroxy-1-[2-oyo-2-(3-oxo-3,4-dihydro-2H-benzo[1,4]thiazin-6-yl)-ethyl]-piperidine-4-carboxylic acid (6-methory-[1,5]naphthyridin-4-yl)-amide oxalate

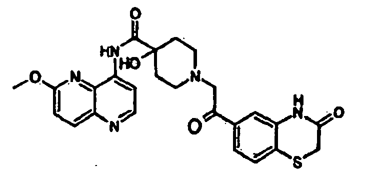

### (a) 6-(2-Chloro-ethanoyl)-4H -benzo[1,4]thiazin-3-one

A solution of 4H -benzo[1,4]thiazin-3-one (6.0g, 36 mmol) and chloroacetylchloride (3.2 ml, 40 mmol) in 1,2-dichloraethane (20 ml) under argon was treated portionwise with aluminium trichloride (10.6g, 80 mmol) while maintaining the internal temperature below 10°C with ice bath cooling. After the addition was complete, the mixture was heated to reflux for 1 hour. The cooled mixture was added to a stirred mixture of ice/concentrated aqueous hydrochloric acid. The resulting precipitate was filtered, washed with water, and dried *in vacuo* (4.0g, 45%).

### (b) Title compound

The piperidine Example (3c) (0.32g) was alkylated with chloroketone (a) (0.095g, 0.33 mmol) by the method of Example (1k). After evaporation of solvent, the residue was dissolved in water and dichloromethane/methanol. The aqueous phase was re-extracted (dichloromethane/methanol) and the combined organics were washed with water, dried and evaporated. Chromatography on silica eluting with 2-5% methanol/dichloromethane gave the free base of the title compound (0.11g, 31%).
δH (d6-DMSO, 250MHz): 11.07 (1H, s), 10.78 (1H, s), 8.70 (1H, d), 8.45 (1H, d). 828 (1H, d), 7.65 (2H, m), 7.48 (1H, d), 7.34 (1H, d), 6.21 (1H, s), 4.10 (3H, s), 3.76 (2H, s), 3.56 (2H, s), 2.76 (2H, m), 2.46 (2H, m), 2.09 (2H, m), 1.63 (2H, br d).
MS (+ve ion electrospray) m/z 508(MH+).
The free base in dichloromethane/memanol was treated with one equivalent of oxalic acid (0.1M in ether) to give the title compound as a white solid.

### Example 9 4-Hydroay-1-[2-R,S-hydroxy-2-(3-oxo-3,4-dihydro-2H-benzo[1,4]thiazin-6-yl)-ethyl]-piperidine-4-carboxylic acid (6-methoxy-[1,5]naphthyridin-4-yl)-amide oxalate

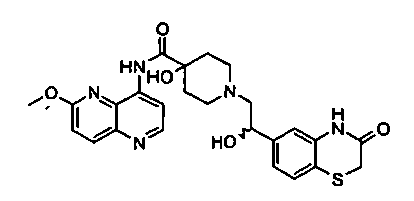

A solution of the ketone Example 8 (0.08g, 0.16 mmol) in a mixture of dichloromethane, methanol and tetrahydrofuran (20 ml each) was treated with sodium borohydride (0.018g, 0.48 mmol). After stirring for 2.5 hours, the mixture was evaporated and the residue was dissolved in dichloromethane/methanol, washed with water, dried and evaporated. Chromatography on silica eluting with 5-20% methanol in dichloromethane gave the free base of the title compound (0.024g, 29%). δH (d6-DMSO, 400MHz): 11.06 (1H, s), 10.52 (1H, s), 8.70 (1H, d), 8.45 (1H, d), 8.28 (1H, d), 7.33 (1H, d), 7.25 (1H, d), 7.03(1H, s), 6.97 (1H, d), 6.14 (1H, br), 5.05 (1H, br), 4.68 (1H, br), 4.10 (3H, s), 3.44 (2H, s), 2.82 (2H, m), 2.45 (2H, m), 2.10 (2H, m), 1.62 (2H, br d).
MS (+ve ion electrospray) m/z 510(MH+).
The free base in dichloromethane/methanol was treated with one equivalent of oxalic acid (O.1M in ether) to give the title compound as a white solid.

### Example 10 4-Hydroxymethyl-1-[2-(3-oxo-3,4-dihydro-2H-benzo[1,4]thiazin-6-yl)-ethyl]-piperidine-4-carboxylic acid (6-metboxy-[1,5]naphthyridin-4-yl)-amide oxalate

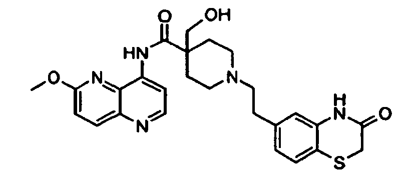

### (a) 4-(6-Methoxy-[1,5]naphthyridin-4-ylcarbamoyl)-piperidine-1,4-dicarboxylic acid 1-benzyl ester

The diester Example (6d) (1.64g, 3.15 mmol) in dichloromethane (10 ml) was treated with trifluoroacetic acid (10 ml). After 3.75 hours at room temperature, the mixture was evaporated and the residue was dissolved in water, basified with sodium bicarbonate, and extracted with dichloromethane. The extract was dried and evaporated then the crude product was chromatographed on silica eluting with 10% methanol/dichloromethane to give a white solid (1.40g, 96%)
MS (+ve ion electrospray) m/z 465(MH+).

### (b) 4-Hydroxymethyl-4-(6-methoxy-[1,5]naphthyridin-4-ylcarbamoyl)-piperidine-1-carboxylic acid benzyl ester

The acid (a) (0.70g, 1.51 mmol) in dry tetrahydrofuran (10 ml) was treated with triethylamine (0.22 ml, 1.6 mmol) and cooled in an ice-bath. Iso-butyl chloroformate (0.19 ml, 1.51 mmol), was added and the mixture was stirred for 2 hours at 0°C. The mixture was filtered under suction into a solution of sodium borohydride (0.60g) in iced water (15 ml) and the mixture was stirred at 0°C for 2 hours, then partially evaporated. The aqueous residue was treated with dilute hydrochloric acid to give pH 8-9, then extracted with ethyl acetate. The extract was washed with brine, dried and evaporated. Chromatography on silica eluting with 2.5-5% methanol/dichloromethane gave a clear gum (0.19g, 28%).
MS (+ve ion electrospray) m/z 451(MH+).

### (c) 4-Hydroxymethyl-4-(6-methoxy-[1,5]naphthyridin-4-ylcarbamoyl)-piperidine

A solution of the benzyl carbamate (b) (0.18g, 0.4 mmol) in ethanol (20 ml) was hydrogenated at 1 atmosphere pressure over 10% palladium/charcoal for 20 hours. Filtration through kieselguhr and evaporation gave a white solid (0.13g, 100%).
MS (+ve ion electrospray) m/z 317(MH+).

### (d) Title compound

The piperidine (c) (0.13g, 0.41 mmol) was alkylated with mesylate (1f) (0.118g, 0.41 mmol) by the method of Example (1k), except that heating at 50-60°C overnight was required to give complete reaction. Chromatography on silica eluting with 2-10% methanol/dichloromethane gave the free base of the title compound (52 mg). δH (CDCl₃, 250MHz): 10.28 (1H, s), 8.53 (1H, d), 8.37(1H, s), 8.36 (1H, d), 8.10 (1H, d), 7.19 (1H, d), 7.06 (1H, d), 6.83 (1H, dd), 6.69 (1H, d), 4.05 (3H, s), 3.84 (2H, s), 3.39 (2H, s), 2.9-2.7 (m), 2.60 (m), 2.5-2.3 (m), 1.9-1.7 (m).
MS (+ve ion electrospray) m/z 508(MH+).
The free base in dichloromethane/chloroform was treated with 1 equivalent of oxalic acid (O.1M in ether) to give the title compound as a white solid.

### Example 11 4-Amino-1-[2-(7-fluoro-3-oxo-3,4-dihydro-2H-benzo[1,4]thiazin-6-yl)-2-R,S-hydroxy-ethyl]-piperidine-4-carboxylic acid (6-methoxy-[1,5]naphthyridin-4-yl)-amide dioxalate

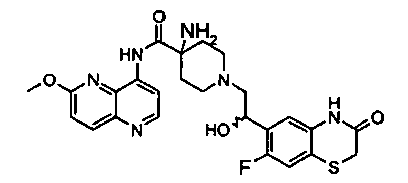

### (a) (5-Fluoro-2-nitro-phenylsulfanyl)-acetic acid ethyl ester

A solution of 2,4-difluoronitrobenzene (38.2g, 240 mmol) and triethylamine (38.4 ml, 28g, 280 mmol) in dichloromethane (240 ml) was treated at 0°C with ethylthioglycolate (20.4 ml, 30.5g, 240 mmol) over 0.25 hour. The mixture was stirred at room temperature overnight then added to a column of silica eluting with hexane/dichloromethane (1/1) affording a yellow oil (46g, 74%).
δH (CDCl₃, 250MHz): 8.30 (1H, m), 7.27-7.15 (2H, m), 4.25 (2H, q), 3.75 (2H, s), 1.30 (3H, t).

### (b) 7-Fluoro-4H-benzo[1,4]thiazin-3-one

A vigorously stired mixture of (a) (46g) in acetic acid (500 ml) was treated with iron (120g). An exothermic reaction occurred. After stirring at ambient temperature for a further 2 hours, the mixture was filtered through keiselguhr. The filtrate was diluted with ethyl acetate and washed with water. The organic extract was dried and concentrated *in vacuo* to approximately 500 ml, when crystallisation commenced. The mixture was set aside in the fridge for 3 hours, then filtered and the resulting solid dried *in vacuo* (12.2g, 38%).
δH (CDCl₃, 250MHz) : 7.05-6.80 (3H, m), 3.38 (2H, s).

### (c) 6-(2-Chloro-ethanoyl)-7-fluoro-4H-benzo[1,4]thiazin-3-one

This was prepared from (b) (1.5g) and chloroacetylchloride (0.7 ml) according to the procedure for (8a) with the modification that the mixture was heated to reflux for 4 hours. After precipitation, a solid (0.25g, 12%) was obtained.
MS (+ve ion electrospray) m/z 260 (MH+).

### (d) Title compound

A solution of amine (2d) (116 mg, 0.38 mmol) and chloromethyl ketone (c) (100 mg, 0.38 mmol) in N,N-dimethylformamide (2 ml) was treated with potassium carbonate (58 mg, 0.42 mmol). After 3 hours the mixture was filtered into a solution of tetrahydrofuran/water/methanol (1 ml/5 ml/3 ml) containing sodium borohydride (20 mg). After 1 hour the mixture was partitioned between ethyl acetate and brine. The organic extract was dried and evaporated. The residue was chromatographed eluting with a methanol/ethyl acetate gradient affording the free base of the title compound as an oil (10 mg, 5%).
δH (CD₃OD, 250MHz): 8.60 (1H, d), 8.55 (1H, d), 8.17 (1H, d), 7.25 (1H, d), 7.18 (1H, d), 7.07 (1H, d), 5.20 (1H, t), 4.15 (3H, s), 3.40 (2H, s), 3.20-3.05 (2H, m), 2.85-2.70 (4H, m), 2.50-2.35 (2H, m), 1.75-1.65 (2H, m).
The free base in dichloromethane was treated with 2 equivalents of oxalic acid (0.1 M in ether) to give the title compound as a white solid.

### Example 12 4-Amino-1-[2-R,S-hydroxy-2-(3-oxo-3,4-dihydro-2H-benzo[1,4]thiazin-6-yl)-ethyl]-piperidine-4-carboxylic acid (6-methoxy-[1,5]naphthyridin-4-yl)-amide dihydrochloride

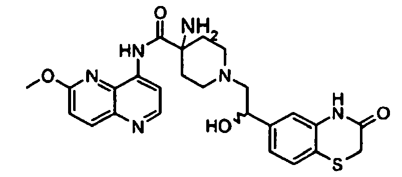

### (a) 4-Amino-1-[2-oxo-2-(3-oxo-3,4-dihydro-2H-benzo[1,4]thiazin-6-yl)-ethyl]-piperidine-4-carboxylic acid (6-methoxy-[1,5]naphthyridin-4-yl)-amide

This was prepared by reaction of chloromethyl ketone (8a) and amine (2d) by the same procedure as for Example (8b). After 6 hours at room temperature the mixture was filtered and evaporated. Trituration with water afforded a solid (390 mg, 90%).

MS (+ve ion electrospray) m/z 507 (MH⁺)

### (b) Title compound

A solution of ketone (a) (200 mg, 0.4 mmol) in tetrahydrofuran/methanol (15 ml/15 ml) was treated at 0°C with sodium borohydride (15 mg, 0.4 mmol). After 3 hours at room temperature, workup and chromatography in a similar manner as for Example (11d) afforded the free base of the title compound as a solid (35 mg, 20%). δH (CD₃OD 250MHz): 8.60 (1H, m), 8.50 (1H, m), 8.15 (1H, m), 7.30-7.20 (2H, m), 7.07-7.02 (2H, m), 4.85(1H, m), 4.12(3H, s), 3.40 (2H, s), 2.95 (2H, m), 2.80-2.30 (6H, m), 1.60 (2H, m).
MS (+ve ion electrospray) m/z 509 (MH+).
The free base in dichloromethane/methanol was treated with 2 equivalents of hydrochloric acid (1M in ether) to give the title compound as a white solid.

### Example 13 1-[2-(2,3-Dihydro-benzo[1,4]dioxin-6-yl)-2-(R,S)-hydroxy-ethyl]-4-hydroxy-piperidine-4-carboxylic acid (6-methoxy-[1,5]naphthyridin-4-yl)-amide dihydrochloride

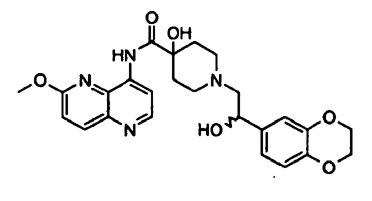

A mixture of piperidine Example (3c) (0.10g, 0.33 mmol), 6-(2-bromoethanoyl)-2,3-dihydro-benzo[1,4]dioxin (0.090g, 0.35 mmol), potassium carbonate (0.049g, 0.35 mmol) and tetrabutylammonium iodide (10 mg) in N,N-dimethylformamide (5 ml) was stirred at room temperature for 6.5 hours. Water was added to give a total volume of 40 ml, and the resulting precipitate was filtered off, washing with water. This solid was dissolved in 1:1 dichloromethane/methanol (20 ml). Sodium borohydride was added and the mixture was stirred for 1 hour at room temperature, then slightly acidified with dilute hydrochloric acid and evaporated. The residue was dissolved in water, basified, and extracted with dichloromethane/methanol. The extract was washed with brine, dried and evaporated. Chromatography on silica eluting with 2-5% methanol/dichloromethane gave the free base of the title compound (0.109g, 69%).
δH (CD₃OD/CDCl₃, 250MHz): 8.62 (1H, d), 8.55 (1H, d), 8.18 (1H, d), 7.23 (1H, d), 6.90 (1H, s), 6.83 (2H, m), 4.73, (1H, dd), 4.25(4H, s), 4.16 (3H, s), 3.01 (1H, br d), 2.87 (1H, br d), 2.75-2.4 (4H, m), 2.39 (1H, dd), 2.28 (1H, dd), 1.76 (2H, br d).
MS (+ve ion electrospray) m/z 481(MH+).
A solution of the free base in dichloromethane/methanol was treated with 2 equivalents of hydrogen chloride (4M solution in 1,4-dioxan). The solvent was evaporated and the residue was triturated with ether and dried to give the title compound.

### Example 14 4-Hydroxy-1-[2-(6-oxo-6,7-dihydro-5H-pyridazino[3,4-b][1,4]thiazin-3-yl)-ethyl]-piperidine-4-carboxylic acid (6-methoxy-[1,5]naphthyridin-4-yl)-amide dihydrochloride

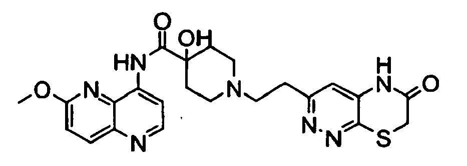

### (a) 4-Amino-3,6-dichloropyridazine

A suspension of 3,4,6-trichloropyridazine (prepared by the method ofB. Kasnar et al, Nucleosides and Nucleotides, 1994, *13,* 459) (10.0g) in conc. aqueous ammonia (1L) was heated at 75°C for 16h. The mixture was concentrated to a small volume and extracted several times with ethyl acetate. The extracts were washed with brine, dried and evaporated. The crude product was recrystallised from ethyl acetate to give a solid (5.03g)

### (b) 3-Chloro-6-oxo-6,7-dihydro-5H-pyridazino[3,4-b][1,4]thiazine

To a well-stirred suspension of sodium hydride (60% in mineral oil, 0.35g, 8.5 mmol) in anhydrous dimethylformamide (10mL) at 0°C was added methyl mercaptoacetate (0.70mL, 7.9 mmol). After stirring at this temperature for 20min, a solution of 4-amino-3,6-dichloropyridazine (14a)(1.29g, 7.87 mmol) in dimethylformamide (10mL) was added. The mixture was stirred at room temperature for 16h, then most of the solvent was removed in vacuo. The residue was diluted with water, the precipitate was filtered off, washed with water and dried. Chromatography on silica (0-2% methanol/dichloromethane) gave the product (0.21g, 13%).
MS (+ve ion electrospray) *m*/*z* 202/204 (MH⁺)

### (c) 6-Oxo-3-vinyl-6,7-dihydro-5H-pyridazino[3,4-b][1,4]thiazine

To a mixture of the pyridazinothiazine (14b) (0.15g, 0.75 mmol), bis(triphenylphosphine)palladium(II) chloride (84mg, 0.12 mmol) and lithium chloride (63mg, 1.2 mmol) in dimethylformamide (3mL) was added tributyl(vinyl)tin (0.36mL, 1.2 mmol). The mixture was heated at 110-120°C for 16h, then evaporated. The residue was partitioned between water and ethyl acetate, the aqueous phase was extracted further with ethyl acetate and the combined organics were dried and evaporated. Chromatography on silica (0-3% methanol/dichloromethane) gave the product (45mg, 31%).
MS (+ve ion electrospray) m/z 194 (MH⁺)

### (d) Title compound

A mixture of the vinyl compound (14c) (67mg, 0.35 mmol), piperidine (3c) (0.106g, 0.35 mmol) and acetic acid (0.03mL) in ethanol (2mL) was heated under reflux for 24h. Ethyl acetate and aqueous sodium bicarbonate were added and the phases separated. The aqueous phase was extracted several times with dichloromethane/methanol and the combined organics were dried and evaporated. Chromatography on silica gel (0-10% methanol/dichloromethane) gave the free base of the title compound (83mg, 48%).
MS (+ve ion electrospray) m/z 496 (MH⁺)
¹H NMR δ(CDCl₃/CD₃OD) 8.63 (1H, br s), 8.55 (1H, br s), 8.19 (1H, d), 7.72 (broad), 7.26 (1H, d), 7.00 (1H, s), 4.17 (3H, s), 3.72 (2H, s), 3.22-2.92 (6H, m), 2.68 (2H, t), 2.35 (2H, t), 1.81 (2H, d).
The free base in chloroform/methanol was treated with 2 equivalents of 0.4M hydrochloric acid in dioxan. Evaporation of solvent and trituration with ether gave the dihydrochloride salt.

### Example 15 4-Hydroxy-1-[2-R-hydroxy-2-(3-oxo-3,4-dihydro-2H-benzo[1,4]thiazin-6-yl)-ethyl]-piperidine-4-carboxylic acid (6-methoxy-[1,5]naphthyridin-4-yl)-amide dihydrochloride and 4-Hydroxy-1-[2-S-hydroxy-2-(3-oxo-3,4-dihydro-2H-benzo[1,4]thiazin-6-yl)-ethyl]-piperidine-4-carboxylic acid (6-methoxy-[1,5]naphthyridin-4-yl)-amide dihydrochloride

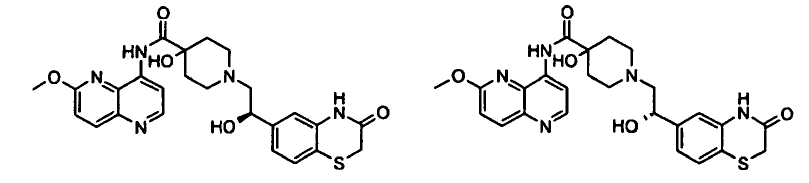

A sample of the racemate Example 9 (200mg) was separated by HPLC on a Chirobiotic V column (vancomycin stationary phase, 250mm x 4.6mm i.d.) eluted with 0.1% acetic acid and 0.1% triethylamine in methanol, 1.0mL/min. to give a faster-eluting isomer (r.t. 13.0 min., 63mg, 89.8% e.e.) and a slower-eluting isomer (r.t. 14.1 min., 70mg, 93.6% e.e.). For each isomer, the compound was suspended in aqueous sodium bicarbonate and extracted several times with dichloromethane/methanol. After drying and evaporation of the extracts, the residue, together with undissolved solid, was dissolved in methanol and treated with a few drops of 5M hydrochloric acid. Evaporation of the methanol gave the dihydrochloride salts.
MS (+ve ion electrospray) m/z 510(MH+).

### Example 16 1-[2- R,S-Hydroxy-2-(3-oxo-3,4-dihydro-2H-benzo[1,4]thiazin-6-yl)-ethyl]- 4-methoxy-piperidine-4-carboxylic acid (6-methoxy-[1,5]naphthyridin-4-yl)-amide dihydrochloride

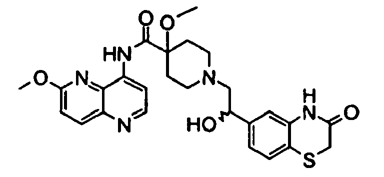

### (a) 4-Methoxypiperidine-1,4-dicarboxylic acid, 1-tert-butyl ester 4-methyl ester

4-Hydroxypiperidine-1,4-dicarboxylic acid, 1-tert-butyl ester (3a) was first methylated with methyl iodide and potassium carbonate in dimethylformamide (room temperature, overnight) to give the 4-methyl ester (100%), then methylated again by treatment with sodium hydride in THF (room temp., 1h) followed by methyl iodide (room temp., overnight), to give the 4-methoxy diester as a yellow gum (61% from 4-hydroxy acid).
MS (+ve ion APCI) m/z 174(loss of C₅H₈O₂ from MH+).

### (b) 4-Methoxypiperidine-1,4-dicarboxylic acid, 1-tert-butyl ester 4-amide

A solution of the ester (16a)(1.34g.4.9 mmol) in THF (20 mL) was saturated with gaseous ammonia and left to stand at room temperature for three days. Aqueous ammonia (d=0.88, 20mL) was then added and the mixture was stirred vigorously for 4 days, then evaporated. The residue was chromatographed on silica (50- 100% ethyl acetate/hexanes) to give the amide as a white solid (0.25g, 20%), and recovered ester (0.83g, 62%).
MS (+ve ion APCI) m/z 159 (loss of C₅H₈O₂ from MH+).

### (c) 4-Methoxy-4-(6-methoxy-[1,5]naphthyridin-4-ylcarbamoyl)-piperidine-1-carboxylic acid tert-butyl ester

This was prepared from the amide (16b) (0.24g, 0.93 mmol) and triflate (1b) (0.29g, 0.93mmol) by the method of Example (3b, method B). Chromatography on silica gel (0-1.5% methanol/dichloromethane) gave the coupled product (0.35g, 90%).
MS (+ve ion electrospray) m/z 417 (MH+).

### (d) 4-Methoxy-4-(6-methoxy-[1,5]naphthyridin-4-ylcarbamoyl)- piperidine

The tert-butyl ester (16c) was treated with trifluoroacetic acid by the method of Example (1j) to give a white solid (100%).
MS (+ve ion electrospray) m/z 317 (MH+).

### (e) Title compound

This was prepared from piperidine (16d) (0.10g, 0.32 mmol) and chloroketone (8a) (81mg, 0.33mmol) by the method of Example 13. Chromatography on silica (0-5% methanol/dichloromethane) gave the free base (115mg, 69%).
δH (CDCl₃): 10.53 (1H, s), 8.72 (1H, d), 8.50 (1H, d), 8.23 (1H, d), 8.09(1H, broad s), 7.29 (1H, d), 7.17 (1H, d), 6.99(1H, d), 6.95(1H, s), 4.72 (1H, dd), 4.13 (3H, s), 3.43(2H, s), 3.42 (3H, s), 3.05 (1H, m) 2.72 (2H, m), 2.60 (1H, dd), 2.45 (2H, m), 2.30 (2H, m), 1.99 (2H, br m).
MS (+ve ion electrospray) m/z 524 (MH+).
The dihydrochloride salt was prepared as described for Example 13, but using 0.4M hydrogen chloride in dioxan.

### Example 17 1-[2-(2,3-Dibydro-benzo[1,4]dioxin-6-yl)-2-R,S-hydroxy-ethyl]-4-methoxy-piperidine-4-carboxylic acid (6-methoxy-[1,5]naphthyridin-4-yl)-amide dihydrochloride

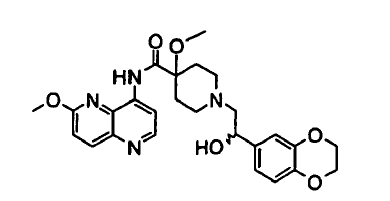

This was prepared from piperidine (16d) (0.10g, 0.32 mmol) and 6-(2-bromoethanoyl)-2,3-dihydro-benzo[1,4]dioxin (84mg, 0.33mmol) by the method of Example 13. Chromatography on silica (0-5% methanol/dichloromethane) gave the free base (118mg, 75%).
δH (CDCl₃): 10.52 (1H, s), 8.72 (1H, d), 8.50 (1H, d), 8.22 (1H, d), 7.17 (1H, d), 6.91(1H, s), 6.84 (2H, s), 4.65 (1H, dd), 4.25 (4H, s), 4.13 (3H, s), 3.41 (3H, s), 3.04 (1H, m) 2.75 (1H, m), 2.68 (1H, dd), 2.55-2.17 (5H, complex m), 1.97 (2H, br m).
MS (+ve ion electrospray) m/z 495 (MH+).
The dihydrochloride salt was prepared as described for Example 16.

### Example 18 (3S/R,4R/S)-3,4-Dihydroxy-1-[2-(3-oxo-3,4-dihydro-2H-benzo[1,4]thiazin-6-yl)-ethyl]-piperidine-4-carboxylic acid (6-methoxy-[1,5]naphthyridin-4-yl)-amide dihydrochloride

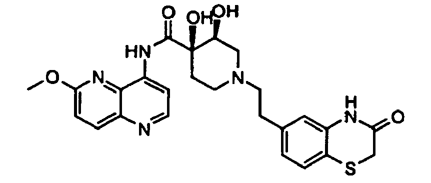

### (a) 1,2,3,6-Tetrahydro-pyridine-1,4-dicarboxylic acid 1-tert-butyl ester

A solution of 1-methyl-1,2,3,6-tetrahydro-pyridine-4-carboxylic acid ethyl ester (54.5g, 323.5 mmol) in anhydrous 1,2-dichloroethane (150mL) was cooled in ice. 1-Chloroethyl chloroformate (34.88mL, 323.5 mmol) was added and the mixture was stirred at 0°C for 10 min, then heated under reflux for 3h. Solvent was then evaporated and the residue was dissolved in methanol and heated under reflux for 2h, then evaporated again. The crude product was dissoved in dichloromethane (500mL) containing triethylamine (90.8mL), and di-tert-butyl dicarbonate (77.4g, 355mmol) was added in portions. The mixture was stirred at room temperature for 3 days, then washed with water and dilute hydrochloric acid, dried and evaporated. Chromatography on silica (5-10% ethyl acetate/hexanes) gave the 1-tert-butyl carbamate as methyl and ethyl esters (partially separated), which were hydrolysed by treatment with 2% aqueous sodium hydroxide (14.5mL) in THF (30mL) at room temperature for 24h. Acidification to pH2 and extraction with ethyl acetate gave the carboxylic acid (6.79g).
MS (-ve ion electrospray) m/z 226 (M-H⁻).

### (b) 1,2,3,6-Tetrahydro-pyridine-1,4-dicarboxylic acid 1-tert-butyl ester 4-benzyl ester

The acid (18a) (5.56, 24.5 mmol) was esterified by treatment with potassium carbonate (3.84g, 30 mmol) and benzyl bromide (2.83mL, 24 mmol) in dimethylformamide (60mL) at room temperature for 3 days. The mixture was filtered and evaporated, the residue was dissolved in ethyl acetate, filtered and evaporated again to give the ester (7.92g, 100%).

### (c) (3S/R,4R/S)-3,4-Dihydroxy-piperidine,-1,4-dicarboxylic acid 1-tert-butyl ester 4-benzyl ester

To a solution of ester (18b) (6.15g, 19.5 mmol) and N-methylmorpholine N-oxide (4.8g, 41 mmol) in 1:1 acetone/water (160mL) was added osmium tetroxide (1% aqueous solution, 10.3mL, 0.33 mmol). After stirring overnight, solid sodium metabisulphite was added, the mixture was stirred for 30 min, filtered through kieselguhr and partially evaporated. The aqueous residue was acidified to pH1 (dil. HCl) and extracted with ethyl acetate. Extracts were washed with brine, dried and evaporated. Chromatography on silica (20-50% ethyl acetate/petroleum ether) gave the diol (3.07g, 45%).

### (d) (3S/R,4R/S)-3,4-Dihydroxy-piperidine-1,4-dicarboxylic acid 1-tert-butyl ester 4-amide

The diol ester (18c) (3.07g, 8.75 mmol) was dissolved in THF (50mL) and stirred with aqueous ammonia (d=0.88, 50mL) at room temperature for 3 days. Evaporation of solvent and chromatography on silica (0-20% methanol/ethyl acetate) gave the amide (1.16g, 51%).
MS (-ve ion electrospray) m/z 259 (M-H⁻).

### (e) (3S/R,4R/S)-3,4-Dihydroxy-(6-methoxy-[1,5]naphthyridin-4-ylcarbamoyl)-piperidine-1-carboxylic acid tert-butyl ester

This was prepared from the amide (18d) (1.35g, 5.2 mmol) and triflate (1b) (1.63g, 5.2 mmol) by the method of Example (3b, Method B). Chromatography on silica gel (0-10% methanol/dichloromethane) and recrystallisation from dichloromethane gave the coupled product (0.43g, 20%).
MS (+ve ion electrospray) m/z 419 (MH⁺).

### (f) (3S/R,4R/S)-3,4-Dihydroxy-(6-methoxy-[1,5]naphthyridin-4-ylcarbamoyl)-piperidine

The tert-butyl carbamate (18e) (0.42g, 1 mmol) was suspended in dichloromethane (5mL) and treated with trifluoroacetic acid (5mL). The resulting solution was allowed to stand for 2h, then evaporated. The residue was dissolved in a small volume of water, basified with sodium bicarbonate and the precipitate was filtered off, washed with water and dried to give the piperidine (0.29g, 91 %).
MS (+ve ion electrospray) m/z 319 (MH⁺).

### (g) Title compound

The piperidine (18f) (0.13g, 0.41 mmol) was alkylated with the mesylate (1f) (0.12g, 0.42 mmol) by the method of Example 1 (at 60°C rather than room temperature). Chromatography on silica (0-10% methanol/dichloromethane) gave the free base of the title compound (50mg, 24%).
δH (d6-DMSO, 400MHz): 11.06 (1H, s), 10.49 (1H, s), 8.69 (1H, d), 8.50 (1H, d), 8.28 (1H, d), 7.33 (1H, d), 7.22 (1H, d), 6.87 (1H, d), 6.85 (1H, s), 5.89 (1H, s), 5.07 (1H, d), 4.12 (3H s), 4.01 (18, s), 3.96 (1H,s), 3.43 (2H, s), 2.78 (1H, m), 2.67 (3H, m), 2.22 (2H, m), 1.99 (1H, br t), 1.69 (1H, br d).
MS (+ve ion electrospray) m/z 510(MH+).
The dihydrochloride salt was prepared as described for Example 16.

### Example 19 4-R/S-Hydroxy-3-S/R-methoxy-1-[2-(3-ozo-3,4-dihro-2H-benzo[1,4]thiazin-6-yl)-ethyl]-piperidine-4-carbozytic acid (6-methoxy-[1,5]naphthyridin-4-yl)-amide dihydrochloride

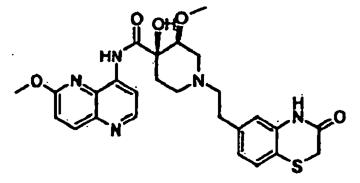

### (a) 1,2,3,6-Tetrahydro-pyridine-1,4-dicarboxylic acid 1-tert-butyl ester 4-methyl ester

The acid (18a) (5.42 g, 24.9 mmol) was esterified by treatment with potassium carbonate (3.84g, 30 mmol) and methyl iodide (1.63mL, 26.3 mmol) in dimethylformamide (60mL) at room temperature overnight. The mixture was filtered and evaporated, the residue was dissolved in ethyl acetate, filtered and evaporated again to give the ester (5.72g, 99%).

### (b) (3S/R,4R/S)-3,4-Dihydroxy-pipendine-1,4-dicaiboxylic acid 1-tert-butyl ester 4-methyl ester

To a solution of ester (19a) (5.72g, 23.7 mmol) and N-methylmorpholine N-oxide (5.84g, 49.9 mmol) in 1:1 acetone/water (160mL) was added osmium tetroxide (4% aqueous solution, 3 12mL, 0.4 mmol). After stirring overnight, the mixture was filtered through kieselguhr and partially evaporated. The aqueous residue was acidified to pH1 (dil. HCl) and extracted with ethyl acetate. Extracts were washed with brine, dried and evaporated. Chromatography on silica (30-60% ethyl acetate/ hexanes) gave the diol (4.8g, 74%).

### (c) 4-R/S-Hydroxy-3-S/R-methoxy-piperidine-1,4-dicarboxylic acid 1-tert-butyl ester 4-methyl ester

Sodium hydride (60% in mineral oil, 0.54g, 13.5 mmol) was washed with anhydrous THF then suspended in THF (60mL). A solution of diol (19b) (azeotropically dried with toluene, 3.37g, 12.27 mmol) in THF (60mL) was then added and the mixture was stirred for 0.5h. To the resulting solution was added dimethyl sulphate (1.15mL, 12.13 mmol) and the mixture was stirred at room temperature for 24h. Solvent was evaporated, the residue was dissolved in ethyl acetate/water, and the aqueous phase was extracted again (EtOAc). Extracts were washed with brine, dried and evaporated. Chromatography (together with crude product from a smaller preparation, 0.25g diol) gave the monomethyl ether (1.14g, 32%) [and some 3,4-dimethyl diether (0.19g, 5%) and recovered diol (1.0g,30%)].
MS (+ve ion APCI) m/z 190 (loss of C₅H₈O₂ from MH+).

### (d) 4-R/S-hydroxy-3-S/R-methoxy-piperidine-1,4-dicarboxylic acid 1-tert-butyl ester

The ester (19c) (1.13g, 3.9 mmol) was hydrolysed by treatment with aqueous 2M sodium hydroxide (5ml, 10mmol) in THF (10mL) at room temperature overnight. The mixture was made mildly acidic (dil. HCl) and extracted three times with ethyl acetate. Extracts were dried and evaporated to give the acid (1.08g, 100%).
MS (-ve ion electrospray) m/z 274 (M-H⁻).

### (e) 4-R/S-hydroxy-3-S/R-rnethoxy-piperidine-1,4-dicarboxylic acid 1-tert-butyl ester 4-amide

To a solution of acid (19d) (1.08g, 3.93 mmol) and N-hydroxysuccinimide (0.45g, 3.93 mmol) in dry ethyl acetate (15mL) was added a solution of 1,3-dicyclohexylcarbodiimide (0.8g, 3.93 mmol) in dry ethyl acetate (3mL). The mixture was stirred for 4h, cooled in ice and filtered, washing through with cold EtOAc. Evaporation of the filtrate gave the succinimide ester (1.23g).This was dissolved in THF (20mL) and gaseous ammonia was bubbled through for 10 min. Further ammonia was passed through after 1.5h, and after another 1h the mixture was evaporated.The residue was dissolved in dichloromethane/water and the aqueous phase was extracted with dichloromethane/methanol. Evaporation of the extracts gave the amide (0.89g, 83% from acid).
MS (+ve ion electrospray) m/z 175 (loss of C₅H₈O₂ from MH+).

### (f) 4-R/S-hydroxy-3-S/R-methoxy-4-(6-methoxy-[1,5]naphthyridin-4-ylcarbamoyl)-piperidine-1-carboxylic acid tert-butyl ester

This was prepared from the amide (19e) (0.89g, 3.25 mmol) and triflate (1b) (1.02g, 3.25 mmol) by the method of Example (3b, Method B). Chromatography on silica gel (0-3% methanol/dichloromethane) gave the coupled product (0.89g, 63%).
MS (+ve ion electrospray) m/z 433 (MH⁺).

### (g) 4-R/S-hydroxy-3-S/R-methoxy-4-(6-methoxy-[1,5]naphthyridin-4-ylcarbamoyl)-piperidine

The tert-butyl carbamate (19f) (0.88g, 2.04 mmol) was suspended in dichloromethane (5mL) and treated with trifluoroacetic acid (5mL). The resulting solution was allowed to stand for 1.75h, then evaporated. The residue was dissolved in a small volume of water, basified with sodium bicarbonate and the precipitate was filtered off, washed with water and dried to give the piperidine (0.83g, 100%).
MS (+ve ion electrospray) m/z 333 (MH⁺).

### (h) Title compound

The piperidine (19g) (0.25g, 0.75 mmol) was alkylated with the mesylate (1f) (0.21g, 0.75 mmol) by the method of Example 1 (at 50°C rather than room temperature), with further additions of mesylate (0.21g) and potassium carbonate (55mg) after 48h followed by overnight heating.. Chromatography on silica (0-5% methanol/dichloromethane) gave the free base of the title compound (106mg, 27%).
δH (CDCl₃/CD₃OD, 250MHz): 8.65 (1H, d), 8.59 (1H, d), 8.18 (1H, d), 7.23 (2H, d), 6.90 (1H, d), 6.84 (1H, s), 4.18 (3H, s), 4.02 (1H, dd), 3.39 (5H, s), 3.17 (1H, dd), 2.83 (3H, m), 2.72 (2H, m), 2.47 (1H, t), 2.31 (1H, t), 2.20 (1H, td), 1.87 (1H, d).
MS (+ve ion electrospray) m/z 524(MH+).
The dihydrochloride salt was prepared as described for Example 16.

### Example 20 4-Cyano-1-[2-(3-oxo-3,4-dihydro-2H-benzo[1,4]thiazin-6-yl)-ethyl]-piperidine-4-carboxylic acid (6-methoxy-[1,5]naphthyridin-4-yl)-amide dihydrochloride

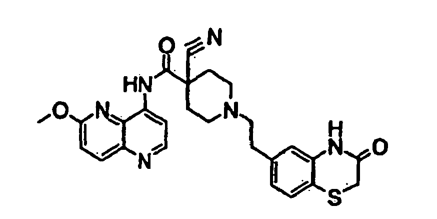

### (a) Bis-(2-chloroethyl)carbamic acid tert-butyl ester

A solution of bis-(2-chloroethyl)amine hydrochloride (50g, 0.28 mol) and di-tert-butyl dicarbonate (61 g, 0.28 mol) in dichloromethane (250mL) was treated with triethylamine (44.2mL) over 20 min, then stirred overnight. The mixture was filtered and evaporated, and the residue was dissolved in water and extracted with ether (X3). Extracts were dried and evaporated to give an oil (66.9g, 99%).

### (b) 4-Cyano-piperidine-1,4-dicarboxylic acid 1-tert-butyl ester 4-methyl ester

This compound was prepared from carbamate (20a) (27.2g, 0.112 mol) and methyl cyanoacetate (10mL, 0.112 mol) by the method of Example (6b). Chromatography on silica (30% ethyl acetate/hexane) gave the pure product (1g).

### (c) 4-Cyano-pipendine-1,4-dicarboxylic acid 1-tert-butyl ester

The ester (20b) (1g) was hydrolysed by the method of Example (19d) (reaction time 4 days) in 53% yield.
MS (-ve ion electrospray) m/z 253 (M-H⁻).

### (d) 4-Cyano-piperidine-1,4-dicarboxylic acid 1-tert-botyl ester 4-amide

This was prepared from the acid (20c) (0.5g, 1.97 mmol) via the succinimide ester by the method of Example (19e).Yield 0.4 g, 93%.

### (e) 4-Cyano-4-(6-mothoxy-[1,5]naphthyridin-4-ylcarbamoyl)-piperidine-1-carboxylic acid 1-tert-butyl ester

This was prepared from the amide (20d) (0.38g, 1.5 mmol) and triflate (1b) (0.47g, 1.5 mmol) by the method of Example (3b, Method B). Chromatography on silica gel (1-3% methanol/dichloromethane) gave the coupled product (0.45g, 73%).
MS (+ve ion APCI) m/z 412 (MH⁺).

### (f) 4-Cyano-4-(6-methoxy-[1,5]naphthyridin-4-ylcarbamoyl)-piperidine

The carbamate (20e) (0.44g, 1.07 mmol) was deprotected by the method of Example (19g) to give the piperidine (0.24g, 72%).
MS (+ve ion APCI) m/z 312 (MH⁺).

### (g) Title compound

The piperidine (20f) (0.112g, 0.36 mmol) was alkylated with the mesylate (1f) (0.103g, 0.36 mmol) by the method of Example 1 (at 80°C rather than room temperature) with potassium carbonate replaced by sodium hydrogen phosphate (2 equivalents). Chromatography on silica (0-3% methanol/dichloromethane) gave the free base of the title compound (0.11g, 61%).
δH (CDCl₃, 250MHz): 10.64 (1H, br s), 8.73(1H, d), 8.44(1H, d), 8.23(1H, d), 7.24(1H, d), 7.19(1H, d), 6.88(1H, dd), 6.74(1H, dd),, 4.15(3H, s), 3.44(2H, s), 3.09(2H, m), 2.77(2H, m), 2.68(2H, m), 2.42 (4H, m), 2.12 (2H, m)
MS (+ve ion electrospray) m/z 503(MH+).
The dihydrochloride salt was prepared as described for Example 16.

### Example 21 1-[2-(2,3-Dihydro-benzo[1,4]dioxin-6-yl)-2-R,S-hydroxy-ethyl]-piperidine-4,4-dicarboxylic acid amide (6-methoxy-[1,5]naphthyridin-4-yl)-amide dihydrochloride

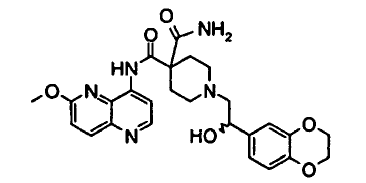

### (a) Piperidine-1,4,4-tricarboxylic acid 1-tert-butyl ester 4,4-dimethyl ester

This compound was prepared from carbamate (20a) (13.4g, 55.3 mmol) and dimethyl malonate (7.3g, 55.3 mmol) by the method of Example (6b). Chromatography on silica (20-30% ethyl acetate/hexane) gave an oil (1.73g, 10%).
MS (+ve ion APCI) m/z 202 (loss of C₅H₈O₂ from MH+).

### (b) Piperidine-1,4,4-tricarboxylic acid 1-tert-butyl ester 4,4-diamide

The dimethyl ester (21a) (1.05g, 3.5 mmol) was stirred in aqueous ammonia (d=0.88, 10mL) at room temperature for 3 days. Evaporation and trituration with dichloromethane gave a white solid (0.76g, approx. 65% pure).
MS (-ve ion electrospray) m/z 270 (M-H⁻).

### (c) 4-(6-methoxy-[1,5]naphthyridin-4-ylcarbamoyl)-piperidine-1,4-dicarboxylic acid 1-tert-butyl ester 4-amide

This was prepared from the amide (21b) (approx. 65% pure ,0.76g, 1.8 mmol) and triflate (1b) (0.55g, 1.8 mmol) by the method of Example (3b, method B).
Chromatography on silica gel (2-5% methanol/dichloromethane) gave the coupled product (0.33g, 43%).
MS (+ve ion APCI) m/z 430 (MH⁺).

### (d) 4-(6-methoxy-[1,5]naphthyridin-4-ylcarbamoyl)-piperidine-4-carboxylic acid amide

A solution of amide (21c) (0.32g, 0.75 mmol) in dichloromethane (5mL) was treated with trifluoroacetic acid at room temperature. After 2.5h the mixture was evaporated, the residue was basified with sodium bicarbonate, evaporated again and the residue adsorbed onto silica from dichloromethane/methanol. Chromatography on silica (10% methanol/dichloromethane) gave a solid (0.74g, >100%), probably containing sodium trifluoroacetate.

### (e) Title compound

This was prepared from piperidine (21d) (0.30g, 0.30 mmol) and 6-(2-bromoethanoyl)-2,3-dihydro-benzo[1,4]dioxin (64mg, 0.30mmol) by the method of Example 13. Chromatography on silica (5% methanol/dichloromethane) gave the free base (90mg, 59%).
δH (CDCl₃): 10.43 (1H, s), 8.70 (1H, d), 8.43 (1H, d), 8.21 (1H, d), 7.16 (1H, d), 6.88(1H, s), 6.82 (2H, s), 6.14 (1H, br s), 5.56 (1H, br s), 4.63 (1H, t), 4.24 (4H, S), 4.16 (3H, s), 2.87 (2H, m), 2.59 (2H, m) 2.48 (2H, d), 2.39 (4H, m)
MS (+ve ion APCI) m/z 530 (MNa+).
The dihydrochloride salt was prepared as described for Example 16.

### Example 22 4-Hydroxy-1-[2-(RS)-2-hydroxy-2-(3-oxo-3,4-dihydro-2H-pyrido[3,2-b][1,4]thiazin-6-yl)-ethyl]-piperidine-4-carboxylic acid (6-methoxy-[1,5]naphthyridin-4-yl) amide dihydrochloride

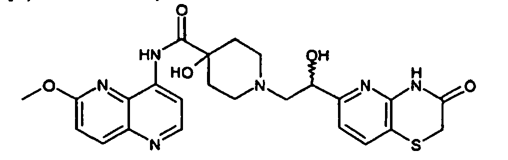

### (a) Methyl 3-oxo-3,4-dihydro-2H-pyrido[3,2-b][1,4]thiazine-6-carboxylate

A solution of ethyl 2-mercaptoacetate (1.473ml) in DMF (48ml) was ice-cooled and treated with sodium hydride (540mg of a 60% dispersion in oil). After 1 hour methyl 6-amino-5-bromopyridine-2-carboxylate (3g) (T. R. Kelly and F. Lang, J. Org. Chem. 61, 1996, 4623-4633) was added and the mixture stirred 16 hours at room temp. The solution was diluted with EtOAc (1 litre), washed with water (3x300ml), dried and evaporated to about 10ml. The white solid was filtered off and washed with a little EtOAc (0.95g).
MS (APCI-) *m*/*z* 223 ([M-H]-, 100%)

### (b) 3-Oxo-3,4-dihydro-2H-pyrido[3,2-b][1,4]thiazine-6-carboxylic acid

A solution of the ester (22a) (788mg) in dioxan (120ml)/water (30ml) was treated dropwise over 2 hours with 0.5M NaOH solution (8ml) and stirred overnight. After evaporation to approx. 3ml, water (5ml) was added and 2N HCl to pH4. The precipitated solid was filtered off, washed with a small volume of water and dried under vacuum (636mg).
MS (APCI-) *m*/*z* 209 ([M-H]-,5%), 165([M-COOH]-, 100%)

### (c) 6-Chloroacetyl-3-oxo-3,4-dihydro-2H-pyrido[3,2-b][1,4]thiazine

A suspension of the carboxylic acid (22b) (500 mg) in dry THF (20 ml) was treated with triethylamine (0.396 ml) and stirred 5 minutes under argon. The solution was cooled to -10°C, treated with isobutyl chloroformate (0.339 ml), stirred 20 mins. and filtered through kieselguhr. The filtrate was cooled to -20°C, treated with excess diazomethane solution in ether and allowed to warm to room temperature overnight. It was evaporated to dryness and the residue dissolved in dichloromethane (20 ml), treated with 5N HCl (10 ml) and stirred vigorously 2 hours. After ice-cooling, sodium bicarbonate was added until the mixture was basic and the solid filtered off, washed with water and dried.The layers of the filtrate were separated and the aqueous re-extracted with dichloromethane (4x10 ml). The organic was dried and evaporated to give a solid. The solids were combined to give the product (0.53 g).
MS (APCI⁺) m/z 243 (MH⁺, 100%)

### (d) Title compound

A suspension of piperidine (3c) (100 mg) in DMF (3 ml) was ice-cooled, treated with potassium carbonate (46 mg) and chloromethyl ketone (22c) (80 mg). After stirring 3 hours at room temp., water (30 ml) was added and the solid filtered, dried by suction and dissolved in dichloromethane/methanol (1:1, 20 ml). To the ice-cooled solution was added sodium borohydride (25 mg), and after stirring for 2 hours it was acidified slightly with dilute HCl and evaporated to low volume. Aqueous sodium bicarbonate (10 ml) was added and the mixture extracted with 10% ethanol/chloroform (4x20 ml). The organic extract was dried and evaporated and the residue chromatographed on silica gel (eluent chloroform/methanol/0.88 ammonia 95:5:0.5) to give free base of title compound (75 mg).
¹H NMR δ (CDCl₃) 1.79 (2H, dt), 2.3-2.6 (4H, m), 2.69 (1H, dt), 2.75-2.85 (2H, m), 2.96 (1H, broad s),3.07 (1H, d), 3.49 (2H, s), 4.08 (3H, s), 4.74 (1H, dd), 7.14 (1H, d), 7.22 (1H, d), 7.64 (1H, d), 8.22 (1H, d), 8.28 (1H, broad s), 8.52 (1H, d), 8.70 (1H, d).
A solution of the free base in 1:1 chloroform/methanol (5 ml) was treated with 1M HCl in ether (0.4 ml) and evaporated to give title compound.
MS (+ve ion electrospray) m/z 511 (MH⁺, 100%)

### Example 23 4-Methoxy-1-[2-(RS)-2-hydroxy-2-(3-oxo-3,4-dihydro-2H-pyrido[3,2-b][1,4]thiazin-6-yl)-ethyl]-piperidine-4-carboxylic acid (6-methoxy-[1,5]naphthyridin-4-yl) amide dihydrochloride

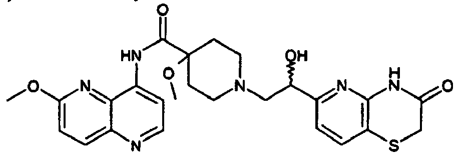

This was prepared by reaction of piperidine (16d) with chloromethyl ketone (22c), followed by reaction with sodium borohydride, by the method of Example (22d) to give title compound (49%).
¹H NMR (free base) δ (CDCl₃) 1.96 (2H, dt), 2.2-2.35 (2H, m), 2.47 (1H, dt), 2.5-2.85 (4H, m), 3.02 (1H, d), 3.41 (3H, s), 3.49 (2H, s), 4.13 (3H, s), 4.76 (1H, dd), 7.17 (1H, d), 7.23 (1H, d), 7.64 (1H, d), 8.24 (1H, d), 8.50 (1H, d), 8.61 (1H, broad s), 8.73 (1H, d).
MS (HCl salt) (+ve ion electrospray) m/z 525 (MH⁺, 100%)

### Example 24 1-[2-(6-Oxo-6,7-dihydro-5H-8-thia-1,2,5-triazanaphthalen-3-yl)ethyl]-4-trifluoromethylpiperidine-4-carboxylic acid (6-metboxy-[1,5]naphthyridin-4-yl) amide dihydrochloride

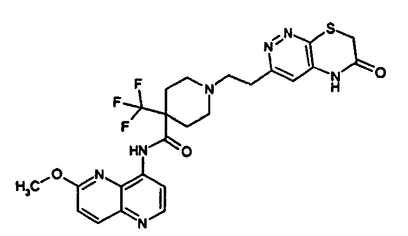

### (a) Methyl 1-t-butoxycarbonyl-4-trifluoromethylpiperidine-4-carboxylate

A solution of 1,1,1,3,3,3 hexamethyldisilazane (6.94 g) in THF (150 ml)/1,3-dimethyl-3,4,5,6-tetrahydro-2(1*H*)-pyrimidinone (25 ml) was cooled under argon to -20°C and treated with n-BuLi (18.9 ml of 2.5M solution in hexane). After 10 mins.it was cooled to -78°C and a solution of methyl 1-t-butoxycarbonylpiperidine-4-carboxylate (9.5 g) in THF (20 ml) added. After 1 hour 5-(trifluoromethyl)dibenzothiophenium trifluoromethanesulfonate (17.3 g) was added, the reaction allowed to warm to room temp. and stirred 3 days. Excess aqueous citric acid was added, followed by EtOAc (600 ml). The solution was washed with water (3x400 ml), dried and evaporated.
Chromatography on silica gel gave the product (2.65 g).
MS (+ve ion electrospray) *m*/*z* 238 ([M-tBuO]⁺, 100%)

### (b) 1-t-Butoxycarbonyl-4-trifluoromethylpiperidine-4-carboxylic acid

A solution of ester (24a) (2.65 g) in THF (15 ml) was treated with 2N NaOH solution (4.7 ml), stirred overnight at room temp. and for 24 hours at 60°C, diluted with water (20 ml) and washed with ether (30 ml).The aqueous was acidified to pH2 with dilute HCl and extracted with EtOAc (3x20 ml). The organic fraction was dried and evaporated to give the product as a white solid (2.48 g).

### (c) 1-t-Butoxycarbonyl-4-trifluoromethylpiperidine-4-carboxamide

A solution of the carboxylic acid (24b) (2.48 g) in EtOAc (25 ml) was treated with N-hydroxysuccinimide (0.97 g) and a solution of dicyclohexylcarbodiimide (1.72 g) in EtOAc (5 ml) and stirred overnight. The mixture was filtered, the filtrate evaporated and the residue taken up in THF (25 ml). Ammonia gas was bubbled through for 15 mins., the solvent evaporated and water (25 ml) added. The aqueous was extracted with 10% methanol/dichloromethane (3x25 ml), the organic fraction dried and evaporated to give product (2.37 g).

### (d) 1-t-Butoxycarbonyl-4-trifluoromethylpiperidine-4-carboxylic acid (6-methoxy-[1,5]naphthyridin-4-yl) amide

A mixture of the carboxamide (24c)(1.0 g), tris(dibenzylideneacetone)dipalladium(0) (70 mg), racemic 2,2'-bis(diphenylphosphino)-1,1'-binaphthyl (140 mg), caesium carbonate (1.65 g) and dioxan (45 ml) was purged with argon for 10 mins., held in an ultrasonic bath for 5 mins. and treated with triflate (1b) (1.05 g). The mixture was heated under argon at 80°C overnight, filtered through kieselguhr and evaporated. Chromatography on silica gel (30% EtOAc/hexane) gave the product (730 mg).
MS (+ve ion electrospray) m/z 355 ([M-BOC+2H]⁺, 100%)

### (e) 4-Trifluoromethylpiperidine-4-carboxylic acid (6-methoxy-[1,5]naphthyridin-4-yl) amide

The amide (24d) (730 mg) in dichloromethane (30 ml) was treated with TFA (20 ml), left 2 hours at room temp. and evaporated. Excess aqueous sodium bicarbonate was added and the mixture extracted with 10% methanol/chloroform (2x 20 ml). The organic fraction was dried and evaporated to give the product (502 mg).
MS (+ve ion electrospray) *m*/*z* 355 (MH⁺, 100%)

### (f) Title compound

A mixture of the amine (24e) (144 mg) and 3-vinyl-pyridazinothiazine (14c) (78 mg) in ethanol (3 ml)/acetic acid (0.04 ml) was refluxed overnight. Aqueous sodium bicarbonate (20 ml) was added and the mixture extracted with 10% methanol/chloroform (3x20 ml). The organic extract was dried and evaporated and the residue chromatographed (chloroform/methanol/0.88 ammonia 97:3:0.3) to give free base of the title compound (80 mg).
¹H NMR δ (CDCl₃/CD₃OD) 2.12 (2H, dt), 2.32 (2H, broad t), 2.43 (2H, broad d), 2.77 (2H, t), 3.0-3.1 (4H, m), 3.61 (2H, s), 4.09 (3H, s), 6.81 (1H, s), 7.22 (1H, d), 8.23 (1H, d), 8.49 (1H, d), 8.70 (1H, d).
The free base in chloroform (5 ml) was treated with 1M HCl in ether (0.5 ml) and evaporated to give title compound as the hydrochloride salt.
MS (APCI+) *m*/*z* 548 (MH⁺).

### Example 25 1-[2-(2,3-Dihydro-[1,4]dioxino[2,3-c]pyridin-7-yl)-ethyl]4-hydroxy-piperidine-4-carboxylic acid (6-methoxy-[1,5]naphthyridin-4-yl)-amide hydrochloride

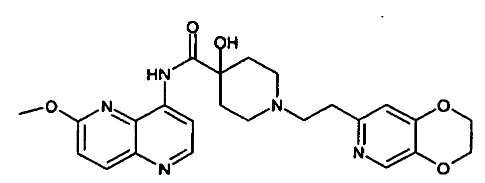

### (a) 5-Benzyloxy-2-hydroxymethyl-1H-pyridin-4-one

A mixture of 5-benzyloxy-2-hydroxymethyl-4-pyrone (prepared from Kojic acid by the method of D. Erol, J. Med. Chem., 1994, 29, 893) (9.7g, 40 mmol), concentrated aqueous (880) ammonia (100 ml), and ethanol (20 ml) was heated to reflux overnight. The mixture was allowed to cool to room temperature then filtered. The resultant solid was washed with ether and dried *in vacuo* (5.9g).
MS (+ve ion electrospray) *m*/*z* 232 (MH+).

### (b) (2,3-Dihydro-[1,4]dioxino[2,3-c]pyridin-7-yl)-methanol

A solution of (25a) (2g, 8.7 mmol) in water (220 ml) containing sodium hydroxide (17 mmol) was hydrogenated over 10% palladium on charcoal (1g) for 4 hours. The mixture was filtered and evaporated to give a white solid. This solid was dissolved in N,N-dimethylformamide (8 ml) then treated with potassium carbonate (2.9g) and 1,2-dibromoethane (0.6 ml, 7 mmol). The mixture was heated at 85°C overnight. The cooled mixture was evaporated onto silica and chromatographed eluting with 10-30% methanol in ethyl acetate affording a white solid (250 mg, 21 %).
MS (+ve ion electrospray) *mlz* 232 (MH+).

### (c) 2,3-Dihydm-[1,4]dioidno[2,3-c]pyridine-7-caiboxaldehyde

A solution of (25b) (250mg, 1.5 mmol) in dichloromethane (5 ml) was stirred with manganese dioxide (650 mg, 7.5 mmol). After 3 days the mixture was filtered and evaporated affording a white solid (150 mg, 61 %).
MS (+ve ion electrospray) *m*/*z* 166 (MH+).

### (d) 1-(2,3-Dihydro-[1,4]dioxino[2,3-c]pyridin-7-yl)-2-trimethylsilanyl-ethanol

A solution of aldehyde (25c) (1.2g) in tetrahydrofuran (20 ml) was treated at 0°C under argon with a solution of trimethylsilylmethylmagnesium chloride in ether (1M; 8 ml, 8 mmol). After 2 hours the mixture was partitioned between ether - half saturated aqueous ammonium chloride solution. The organic extract was dried and evaporated affording an oil (1.6g).
MS (+ve ion electrospray)*m*/*z* 254 (MH+).

### (e) 7-Vinyl-2,3-dihydro-[1,4]dioxino[2,3-c]pyridine

A solution of (25d) (1.6g) in tetrahydrofuran (20 ml) was treated at 0°C under argon with a solution of potassium t-butoxide in tetrahydrofuran (1M; 7.6 ml, 7.6 mmol). After 2 hours the mixture was partitioned between ethyl acetate - half saturated aqueous ammonium chloride solution. The organic extract was dried and evaporated affording an oil (1.5g).
MS (+ve ion electrospray) *m*/*z* 164 (MH+).

### (f) Title compound

A mixture of amine (3c) (302 mg, 1 mmol), olefin (25e) (163 mg, 1 mmol), acetic acid (60 mg) and *n*-butanol (1 ml) was heated to reflux. After 2 hours, ethanol (2 ml)and N,N-dimethylformamide (2 ml) were added and the mixture heated to reflux for 24 hours. The mixture was partitioned between ethyl acetate/half saturated aqueous sodium bicarbonate solution The organic extract was dried and evaporated Chromatography on silica eluting with a 0-10% methanol in ethyl acetate gradient afforded a white solid (100 mg, 21%).
δH(CDCl3, 200MHz): 10.90 (1H, br), 8.72 (1H, d), 8.55 (1H, d), 8.20 (1H, d) 8.05 (1H, s), 7.15 (1H, d), 6.72 (1H, s), 4.80 (1H, dd), 4.35 (2H, m), 4.25 (2H, m), 4.15 (3H, s), 3.00-2.85 (4H, m), 2.80 (2H, m), 2.45-2.30 (4H, m), 1.75-1.70 (2H, m).
MS (+ve ion electrospray) *m*/*z* 466 (MH+).
This material was converted to the hydrochloride salt by the method of Example 13.

### Example 26 1-[2-(2,3-Dibydro-[1,4]dioxino[2,3-c]pyridin-7-yl)-etbyl]-4-hydroxy-piperidine-4-carboxylic acid (8-fluoro-6-methoxy-quinolin-4-yl)-amide hydrochloride

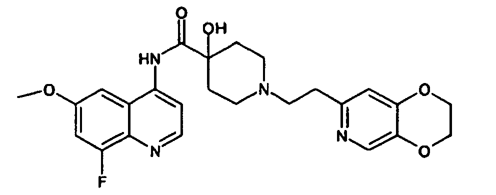

### (a) 8-Fluoro-6-methoxy-quinolin-4-ol

A mixture of 2-fluoro-4-methoxy-phenylamine (3.80g, 26.7mmol) and methyl propiolate (2.37ml, 0.267mol) in methanol (100ml) was stirred for 72 hours at room temperature, then heated at 50°C for 24 hours. It was evaporated and the product purified by chromatography on silica gel (dichloromethane) to give a solid (1.66g), a portion of which was recrystallised from dichloromethane-hexane. The unsaturated ester (0.96g) in warm Dowtherm A (5ml) was added over 3 minutes to refluxing Dowtherm A (15ml), and after a further 20 minutes at reflux the mixture was cooled and poured into diethyl ether. The precipate was filtered to give the title compound (0.50g, 61 %)

### (b) 1,1,1-Trifluoro-methanesulfonic acid 8-fluoro-6-methoxy-quinolin-4-yl ester

The pyridone (26a) (0.48g) and dimethylaminopyridine (0.03g) in dichloromethane (20ml) and 2,6-lutidine (0.48ml) was treated dropwise with triflic anhydride (0.48ml) and the mixture was stirred at room temperature for 4 hours. It was washed with saturated ammonium chloride, dried, evaporated, and chromatographed on silica gel (dichloromethane) to afford a yellow solid (0.69g).
MS (+ve ion electrospray) m/z 326 (MH+).

### (c) 8-Fluoro-6-methoxy-quinolin-4-ylamine

A solution of triflate (26b) (0.69g) in pyridine (10ml) was treated with n-propylamine hydrochloride (1.2g) and the mixture heated at reflux for 16 hours. The reaction mixture was evaporated, dissolved in 0.05M HCl, washed with dichloromethane, basified with sodium hydroxide solution and re-extracted with dichloromethane. The combined organic phases were dried over sodium sulfate, evaporated, and chromatographed on silica gel (2-5% methanol in dichloromethane) to afford an orange solid (1.0g).
MS (+ve ion electrospray) m/z 193 (MH⁺).

### (d) 4-Methoxymethoxy-piperidine-1,4-dicarboxylic acid 1-tert-butyl ester 4-methoxymethyl ester

Methoxymethyl chloride (22.8 ml, 300 mmol) was added at 0°C to a stirred mixture of 4-hydroxy-piperidine-1,4-dicarboxylic acid mono-*tert*-butyl ester (24.5g, 100 mmol) and diisopropylethylamine (17.4 ml, 300 mmol) in dichloromethane (250 ml). The mixture was heated to reflux overnight then extracted with dilute aqueous hydrochloric acid. The organic extract was dried and evaporated. The residue was chromatographed on silica eluting with an ethyl acetate in petrol gradient affording an oil (4.61g).
MS (+ve ion electrospray) *mlz* 334 (MH+)

### (e) 4-Methoxymethoxy-piperidine-1,4-dicarboxylic acid mono-tert-butyl ester

A mixture of (26d) (6.1g), tetrahydrofuran (250 ml), and 2M aqueous sodium hydroxide solution (125 ml) was stirred at room temperature. After 2 hours dioxan (50 ml) was added and the mixture heated to 40°C for a further 4 hours, then acidified with 5M aqueous hydrochloric acid solution and extracted with ethyl acetate. The organic extracts were dried and evaporated affording an oil (5.3g).
MS (+ve ion electrospray) m/z 290 (MH+)

### (f) 4-(8-Fluoro-6-methoxy-quinolin-4-ylcarbamoyl)-4-methoxymethoxy-piperidine-1-carboxylic acid tert-butyl ester

A solution of amine (26c) (840 mg, 4.4 mmol), carboxylic acid (26e) (1.3g, 4.4 mmol), triethylamine (1.22 ml, 8.8.mmol), and O-(7-azabenzotriazol-1-yl)-N,N,N',N'-tetramethyluronium hexafluorophosphate (HATU) (1.7g, 4.4 mmol) in N,N-dimethylformamide (10 ml) was heated at 80°C for 16 hours. After this period the solvent was removed under vacuum and the residue partitioned between ethyl acetate and brine. The organic phase was dried over magnesium sulfate and evaporated. The resulting oil was purified by column chromatography on silica gel using a dichloromethane and methanol solvent gradient affording a white foam (310 mg, 15%).
MS (+ve ion electrospray) *m*/*z* 464 (MH+).

### (g) 4-Hydroxy-piperidine-4-carboxylic acid (8-fluoro-6-methoxy-quinolin-4-yl)-amide

A solution of (26f) (310 mg) in trifluoroacetic acid/dichloromethane (20 ml/20 ml) was stirred at room temperature for 4 hours then evaporated to dryness, azeotroping with chloroform. The residue was dissolved in water and basified with saturated aqueous sodium hydrogen carbonate solution. The resulting precipitate was isolated by centrifugation affording a white solid (210 mg).
MS (+ve ion electrospray) *m*/*z* 320 (MH+).

### (h) Title compound

This was prepared from amine (26g) (200 mg) and olefin (25e) (300 mg) by the procedure of Example (25f) to afford the free base of the title compound as a white solid (105mg,31%).
δH(CDCl3, 200MHz): 8.70 (1H, d), 8.35 (1H, d), 8.00 (1H, s), 7.15 (1H, d), 6.90 (1H, m), 6.80 (1H, s), 4.35 (2H, m), 4.25 (2H, m), 3.95 (3H, s), 3.00-2.85 (4H, m), 2.80 (2H, m), 2.45-2.30 (4H, m), 1.8-1.72(2H, m).
MS (+ve ion electrospray) m/z 483 (MH+).

This material was dissolved in N,N-dimethylformamide-methanol-chloroform (10 m-10ml-5ml) and treated with 1M hydrochloric acid in ether (4 ml). The mixture was concentrated to ca. 10 ml and added to ether (40 ml). The title compound was isolated by centrifugation as a white solid (110 mg).

### Example 27 6-((R,S)-1-Hydroxy-2-{4-hydroxy-4-[2-(6-methoxy-[1,5]naphthyridiu-4-yl)ethyl]piperidin-lyl}ethyl)-4H-benzo[1,4]thiazin-3-one

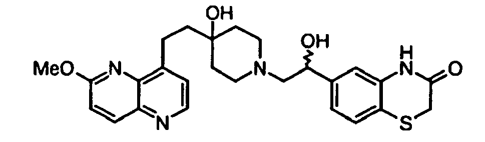

### (a) 4-Hydroxy-4-trimethylsilanylethynylpiperidine-1-carboxylic acid benzyl ester

To a stirred -78 °C solution of trimethylsilylacetylene (3.6 mL, 25.7 mmol; Aldrich) in THF (20 mL) was added n-butyl lithium (16 mL, 25.7 mmol). The reaction was maintained at this temperature for 30 minutes. A solution of 1-(benzyloxycarbonyl)-4-piperidinone (5 g, 21.4 mmol) in THF (40 mL) was added via dropwise addition while maintaining the temperature at -78 °C. After 1 h, the reaction solution was warmed to room temperature and the reaction was quenched with aq NH₄Cl and diluted with EtOAc. The organic layer was washed with brine and dried (Na₂SO₄). Evaporation provided 7.3 g (100%) of the product as an oil, which was used without additional purification.
LC/MS (+ve ion electrospray): *m*/*z* 332 [M+H]⁺.

### (b) 4-Ethynyl-4-hydroxypiperidine-1-carboxylic acid benzyl ester

The ester (27a) (7.1 g, 21.4 mmol) in MeOH (200 mL) was treated with K₂CO₃ (8.8 g, 64 mmol) and vigorously stirred at room temperature for 4 h. The solvent was removed *in vacuo* and the product was extracted into EtOAc. The combined organic extracts were washed with water and dried (Na₂SO₄). Evaporation of solvent yielded 4.9 g (89%) as a solid which was used without additional purification.
LC/MS (+ve ion electrospray): m/z 260 [M+H]⁺.

### (c) 4-Hydroxy-4-(6-methoxy-[1,5]naphthyridin-4-ylethynyl)piperidine-1-carboxylic acid benzyl ester

A stirred solution of the ester (27b) (1.0 g, 3.9 mmol) and triflate (1b) (1.0 g, 3.2 mmol) in a 1:1 mixture of triethylamine and DMF (20 mL total volume) was treated with (Ph₃P)₂PdCl₂ (0.1 g; 4% mol) and CuI (0.06 g, 8% mol). The resulting mixture was heated at 70 °C for 24 h. The solvent was removed under reduced pressure and the residue was purified on an ISCO Combiflash^{®} automated column chromatography unit (silica, 0% to 5% MeOH/CHCl₃) to provide 1.14 g (84%) of the desired product as an oil. LC/MS (+ve ion electrospray): m/z 418 [M+H]⁺.

### (d) 4-[2-(6-Methoxy-[1,5]naphthyridin-4-yl)ethyl]piperidin-4-ol

A stirred solution of ester (27c) (1.14 g, 2.7 mmol) in MeOH (30 mL) was treated with 10% Pd/C (0.5 g) and stirred under an atmosphere of H₂ (50 psi) for 6 h. The reaction mixture was filtered through a pad of Celite^{®} and the solvent was evaporated to give 0.65 g (84%) of desired material as a solid which was used without additional purification.
LC/MS (+ve ion electrospray): *m*/*z* 288 [M+H]⁺.

### (e) (4-Acetyl-2-nitrophenylsulfanyl)acetic acid methyl ester

To a stirred solution of 4-chloro-3-nitroacetophenone (8.37 g, 0.042 mol) and methyl thioglycolate (1.92 g, 0.016 mol) in dimethylformamide (30 mL) was added anhydrous K₂CO₃ (0.048 mol, 6.23g). After stirring at ambient temperature for 16 h, the reaction was quenched with ice water. The precipitated product was filtered, washed well with water and dried under vacuum to give a bright yellow solid (11.08 g, 97%).
LC/MS (+ve ion electrospray) *m*/*z* 270.2 [M+H]⁺.

### (f) 6-Acetyl-4H-benzo[1,4]thiazin-3-one

To a stirred solution of the ester (27e) (12.6 g, 0.047 mol) in glacial acetic acid (210 mL) was added iron powder (26.5 g, 0.473 g atom). After heating at 60 °C for 6 h, the warm mixture was filtered and the filtrate concentrated under reduced pressure. The residue was partitioned between EtOAc and aqueous sodium chloride, and the organic layer was dried (MgSO₄). Purification by recrystallization (EtOAc) gave an off-white solid (7.96 g, 82%).
LC/MS (+ve ion electrospray) *m*/*z* 208.0 [M+H]⁺.

### (g) 6-(2-Chloroethanoyl)-4H-benzo[1,4]thiazin-3-one

To a stirred solution of the benzothiazin-3-one (27f) (2.49 g, 0.012 mol) in a mixture of dichloroethane(7.3mL) and 6:1 glacial acetic acid:water(28mL) was added benzyltrimethylammonium dichloroiodate (9.4g, 0.024 mol). After heating at 65 °C for 3 h, the cooled reaction was diluted with EtOAc and washed with saturated aqueous sodium bisulfite until clear. The organic layer was washed well with water, aqueous sodium chloride and dried (MgSO₄). The product, which precipitated during this process, was collected by filtration, dried under vacuum and combined with that obtained by extraction to give a yellow powder (2.83g, 94%).
LC/MS (+ve ion electrospray) *m*/*z* 242.0 [M+H]⁺.

### (h) Title compound

To a stirred solution of the piperidine (27d) (250 mg, 0.87 mmol) in DMF (5 mL) was added K₂CO₃ (132 mg, 0.96 mmol) followed by the benzothiazinone (27g) (230 mg, 0.96 mmol). The reaction mixture was stirred at room temperature for 2 h. NaBH₄ (300 mg, 7.9 mmol) was added and the reaction was stirred an additional 4 h at room temperature. The reaction was quenched with aq NH₄Cl and diluted with EtOAc. The organic layer was washed with water and brine and dried (Na₂SO₄). The crude product was purified by flash column chromatography on silica gel (gradient: 50-60% EtOAc/hexane) to afford the title compound (85 mg, 20%) as a foamy solid.
¹H NMR (400 MHz, MeOH-d₄) δ 8.7 (d, 1H, J₁ = 8Hz), 8.2 (dd, J₁ = 12Hz, 1H), 7.6 (d, J₁ = 8Hz, 1H), 7.35 (d, J₁ = 10Hz, 1H), 7.25 (d, J₁ = 12Hz, 1H), 7.15 (m, 2H), 4.8 (m, 1H), 4.1 (s, 3H), 3.4 (s, 2H), 2.8 (m, 5H) 1.8 (m, 5H) 1.4 (m, 4H).
LC/MS (+ve ion electrospray): *m*/*z* 495 [M+H]⁺.

### Example 28 6-(2-{4-hydroxy-4-[2-(6-methoxy-[1,5]naphthyridin-4-yl)ethyl]piperidin-1yl}ethyl)-4H-benzo[1,4]thiazin-3-one

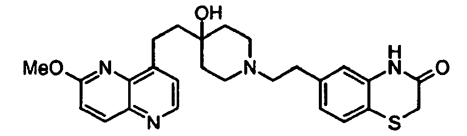

### (a) 3-Oxo-3,4-dihydro-2H-benzo[1,4]thiazine-6-carboxylic acid

3-Oxo-3,4-dihydro-2H-benzo[1,4]thiazine-6-carboxylic acid methyl ester (6.74g) was suspended in tetrahydrofuran (100mL) and 2M sodium hydroxide (30mL) was added followed by water (20mL). The solution was stirred for 2.5 hours, evaporated to half volume and acidified with 2M hydrochloric acid. The product was collected, washed with water and dried *in vacuo,* to give a white solid (6.2g).
MS (-ve ion electrospray) m/z 208 (M-H)⁻

### (b) 6-Hydroxymethyl-4H-benzo[1,4]thiazin-3-one

Acid (28a) in tetrahydrofuran (50mL) and triethylamine (4.7mL) was cooled to 0°C and isobutylchloroformate (4.02mL) was added dropwise and the solution was stirred at 0°C for 2 hours, when it was filtered into a stirred solution of sodium borohydride (3.14g) in ice/water (50mL). The mixture was stirred at 0°C for 1 hour and allowed to warm to room temperature. It was acidified with 2M hydrochloric acid, evaporated to half volume, and the resulting product was collected, washed with water and dried *in vacuo,* to give a white solid (4.5g).
MS (-ve ion electrospray) m/z 194 (M-H)⁻

### (c) 3-Oxo-3,4-dihydro-2H-benzo[1,4]thiazine-6-carboxaldehyde

A stirred solution of alcohol (28b) (3.5g) in chloroform (150mL) and tetrahydrofuran (300mL) was treated with manganese dioxide (7.8g) for 18 hours and was filtered and evaporated to give a white solid (2.5g).
MS (-ve ion electrospray) m/z 194 (M-H)⁻

### (d) 6-Vinyl-4H-benzo[1,4]thiazin-3-one

To a stirred suspension of triphenylmethylphosphonium bromide (20.0 mmole) in dry THF (40 mL) at RT was added 2.5 M n-BuLi (7.5 mL, 3.0 mmole). After 3h the carboxaldehyde (28c) (1.93 g, 10.0 mmole) was added and the reaction contents were stirred at RT overnight. The reaction solution was filtered and the filtrate concentrated, dissolved in EtOAc and washed with 1M HCl (20mL). The organic solution was dried (Na₂SO₄) and concentrated under vacuum. Purification on silica (EtOAc/hexanes, 1:4) afforded the title compound (1.20 g, 63%) as light yellow solid.
LC/MS (+ve ion electrospray): *m*/*z* 192 (M+H)⁺.

### (e) 6-(2-Hydroxyethyl) 4H-benzo[1,4]thiazin-3-one

To a stirred solution of the benzothiazinone (28d) (5.73 g, 30.0 mmole) in dry THF (100 mL) at RT was added 2M BH₃·THF (7.5 mL, 15.0 mmole). After 24h, H₂O (15 mL) was slowly added to the reaction mixture followed by 3M NaOH (5 mL) and 30% H₂O₂ (3.3 mL). After the reaction solution was stirred for 3h at 50 °C, the reaction was concentrated under vacuum. The residue was dissolved in EtOAc and washed with H₂O (20 mL) and 1M HCl (20mL). The organic solution was dried (Na₂SO₄), concentrated under vacuum, and the remaining solid purified on silica (CH₂Cl₂/CH₃OH, 95:5) to afford a light yellow solid (2.60 g, 41%).
LC/MS (+ve ion electrospray): *m*/*z* 210 (M+H)⁺.

### (f) Toluene-4-sulfonic acid 2-(3-oxo-3,4-dihydro-2H-benzo[1,4]thiazin-6-yl)ethyl ester

To a stirred solution of the alcohol (28e) (2.09 g, 10.0 mmole) in dry pyridine (25 mL) at RT was added *p*-toluenesulfonyl chloride (10.0 mmole). After 24h, the reaction solution was concentrated under vacuum. Purification on silica (EtOAc/hexanes, 1:1) afforded a light yellow solid.(0.91 g, 47%).
LC/MS (+ve ion electrospray): *m*/*z* 192 (M+H)⁺.

### (g) Title compound

To a stirred solution of the piperidine (27d) (100 mg, 0.35 mmol) in DMF (3 mL) was added K₂CO₃ (52 mg, 0.38 mmol) followed by the tosylate (28f) (139 mg, 0.38 mmol). The reaction mixture was stirred at room temperature for 12 h. The reaction was quenched with aq NH₄Cl and diluted with EtOAc. The organic layer was washed with water and brine and dried (MgSO₄). The crude product was purified by flash column chromatography on silica gel (gradient: 20-50% EtOAc/hexane) to afford the title compound (91 mg, 54%) as a foamy solid.
¹H NMR (400 MHz, MeOH-d₄) δ 8.7 (d, 1H, J₁ = 8Hz), 8.2 (dd, J₁ = 12Hz, 1H), 7.5 (d, J₁ = 8Hz, 1H), 7.25 (m, 2H), 6.8 (m, 2H), 4.1 (s, 3H), 3.3 (s, 2H), 2.8 (m, 8H) 1.8 (m, 8H).
*LC*/*MS* (+ve ion electrospray): *m*/*z* 479 [M+H]⁺.

### Example 29 6-(2-{4-Fluoro-4-[2-(6-methoxy-[1,5]naphthyridin-4-yl)ethyl]piperidin-1yl}ethyl)-4H-benzo[1,4]thiazin-3-one

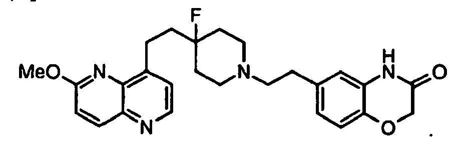

### (a) 4-Ethynyl-4-fluoropiperidine-1-carboxylic acid benzyl ester

This compound was prepared according to the methods reported by van Niel and Collins, *et. al., J. Med. Chem.,* **1999,** *42,* 2087-2104. In this instance, 1-(benzyloxycarbonyl)-4-piperidinone was substituted for 4-oxopiperidine-1-carboxylic acid *tert* butyl ester.
LC/MS (+ve ion electrospray): *m*/*z* 262 (M+H)⁺.

### (b) 4-Fluoro-4-(6-methoxy-[1,5]naphthyridin-4-ylethynyl)piperidine-1-carboxylic acid benzyl ester.

This compound was prepared from ester (29a) and triflate (1b) in 47% yield, by the method of Example (27c).
LC/MS (+ve ion electrospray): *m*/*z* 420 (M+H)⁺.

### (c) 8-[2-(4-Fluoropiperidin-4-yl)ethyl]-2-methoxy-[1,5]naphthyridine

This compound was prepared from ester (29b) in 96% yield, by the method of (27d).
LC/MS (+ve ion electrospray): m/z 290 (M+H)⁺.

### (d) 6-(2-Chloroethyl)-4H-benzo[1,4]oxazin-3-one

To a stirred solution of 6-(2-chloroethanoyl)-4*H*-benzo[1,4]oxazin-3-one (10 g, 44.4 mmol; Aldrich) in trifluoroacetic acid (34 mL ,0.44 mol) at 0 °C was added dropwise, over 5 minutes, triethylsilane (15.3 mL, 0.1 mol). The resulting solution was then stirred at 45 °C for 30 minutes followed by 40 h at room temperature. The reaction solution was poured into a mixture of ice and water and the organic layer was partitioned using EtOAc. The organic phase was washed with water and brine and dried (MgSO₄). Evaporation gave a white solid (9g, 93%), which was used without additional purification.
LC/MS (+ve ion electrospray): *m*/*z* 212 (M+H)⁺.

### (e) Title compound

The title compound was prepared from benzoxazinone (29d) and piperidine (29c) in 11% yield, by the procedure of (28g), using a reaction temperature of 50 °C.
¹H NMR (CDCl₃) δ 8.87 (s, 1H); 8.66 (d, J = 11.2 Hz, 1H); 8.19 (d, J = 22.5 Hz, 1H); 7.39 (d, J = 11.2 Hz, 1H); 7.10 (d, J = 22.6 Hz, 1H); 6.87 (d, J = 4.6 Hz, 1H); 6.82 (d, J = 4.8 Hz, 1H); 6.67 (s, 1H); 4.58 (s, 2H); 4.06 (s, 3H); 3.28 (m, 2H); 2.81 (m, 2H); 2.62 (m, 2H); 2.42 (m, 2H); 2.06 (m, 2H); 2.01 (m, 2H); 1.89 (m, 2H).
LC/MS (+ve ion electrospray): *m*/*z* 465 (M+H)⁺.

The following Examples were prepared by analogous methods.

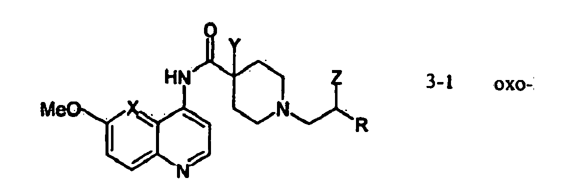

| **Example** | **Method of synthesis** | **salt B Dihydrochloride A Hydrochloride** | **X** | **Y** | **Z** | **R** |
|---|---|---|---|---|---|---|
| 40 | a | B | CH | OH | H | 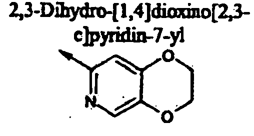 |
| 41 | b | B | N | OH | H | 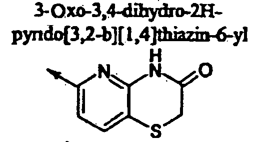 |
| 42 | c | B | N | OH | R,S-OH | 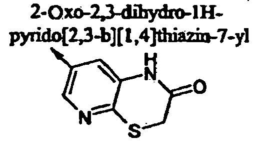 |
| 43 | d | A | N | OH | R,S-OH | 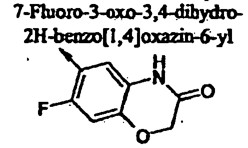 |
| 44 | e | B | N | OH | H | 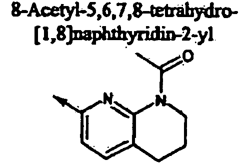 |
| 45 | f | B | N | OH | H | 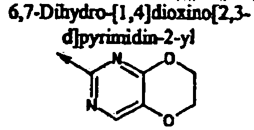 |
| 46 | g | B | N | OH | R,S-OH | 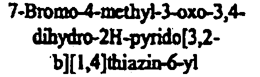 |
| | | | | | | 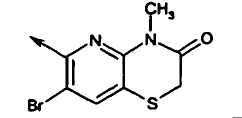 |
| 47 | h | B | N | OH | H | 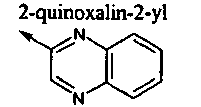 |
| 48 | i | B | N | OH | R,S-OH | 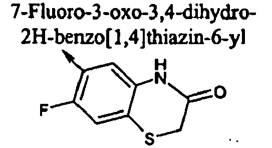 |
| 49 | j | B | N | OMe | R,S-OH | 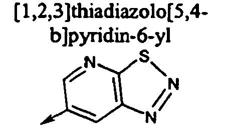 |
| 50 | k | B | N | OCH₂OMe | R,S-OH | 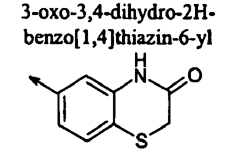 |
| 51 | 1 | B | N | OCH₂OMe | R,S-OH | 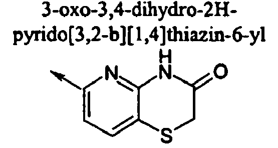 |
| 52 | m | B | N | OCOMe | R,S-OH | 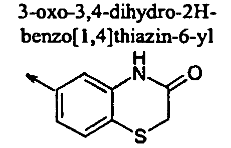 |
| 53 | n | B | N | CONH₂ | R,S-OH | 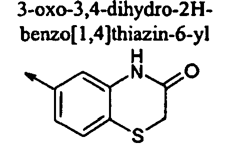 |
| 54 | o | B | N | NH₂ | =O | 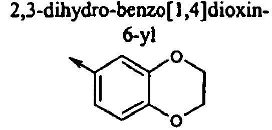 |
| 55 | p | B | N | NH₂ | R,S-OH | 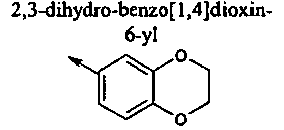 |
| 56 | q | B | N | NH₂ | R,S-OH | 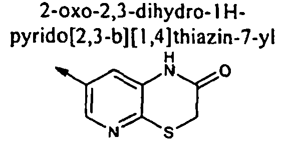 |
| 57 | r | B | N | OH | =NHOH slower running isomer | 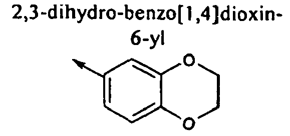 |
| 58 | r | B | N | OH | =NHOH faster running isomer | 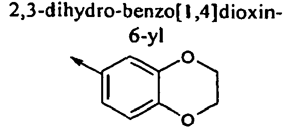 |

| | | | | | | |
|---|---|---|---|---|---|---|
| a reaction of 4-hydroxy-piperidine-4-carboxylic acid (6-methoxy-quinoline-4-yl)-amide [prepared from 6-methoxy-quinolin-4-yl-amine by the method of Example(26 d-g)] and olefin (25e) by the method of Example (25f). b reaction of piperidine (3c) with methanesulfonic acid 2-(3-oxo-3,4-dihydro-2H-pyrido[3,2-b][1,4]thiazin-6-yl)-ethyl ester [prepared from the chloromethyl ketone (Example 22c) by reduction with sodium borohydride and treatment with NaOH/EtOH to give the oxirane, hydrogenation over Pd/C to give the alcohol and reaction with methanesulfonyl chloride by the method of Example (1f)] by the method of Example (1k). c reaction of piperidine (3c) and 7-(2-chloro-ethanoyl)-1H-pyrido[2,3-b][1,4]thiazin-2-one (prepared from 6-chloro-5-nitro-nicotinic acid methyl ester by reaction with methyl thioglycolate, reduction with iron/acetic acid to give the pyridylthiazinone, hydrolysis of the ester with NaOH/THF and reaction with i-butyl chloroformate/Et₃N followed by diazomethane) by the method of Example (22d). d reaction of piperidine (3c) and 6-(2-chtoto-emanoyl)-7-fluoro-4H-beazo[1,4]oxazin-3-one (prepared from 2-fluoro-4-hydroxy-benzonitrile by nitration and hydrogenation to 5-amino-2-fluaro-4-hydroxybenzonitrile, reaction with chloroacetyl chloride by the method of Example (8a), followed by hydrolysis with sodium hydroxide to the the 6-carboxylic acid and conversion to the chloromethyl ketone by the method of Example (22c)) by the method of Example (22d). e reaction of piperidine (3c) with methanesulfonic acid 2-(8-acetyl-5,6,7,8-tetrahydro-[1,8]naphthyridin-2-yl)-ethyl ester [prepared from 2-(5,6,7,8-tetrahydro-[1,8]naphthyridin-2-ylethanol (WO/33939 & WO 01/17959)]-by reaction with TMSCI, acetic anhydride, then mesyl chloride by the method of Example (1f)] by the method of Example (1k). f reaction of piperidine (3c) with 2-vinyl-6,7-dihydro-[1,4]dioxino[2,3-d]pyrimidine [prepared from 2-hydroxymethyl-pyrimidine-4,5-diol (A. Harris, Aust. J. Chem., 1976, 29, 1335) by reaction with dibromoethane and manganese dioxide giving the aldehyde, followed by trimethylsilyl methyl magnesium bromide and then potassium t-butoxide in THF] by the method of Example (25f) g reaction of piperidine (3c) with 7-bromo-6-(2-chloro-ethanoyl)-4-methyl-4H-pyrido[3,2-b][1,4]thiazin-3-one [prepared from methyl 3,6-dibromopyridine-2-carboxylate (see TR. Kelly et al J.Org. Chem. 61, 1996, 4623-4633), by the methods of Example (22a-c), when in this case the amide was methylated with excess diazomethane] by the method of Example (22d). h reaction of piperidine (3c) with 2-vinyl-quinoxaline (prepared from 2-methylquinoxaline by reaction with dimethylamine/formaldehyde followed by quaternisation with dimethyl sulfate and refluxing in ethanol in the presence of triethylamine), by the method of Example (25f). i reaction of piperidine (3c) with 6-(2-chloro-ethanoyl)-7-fluoro-4H-benzo[1,4]thiazin-3-one (Example 11c), by the method of Example (22d). j reaction of piperidine (16d) with 2-chloro-1-[1,2,3]thiadiazolo[5,4-b]pyridin-6-yl-ethanone [prepared from 6-chloro-5-nitro-nicotinic acid methyl ester by reaction with S/Na₂S followed by diazotisation with NaNO₂/HCl to give the thiadiazole, hydrolysis with NaOH/THF to give the 6-carboxylic acid, which was converted to the chloromethyl ketone by the method of Example (22c)], by the method of Example (22d). k reaction of 4-methoxymethoxy-4-(6-methoxy-[1,5]naphthyridin-4-ylcarbamoyl)-piperidine [prepared from hydroxy acid (3a) by dialkylation with chloromethyl methyl ether and N,N-di-isopropylethylamine, followed by hydrolysis of the resulting ester with sodium hydroxide/aqueous THF, conversion to the primary amide and coupling with triflate (1b) by the method of Example (3b, Method B), and N-deprotection with trifluoroacetic acid/dichloromethane] with chloroketone (8a) by the method of Example 13. l reaction of 4-methoxymethoxy-4-(6-methoxy-[1,5]naphmyridin-4-ylcarbamoyl)-pendine (note k) with chloroketone (22c) by the method of Example (22d). m reaction of 4-acetoxy-4-(6-methoxy-[1,5]naphthyridin-4-ylcarbamoyl-piperidine [prepared from hydroxy acid (3a) by acetyiation with acetic anhydride, followed by amide formation with amine (1c) in the presence of O-(7-azabenzotriazol-1-yl)-N,N,N',N'-teramethyluronium hexafluorophosphate and triethylamine, then N-deprotection with trifluoroacetic acid/dichloromethane] with chloroketone (8a) by the method of Example 13. n reaction of piperidine (21d) and chloromethyl ketone (8a) by the method of Example 13. o reaction of piperidine (2d) and 6-(2-bromoethanoyl)-2,3-dihydro-benzo[1,4]dioxin by the method of Example 13 (first stage) p ketone Example 54 was reduced with sodium borohydride by the method of Example 13 (second stage). q reaction of piperidine (2d) with 7-(2-chloro-ethanoyl)-1H-pyrido[2,3-b][1,4]thiazin-2-one followed by reaction with sodium borohydride (see footnote c). r prepared from ketone (Example 13 - solid precipitate in first part) by heating with hydroxylamine in ethanol followed by chromatography on silica gel to separate the oxime geometrical isomers | | | | | | |

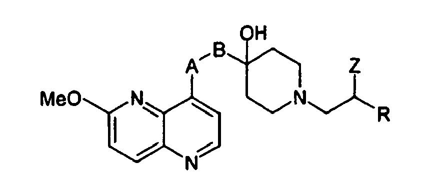

| **Example** | **Method of synthesis** | **salt B Dihydrochloride** | **A-B** | **Z** | **R** |
|---|---|---|---|---|---|
| 60 | a | B | NHCH₂ | H | 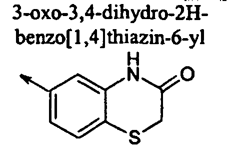 |
| 61 | b | B | NHCH₂ | R,S-OH | 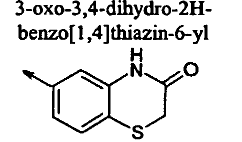 |
| 62 | c | B | NHCH₂ | H | 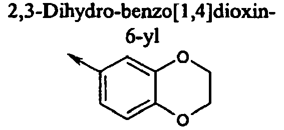 |
| 63 | d | B | CH₂CH₂ | H | 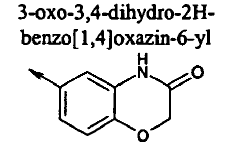 |
| 64 | e | B | NMeCH₂ | H | 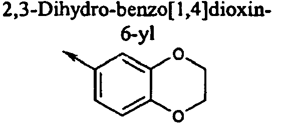 |
| 65 | f | B | NMeCH₂ | R,S-OH | 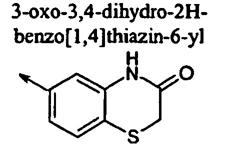 |
| 66 | g | B | NMeCH₂ | H | 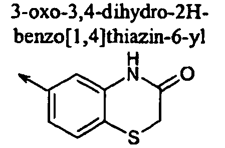 |

| | | | | | |
|---|---|---|---|---|---|
| a Amine (1c) was treated with sodium hydride then reacted with 1-oxa-6-aza-spiro[2.5]octane-6-carboxylic acid tert-butyl ester (prepared from 4-oxo-piperidine-1-carboxylic acid tert-butyl ester according to the procedure of S. Bourrain *et al,* Bioorg Med Chem Lett, 1999, 9 (23), 3369) to give 4-hydroxy-4-[(6-methoxy-[1,5]naphthyridin-4-ylamino)-methyl]-piperidine-1-carboxylic acid tert-butyl ester. This was deprotected according to the procedure of Example (1j) and the resultant 4-[(6-methoxy-[1,5]naphthyridin-4-ylamino)-methyl]-piperidin-4-ol was alkylated with mesylate (1f) according to the procedure of Example (1k). b This was prepared from 4-[(6-methoxy-[1,5]naphthyridin-4-ylamino)-methyl]-piperidin-4-ol (see Example 60 for preparation) by the method of Examples (8b) and (9). c This was prepared from 1-(2,3-dihydro-benzo[1,4]dioxin-6-yl)-ethanone by conversion to (2,3-dihydro-benzo[1,4]dioxin-6-yl)-acetic acid according to the procedure of M. Vazquez *et al,* Eur. J. Med. Chem. Chim Ther., 1997, 32 (6), 529. This was reduced with diborane-dimethylsulphide complex according to the procedure of Example (3d) to give 2-(2,3-dihydro-benzo[1,4]dioxin-6-yl)-ethanol and converted to methanesulfonic acid 2-(2,3-dihydro-benzo[1,4]dioxin-6-yl)-ethyl ester by the procedure of Example (3e). This was reacted with 4-[(6-methoxy-[1,5]naphthyridin-4-ylamino)-methyl]-piperidin-4-ol (see Example 60 for preparation) by the method of Example (1k). d Prepared by reaction of piperidine (27d) with chloroethyl-benzoxazinone (29d) by the method of Example (29e) e Triflate (1b) was reacted with methylamine to give (6-methoxy-[1,5]naphthyridin-4-yl)-methylamine. This was subjected to the same reaction sequence as for for Example 60 to give 4-{[(6-methoxy-[1,5]naphthyridin-4-yl)-methyl-amino]-methyl}-piperidin-4-ol, which was alkylated with methanesulfonic acid 2-(2,3-dihydro-benzo[1,4]dioxin-6-yl)-ethyl ester as described in Example 62. f This was prepared from 4-{[(6-methoxy-[1,5]naphthyridin-4-yl)-methyl-amino]-methyl}-piperidin-4-ol, prepared as in Example (64), by following the procedures of Examples (8b) and (9). g Alkylation of 4-{[(6-methoxy-[1,5]naphthyridin-4-yl)-methyl-amino]-methyl}-piperidin-4-ol, prepared as in example (64), with mesylate (1f) by the method of Example (1k). | | | | | |

### Biological Activity

The MIC (µg/ml) of test compounds against various organisms was determined: **S. aureus Oxford, S. aureus WCUH29, S. pneumoniae 1629, S. pneumoniae N1387, S. pneumoniae ERY 2, E. faecalis I, E. faecalis 7, H. influenzaeQ1, H. influenzae NEMC1, M. catarrhalis 1502, E.coli 7623.**
Examples 1, 2, 9, 10, 12, 14, 16, 18, 22, 23, 25, 26, 27, 28, 40, 49, 50, 51, 53, 55, 56, 58 have an MIC of less than or equal to 4µg/ml versus all these organisms; 3, 4, 7, 11, 13, 15, 17, 21, 52, 54, 57, 60 have an MIC of less than or equal to 16µg/ml versus all these organisms; 5, 8, 20, 42, 43, 46, 47, 61, 62 have an MIC less than or equal to 64µg/ml versus all these organisms; 6, 19, 48, 64, 65, 66 have an MIC less than or equal to 64µ g/ml versus some of these organisms.

The MIC (µg/ml) of further test compounds against an alternative selection of organisms was determined:
**S. aureus WCUH29, S. pneumoniae 1629, H. influenzae ATCC 51907, M. catarrhalis Ravisio.**
Example 41 had MIC values ≤4µg/ml versus all these organisms.
Example 63 had MIC values ≤16µg/ml versus all these organisms.
Examples 24, 29, 44, 45 had MIC values ≤16µg/ml versus some of these organisms.

## Claims

1. A compound of formula (I) or a pharmaceutically acceptable derivative thereof: 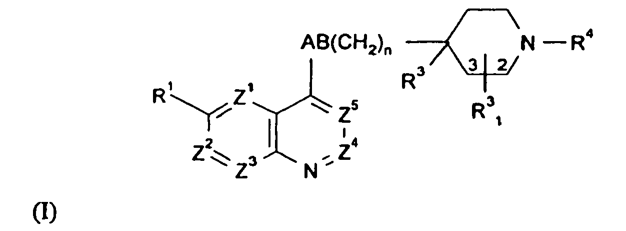 wherein:
one of Z¹, Z², Z³, Z⁴ and Z⁵ is N, one is CR^{1a} and the remainder are CH, or one or two of Z¹, Z², Z³, Z⁴ and Z⁵ are independently CR^{1a} and the remainder are CH;
R¹ and R^{1a} are independently selected from hydrogen; hydroxy; (C₁₋₆) alkoxy optionally substituted by (C₁₋₆)alkoxy, amino, piperidyl, guanidino or amidino any of which is optionally N-substituted by one or two (C ₁₋₆)alkyl, acyl or (C ₁₋₆)alkylsulphonyl groups, CONH₂, hydroxy, (C ₁₋₆)alkylthio, heterocyclylthio, heterocyclyloxy, arylthio, aryloxy, acylthio, acyloxy or (C ₁₋₆)alkylsulphonyloxy; (C ₁₋₆)alkoxy-substituted (C₁₋₆)alkyl; halogen; (C₁₋₆)alkyl; (C₁₋₆)alkylthio; trifluromethyl; trifluoromethoxy; nitro; azido; acyl; acyloxy; acylthio; (C₁₋₆)alkylsulphonyl; (C ₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups, provided that when Z¹, Z², Z³, Z⁴ and Z⁵ are CR^{1a} or CH, then R¹ is not hydrogen;
or when Z⁵ is CR^{1a}, R^{1a} may instead be cyano, hydroxymethyl or carboxy;
R³ is:
carboxy; (C₁₋₆)alkoxycarbonyl; aminocarbonyl wherein the amino group is optionally substituted by hydroxy, (C₁₋₆)alkyl, hydroxy(C₁₋₆)alkyl, aminocarbonyl(C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C ₁₋₆)alkyl, hydroxy(C ₁₋₆)alkyl, aminocarbonyl(C₁₋₆)alkyl or (C₂₋₆)alkenyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; or 5-oxo-1,2,4-oxadiazol-3-yl; or (C₁₋₄)alkyl or ethenyl optionally substituted with any of the groups listed above for R³ and/or 0 to 2 groups R¹² independently selected from:
halogen; (C₁₋₆)alkylthio; trifluoromethyl; (C₁₋₆)alkoxycarbonyl; (C₁₋₆₎ alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C ₁₋₆)alkylcarbonyl, (C₂₋₆)alkenyloxycarbonyl, (C₂₋₆)alkenylcarbonyl or aminocarbonyl wherein the amino group is optionally substituted by (C ₁₋₆)alkyl, (C₂₋₆)alkenyl, (C ₁₋₆)alkylcarbonyl or (C₂₋₆)alkenylcarbonyl; amino optionally mono- or disubstituted by (C ₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C ₁₋₆)alkylsulphonyl, (C₂₋₆)alkenylsulphonyl or aminocarbonyl wherein the amino group is optionally substituted by (C ₁₋₆)alkyl or (C₂₋₆)alkenyl; aminocarbonyl wherein the amino group is optionally substituted by (C ₁₋₆)alkyl, hydroxy(C ₁₋₆)alkyl, aminocarbonyl(C ₁₋₆)alkyl, (C₂₋₆)alkenyl, (C ₁₋₆)alkoxycarbonyl, (C ₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; oxo; (C₁₋₆)alkylsulphonyl; (C₂₋₆)alkenylsulphonyl; or (C ₁₋₆)aminosulphonyl wherein the amino group is optionally substituted by (C ₁₋₆)alkyl or (C₂₋₆)alkenyl; or
hydroxy or thiol optionally substituted by (C ₁₋₆)alkyl, (C ₁₋₄)alkoxy(C ₁₋₄)alkyl, (C₂₋₆)alkenyl, (C ₁₋₆)alkoxycarbonyl, (C ₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C ₁₋₆)alkylcarbonyl or (C₂₋₆)alkenylcarbonyl; or
amino optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl; or
halogen or trifluoromethyl;
in addition when R³ is disubstituted with a hydroxy or amino containing substituent and a carboxy containing substituent these may optionally together form a cyclic ester or amide linkage, respectively;
R¹⁰ is selected from (C₁₋₄)alkyl and (C₂₋₄)alkenyl either of which may be optionally substituted by a group R¹² as defined above; carboxy; aminocarbonyl wherein the amino group is optionally substituted by hydroxy, (C₁₋₆)alkyl, (C₂₋₆)alkenyl, (C_{1- 6})alkylsulphonyl, trifluoromethylsulphonyl, (C₂₋₆)alkenylsulphonyl, (C₁₋₆)alkoxycarbonyl, (C ₁₋₆)alkylcarbonyl, (C₂₋₆)alkenyloxycarbonyl or (C₂₋₆)alkenylcarbonyl and optionally further substituted by (C ₁₋₆)alkyl or (C₂₋₆)alkenyl; (C₁₋₆)alkylsulphonyl; trifluoromethylsulphonyl; (C₂₋₆)alkenylsulphonyl; (C₁₋₆₎ alkoxycarbonyl; (C₁₋₆)alkylcarbonyl; (C₂₋₆)alkenyloxycarbonyl; and (C₂₋₆)alkenylcarbonyl;
R³₁ is in the 2- or 3-position and is hydrogen or a group listed above for R³, provided that R³₁ in the 2-position is not optionally substituted hydroxyl, amino, trifluoromethyl or halogen;
R⁴ is a group ―U-V-R⁵₂ where R⁵₂ is an optionally substituted bicyclic carbocyclic or heterocyclic ring system (A): 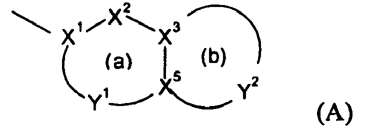
containing up to four heteroatoms in each ring in which
at least one of rings (a) and (b) is aromatic;
X¹ is C or N when part of an aromatic ring or CR¹⁴ when part of a non aromatic ring;
X² is N, NR¹³, O, S(O)ₓ, CO or CR¹⁴ when part of an aromatic or non-aromatic ring or may in addition be CR¹⁴R¹⁵ when part of a non aromatic ring;
X³ and X⁵ are independently N or C;
Y¹ is a 0 to 4 atom linker group each atom of which is independently selected from N, NR¹³, O, S(O)ₓ, CO and CR¹⁴ when part of an aromatic or non-aromatic ring or may additionally be CR¹⁴R¹⁵ when part of a non aromatic ring,
Y² is a 2 to 6 atom linker group, each atom of Y² being independently selected from N, NR¹³, O, S(O)ₓ, CO and CR¹⁴ when part of an aromatic or non-aromatic ring or may additionally be CR¹⁴R¹⁵ when part of a non aromatic ring; each of R¹⁴ and R¹⁵ is independently selected from: H; (C₁₋₄)alkylthio; halo; carboxy(C ₁₋₄)alkyl; halo(C ₁₋₄)alkoxy; halo(C ₁₋₄)alkyl; (C ₁₋₄)alkyl; (C₂₋₄)alkenyl; (C₁₋₄)alkoxycarbonyl; formyl; (C ₁₋₄)alkylcarbonyl; (C₂₋₄)alkenyloxycarbonyl; (C₂₋₄)alkenylcarbonyl; (C ₁₋₄)alkylcarbonyloxy; (C ₁₋₄)alkoxycarbonyl(C₁₋₄)alkyl; hydroxy; hydroxy(C ₁₋₄)alkyl; mercapto(C₁₋₄)alkyl; (C ₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C ₁₋₄)alkyl or (C₂₋₄)alkenyl; aryl; aryl(C ₁₋₄)alkyl; aryl(C₁₋₄)alkoxy;
each R¹³ is independently H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl (C ₁₋₄)alkyl; arylcarbonyl; heteroarylcarbonyl; (C ₁₋₄)alkoxycarbonyl; (C₁₋₄)alkylcarbonyl; formyl; (C₁₋₆)alkylsulphonyl; or aminocarbonyl wherein the amino group is optionally substituted by (C ₁₋₄)alkoxycarbonyl, (C ₁₋₄)alkylcarbonyl, (C₂₋₄)alkenyloxycarbonyl, (C₂₋₄)alkenylcarbonyl, (C ₁₋₄)alkyl or (C₂₋₄)alkenyl and optionally further substituted by (C ₁₋₄)alkyl or (C₂₋₄)alkenyl;
each x is independently 0, 1 or 2;
U is CO, SO₂ or CH₂ and V is CR¹⁷R¹⁸ or U is CH₂ and V is CO, C=NOR¹⁹ or SO₂; R¹⁷ and R¹⁸ are independently selected from hydrogen, hydroxy optionally substituted by (C ₁₋₆)alkyl, (C₂₋₆)alkenyl, (C ₁₋₆)alkoxycarbonyl, (C ₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; and amino optionally mono- or disubstituted by (C₁₋₆)alkoxycarbonyl, (C ₁₋₆)alkylcarbonyl, (C₂₋₆)alkenyloxycarbonyl, (C₂₋₆)alkenylcarbonyl, (C ₁₋₆)alkyl, (C₂₋₆)alkenyl, (C ₁₋₆)alkylsulphonyl, (C₂₋₆)alkenylsulphonyl or aminocarbonyl wherein the amino group is optionally substituted by (C₁₋₆)alkyl or (C₂₋₆)alkenyl;
R¹⁹ is hydrogen or is selected from (C ₁₋₄)alkyl and (C₂₋₄)alkenyl optionally substituted with any of the substituents listed above for R³ (C₁₋₄)alkyl or ethenyl;
n is 0 or 1 and AB is NR¹¹CO, CONR¹¹, CO-CR⁸R⁹, CR⁶R⁷-CO, O-CR⁸R⁹, CR⁶R⁷⁻O, NR¹¹-CR⁸R⁹, CR⁶R⁷- NR¹¹, NR¹¹SO₂, CR⁶R⁷-SO₂ or CR⁶R⁷-CR⁸R⁹,
or n is 0 and AB is NH-CO-NH or NH-CO-O;
or n is 0 and AB is CR⁶R⁷SO₂NR¹¹, CR⁶R⁷CONR¹¹ or CR⁶R⁷CH₂NR¹¹;
provided that when n=0 and AB is linked by a heteroatom to piperidine, R³ is optionally substituted (C₁₋₄)alkyl or ethenyl;
provided that R⁶ and R⁷, and R⁸ and R⁹ are not both optionally substituted hydroxy or amino;
and wherein:
each of R⁶, R⁷, R⁸ and R⁹ is independently selected from: H; (C₁₋₆)alkoxy; (C₁₋₆)alkylthio; halo; trifluoromethyl; azido; (C₁₋₆)alkyl; (C₂₋₆)alkenyl; (C₁₋₆)alkoxycarbonyl; (C₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C ₁₋₆)alkylsulphonyl; (C₂₋₆)alkenylsulphonyl; or (C₁₋₆)aminosulphonyl wherein the amino group is optionally substituted by (C₁₋₆)alkyl or (C₂₋₆)alkenyl;
or R⁶ and R⁸ together represent a bond and R⁷ and R⁹ are as above defined;
and each R¹¹ is independently H; trifluoromethyl; (C₁₋₆)alkyl; (C₂₋₆)alkenyl; (C₁₋₆)alkoxycarbonyl; (C ₁₋₆)alkylcarbonyl; or aminocarbonyl wherein the amino group is optionally substituted by (C₁₋₆)alkoxycarbonyl, (C₁₋₆)alkylcarbonyl, (C₂₋₆₎ alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl;
or where one of R³ or R³₁ and R⁶, R⁷, R⁸ or R⁹ contains a carboxy group and the other contains a hydroxy or amino group they may together form a cyclic ester or amide linkage or where R³ or R³₁ contains a carboxy group and A or B is NH they may be condensed to form a cyclic amide.

2. A compound according to claim 1 wherein Z⁵ is CH or N, Z³ is CH or CF and Z¹, Z² and Z⁴ are each CH, or Z¹ is N, Z³ is CH or CF and Z², Z⁴ and Z⁵ are each CH.

3. A compound according to claim 1 or 2 wherein R¹ is methoxy and R^{1a} is H or when Z³ is CR^{1a} it may be C-F.

4. A compound according to any preceding claim wherein R³ is OH, NH₂, (C₁₋₄)alkyl, (C ₁₋₄)alkoxy, (C ₁₋₄)alkoxy(C₁₋₄)alkoxy, carboxy, cyano, optionally substituted aminocarbonyl, (C ₁₋₄)alkylcarbonyloxy, fluoro, trifluoromethyl or CH₂OH.

5. A compound according to any preceding claim wherein R³₁ is hydrogen.

6. A compound according to any preceding claim wherein n is 0 and either A is CHOH or CH₂ and B is CH₂ or A is NH and B is CO.

7. A compound according to any preceding claim wherein R⁴ is -U-V-R⁵₂, -U-V- is -(CH₂)₂-, CH₂CH(OH), CH₂C=NOH or CH₂CO and R⁵₂ is an aromatic heterocyclic ring (A) having 8-11 ring atoms including 2-4 heteroatoms of which at least one is N or NR¹³ in which preferably Y² contains 2-3 heteroatoms, one of which is S and 1-2 are N, with one N bonded to X³, or the heterocyclic ring (A) has ring (a) aromatic selected from optionally substituted benzo and pyrido and ring (b) non-aromatic and Y² has 3-5 atoms, more preferably 4 atoms, including a heteroatom bonded to X⁵ selected from O, S or NR¹³, where R¹³ is other than hydrogen, and NHCO bonded via N to X³, or O bonded to X³.

8. A compound according to any of claims 1 to 6 wherein R⁵₂ is selected from:3-oxo-3,4-dihydro-2H-benzo[1,4]thiazin-6-yl (4H-benzo[1,4]thiazin-3-one-6-yl), 3,4-dihydro-2H-benzo[1,4]oxazin-6-yl, 3-oxo-3,4-dihydro-2*H*-pyrido[3,2-*b*][1,4]thiazin-6-yl.

9. A compound according to claim 1 selected from:
4-Methyl-1-[2-(3-oxo-3,4-dihydro-2H-benzo[1,4]thiazin-6-yl)-ethyl]-piperidine-4-carboxylic acid (6-methoxy-[1,5]naphthyridin-4-yl)-amide;
4-Amino-1-[2-(3-oxo-3,4-dihydro-2*H*-benzo[1,4]thiazin-6-yl)-ethyl]-piperidine-4-carboxylic acid (6-methoxy-[1,5]naphthyridin-4-yl)-amide;
1-(2-Benzo[1,3]dioxol-5-yl-ethyl)-4-hydroxy-piperidine-4-carboxylic acid (6-methoxy-[1,5]naphthyridin-4-yl)-amide;
1-(2-Benzo[1,3]dioxol-5-yl-ethyl)-4-methylpiperidine-4-carboxylic acid (6-methoxy-[1,5]naphthyridin-4-yl)-amide;
4-Hydroxy-1-[2-(3-oxo-3,4-dihydro-2H-benzo[1,4]thiazin-6-yl)-ethyl]-piperidine-4-carboxylic acid (6-methoxy-[1,5]naphthyridin-4-yl)-amide;
4-(6-Methoxy-[1,5]naphthyridin-4-ylcarbamoyl)-1-[2-(3-oxo-3,4-dihydro-2H-benzo[1,4]thiazin-6-yl)-ethyl]-piperidine-4-carboxylic acid;
6-{1-*R,S*-Hydroxy-2-[2-(6-methoxy-[1,5]naphthyridin-4-yl)-1-oxo-2,8-diaza-spiro[4.5]dec-8-yl]-ethyl}-4H-benzo[1,4]oxazin-3-one;
4-Hydroxy-1-[2-oxo-2-(3-oxo-3,4-dihydro-2H-benzo[1,4]thiazin-6-yl)-ethyl]-piperidine-4-carboxylic acid (6-methoxy-[1,5]naphthyridin-4-yl)-amide;
4-Hydroxy-l-[2- *R,S*-hydroxy-2-(3-oxo-3,4-dihydro-2*H*-benzo[1,4]thiazin-6-yl)-ethyl]-piperidine-4-carboxylic acid (6-methoxy-[1,5]naphthyridin-4-yl)-amide;
4-Hydroxymethyl-1-[2-(3-oxo-3,4-dihydro-2*H*-benzo[1,4]thiazin-6-yl)-ethyl]-piperidine-4-carboxylic acid (6-methoxy-[1,5]naphthyridin-4-yl)-amide;
4-Amino-1-[2-(7-fluoro-3-oxo-3,4-dihydro-2*H*-benzo[1,4]thiazin-6-yl)-2-*R,S*-hydroxyethyl]-piperidine-4-carboxylic acid (6-methoxy-[1,5]naphthyridin-4-yl)-amide;
4-Amino-1-[2- *R,S*-hydroxy-2-(3-oxo-3,4-dihydro-2*H*-benzo[1,4]thiazin-6-yl)-ethyl]-piperidine-4-carboxylic acid (6-methoxy-[1,5]naphthyridin-4-yl)-amide; or
1-[2-(2,3-Dihydro-benzo[1,4]dioxin-6-yl)-2-(R,S)-hydroxy-ethyl]- 4-hydroxy-piperidine-4-carboxylic acid (6-methoxy-[1,5]naphthyridin-4-yl)-amide;
4-Hydroxy-1-[2-(6-oxo-6,7-dihydro-5H-pyridazino[3,4-b][1,4]thiazin-3-yl)-ethyl]-piperidine-4-carboxylic acid (6-methoxy-[1,5]naphthyridin-4-yl)-amide;
4-Hydroxy-1-[2-R-hydroxy-2-(3-oxo-3,4-dihydro-2H-benzo[1,4]thiazin-6-yl)-ethyl]-piperidine-4-carboxylic acid (6-methoxy-[1,5]naphthyridin-4-yl)-amide ;
4-Hydroxy-1-[2-S-hydroxy-2-(3-oxo-3,4-dihydro-2H-benzo[1,4]thiazin-6-yl)-ethyl]-piperidine-4-carboxylic acid (6-methoxy-[1,5]naphthyridin-4-yl)-amide ;
1-[2-*R,S*-Hydroxy-2-(3-oxo-3,4-dihydro-2*H*-benzo[1,4]thiazin-6-yl)-ethyl]-4-methoxy-piperidine-4-carboxylic acid (6-methoxy-[1,5]naphthyridin-4-yl)-amide;
1-[2-(2,3-Dihydro-benzo[1,4]dioxin-6-yl)-2- *R,S*-hydroxy-ethyl]-4-methoxy-piperidine-4-carboxylic acid (6-methoxy-[1,5]naphthyridin-4-yl)-amide;
(3S/R,4R/S)-3,4-Dihydroxy-1-[2-(3-oxo-3,4-dihydro-2*H*-benzo[1,4]thiazin-6-yl)-ethyl]-piperidine-4-carboxylic acid (6-methoxy-[1,5]naphthyridin-4-yl)-amide;
4-*R*/*S*-Hydroxy-3-*S*/*R*-methoxy-1-[2-(3-oxo-3,4-dihydro-2*H*-benzo[1,4]thiazin-6-yl)-ethyl]-piperidine-4-carboxylic acid (6-methoxy-[1,5]naphthyridin-4-yl)-amide;
4-Cyano-1-[2-(3-oxo-3,4-dihydro-2*H*-benzo[ 1,4]thiazin-6-yl)-ethyl]-piperidine-4-carboxylic acid (6-methoxy-[1,5]naphthyridin-4-yl)-amide;
1-[2-(2,3-Dihydro-benzo[1,4]dioxin-6-yl)-2-*R,S*-hydroxy-ethyl]-piperidine-4,4-dicarboxylic acid amide (6-methoxy-[1,5]naphthyridin-4-yl)-amide;
4-Hydroxy-1-[2-(RS)-2-hydroxy-2-(3-oxo-3,4-dihydro-2*H*-pyrido[3,2-*b*][1,4]thiazin-6-yl)-ethyl]-piperidine-4-carboxylic acid (6-methoxy-[1,5]naphthyridin-4-yl) amide;
4-Methoxy-1-[2-(RS)-2-hydroxy-2-(3-oxo-3,4-dihydro-2*H*-pyrido[3,2-*b*][1,4]thiazin-6-yl)-ethyl]-piperidine-4-carboxylic acid (6-methoxy-[1,5]naphthyridin-4-yl) amide;
1-[2-(6-Oxo-6,7-dihydro-5*H*-8-thia-1,2,5-triazanaphthalen-3-yl)ethyl]-4-trifluoromethylpiperidine-4-carboxylic acid (6-methoxy-[1,5]naphthyridin-4-yl) amide;
1-[2-(2,3-Dihydro-[ 1,4]dioxino[2,3-c]pyridin-7-yl)-ethyl]-4-hydroxy-piperidine-4-carboxylic acid (6-methoxy-[1,5]naphthyridin-4-yl)-amide;
1-[2-(2,3-Dihydro-[1,4]dioxino[2,3-c]pyridin-7-yl)-ethyl]-4-hydroxy-piperidine-4-carboxylic acid (8-fluoro-6-methoxy-quinolin-4-yl)-amide;
6-((R,S)-1-Hydroxy-2-{4-hydroxy-4-[2-(6-methoxy-[1,5]naphthyridin-4-yl)ethyl]piperidin-1yl}ethyl)-4*H*-benzo[1,4]thiazin-3-one;
6-(2-{(4-hydroxy-4-[2-(6-methoxy-[1,5]naphthyridin-4-yl)ethyl]piperidin-1yl}ethyl)-4*H-*benzo[1,4]thiazin-3-one;
6-(2-{4-Fluoro-4-[2-(6-methoxy-[1,5]naphthyridin-4-yl)ethyl]piperidin-1yl}ethyl)-4*H-*benzo[1,4]thiazin-3-one;
or a pharmaceutically acceptable derivative thereof.

10. The use of a compound according to claim 1, in the manufacture of a medicament for use in the treatment of bacterial infections in mammals.

11. A pharmaceutical composition comprising a compound according to claim 1, and a pharmaceutically acceptable carrier.

12. A process for preparing compounds according to claim 1, which process comprises reacting a compound of formula (IV) with a compound of formula (V): 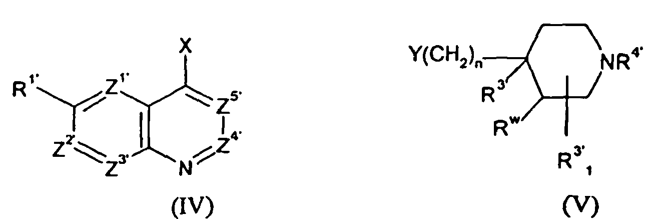 wherein n is as defined in formula (I); Z^{1'}, Z^{2'}, Z^{3'}, Z^{4'}, Z^{5'}, R^{1'}, R^{3'}₁ and R^{3'} are Z¹, Z², Z³, Z⁴, Z⁵, R¹, R³₁ and R³ as defined in formula (I) or groups convertible thereto and R^{w} is hydrogen or R^{w} and R^{3'} represent a bond;
and X and Y may be the following combinations:
(i) one of X and Y is CO₂R^{y} and the other is CH₂CO₂R^{x};
(ii) X is CHR⁶R⁷ and Y is C(=O)R⁹;
(iii) X is CR⁷=PR^{z}₃ and Y is C(=O)R⁹;
(iv) X is C(=O)R⁷ and Y is CR⁹=PR^{z}₃;
(v) one of Y and X is COW and the other is NHR^{11'} or NR¹¹COW;
(vi) X is NHR^{11'} and Y is C(=O)R⁸ or X is C(=O)R⁶ and Y is NHR^{11'};
(vii) X is NHR^{11'} and Y is CR⁸R⁹W;
(viii) X is W or OH and Y is CH₂OH;
(ix) X is NHR^{11'} and Y is SO₂W;
(x) one of X and Y is (CH₂)p-W and the other is (CH₂)_{q}NHR^{11'}, (CH₂)_{q}OH, (CH₂)_{q}SH or (CH₂)_{q}SCOR^{x} where p+q=1;
(xi) one of X and Y is OH and the other is -CH=N₂;
(xii) X is NCO and Y is OH or NH₂;
(xiii) X is CR⁶R⁷SO₂W, A'COW, CR⁶=CH₂ or oxirane and Y is NHR^{11'};
(xiv) X is W and Y is CONHR ^{11'} or OCONH₂;
(xv) X is W and Y is -CH=CH₂;
(xvi) X is W and Y is -C≡CH (followed by hydrogenation of the intermediate -C=C-group);
(xvii) X is NHR^{11'} and together Y and R³ are O and n = 1;
in which W is a leaving group, e.g. halo, methanesulphonyloxy, trifluoromethanesulphonyloxy or imidazolyl; R^{x} and RY are (C₁₋₆)alkyl; R^{Z} is aryl or (C₁₋₆)alkyl; A' and NR^{11'} are A and NR¹¹ as defined in formula (I), or groups convertible thereto; and oxirane is: 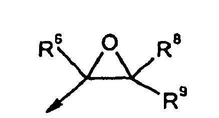 wherein R⁶, R⁸ and R⁹ are as defined in formula (I);
and thereafter optionally or as necessary converting A', Z^{1'}, Z^{2'}, Z^{3'}, Z^{4'}, Z^{5'}, R^{1'}, R^{3'}, R^{3'}₁, R^{4'} and NR^{11'} to A, Z¹, Z², Z³, Z⁴, Z⁵, R¹, R³, R³₁, R⁴ and NR^{11'}; converting R^{3'} and R^{w} when a bond into R³ and hydrogen or R³₁; converting A-B to other A-B, interconverting R¹, R³, R³₁ and/or R⁴, and/or forming a pharmaceutically acceptable derivative thereof.

## Patentansprüche

1. Verbindung der Formel (I) oder ein pharmazeutisch verträgliches Derivat davon: 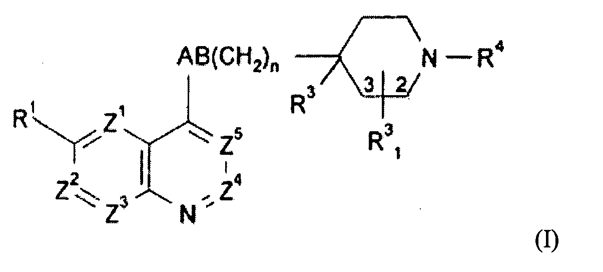 wobei:
einer der Reste Z¹, Z², Z³, Z⁴ und Z⁵ N ist, einer CR^{1a} ist und die übrigen CH sind, oder einer oder zwei der Reste Z¹, Z², Z³, Z⁴ und Z⁵ unabhängig voneinander CR^{1a} sind und die übrigen Reste CH sind;
R¹ und R^{1a} unabhängig voneinander ausgewählt sind aus Wasserstoff; Hydroxy; (C₁₋₆)-Alkoxy, gegebenenfalls substituiert mit (C₁₋₆)-Alkoxy, Amino, Piperidyl, Guanidino oder Amidino, welche jeweils gegebenenfalls N-substituiert sind mit einem oder zwei (C₁₋₆)-Alkyl-, Acyl- oder (C₁₋₆)-Alkylsulfonylresten, CONH₂, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy; (C₁₋₆)-Alkoxy substituiertem (C₁₋₆)-Alkyl; Halogen; (C₁₋₆)-Alkyl; (C₁₋₆)-Alkylthio; Trifluormethyl; Trifluormethoxy; Nitro; Azido; Acyl; Acyloxy; Acylthio; (C₁₋₆)-Alkylsulfonyl; (C₁₋₆)-Alkylsulfoxid; Arylsulfonyl; Arylsulfoxid oder einer Amino-, Piperidyl-, Guanidino- oder Amidinogruppe, gegebenenfalls N-substituiert mit einem oder zwei (C₁₋₆)-Alkyl-, Acyl- oder (C₁₋₆)-Alkylsulfonylresten,
mit der Maßgabe, dass wenn Z¹, Z², Z³, Z⁴ und Z⁵ CR^{1a} oder CH sind, R¹ dann nicht Wasserstoff ist;
oder wenn Z⁵ CR^{1a} ist, R^{1a} stattdessen Cyano, Hydroxymethyl oder Carboxy sein kann;
R³ ist:
Carboxy; (C₁₋₆)-Alkoxycarbonyl; Aminocarbonyl, wobei die Aminogruppe gegebenenfalls substituiert ist mit Hydroxy, (C₁₋₆)-Alkyl, Hydroxy-(C₁₋₆)-alkyl, Aminocarbonyl-(C₁₋₆)-alkyl, (C₂₋₆)-Alkenyl, (C₁₋₆)-Alkylsulfonyl, Trifluormethylsulfonyl, (C₂₋₆)-Alkenylsulfonyl, (C₁₋₆)-Alkoxycarbonyl, (C₁₋₆)-Alkylcarbonyl, (C₂₋₆)-Alkenyloxycarbonyl oder (C₂₋₆)-Alkenylcarbonyl und gegebenenfalls weiterhin substituiert mit (C₁₋₆)-Alkyl, Hydroxy-(C₁₋₆)-alkyl, Aminocarbonyl-(C₁₋₆)-alkyl oder (C₂₋₆)-Alkenyl; Cyano; Tetrazolyl; 2-Oxo-oxazolidinyl, gegebenenfalls substituiert mit R¹⁰; 3-Hydroxy-3-cyclobuten-1,2-dion-4-yl; 2,4-Thiazolidindion-5-yl; Tetrazol-5-ylaminocarbonyl; 1,2,4-Triazol-5-yl, gegebenenfalls substituiert mit R¹⁰; oder 5-Oxo-1,2,4-oxadiazol-3-yl; oder
(C₁₋₄)-Alkyl oder Ethenyl, gegebenenfalls substituiert mit beliebigen der vorstehend für R³ angegebenen Reste und/oder 0 bis 2 Resten R¹², unabhängig voneinander ausgewählt aus: Halogen; (C₁₋₆)-Alkylthio; Trifluormethyl; (C₁₋₆)-Alkoxycarbonyl; (C₁₋₆)-Alkylcarbonyl; (C₂₋₆)-Alkenyloxycarbonyl; (C₂₋₆)-Alkenylcarbonyl; Hydroxy, gegebenenfalls substituiert mit (C₁₋₆)-Alkyl, (C₂₋₆)-Alkenyl, (C₁₋₆)-Alkoxycarbonyl, (C₁₋₆)-Alkylcarbonyl, (C₂₋₆)-Alkenyloxycarbonyl, (C₂₋₆)-Alkenylcarbonyl oder Aminocarbonyl, wobei die Aminogruppe gegebenenfalls substituiert ist mit (C₁₋₆)-Alkyl, (C₂₋₆)-Alkenyl, (C₁₋₆)-Alkylearbonyl oder (C₂₋₆)-Alkenylcarbonyl; Amino, gegebenenfalls mono- oder disubstituiert mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl, (C₂₋₆)-Alkenyl, (C₁₋₆)-Alkylsulfonyl, (C₂₋₆)-Alkenylsulfonyl oder Aminocarbonyl, wobei die Aminogruppe gegebenenfalls substituiert ist mit (C₁₋₆)-Alkyl oder (C₂₋₆)-Alkenyl; Aminocarbonyl, wobei die Aminogruppe gegebenenfalls substituiert ist mit (C₁₋₆)-Alkyl, Hydroxy-(C₁₋₆)-alkyl, Aminocarbonyl-(C₁₋₆)-alkyl, (C₂₋₆)-Alkenyl, (C₁₋₆)-Alkoxycarbonyl, (C₁₋₆)-Alkylcarbonyl, (C₂₋₆)-Alkenyloxycarbonyl oder (C₂₋₆)-Alkenylcarbonyl und gegebenenfalls weiterhin substituiert ist mit (C₁₋₆)-Alkyl, Hydroxy-(C₁₋₆)-Alkyl, Aminocarbonyl-(C₁₋₆)-alkyl oder (C₂₋₆)-Alkenyl; Oxo; (C₁₋₆)-Alkylsulfonyl; (C₂₋₆)-Alkenylsulfonyl oder (C₁₋₆)-Aminosulfonyl, wobei die Aminogruppe gegebenenfalls substituiert ist mit (C₁₋₆)-Alkyl oder (C₂₋₆)-Alkenyl; oder
Hydroxy oder Thiol, gegebenenfalls substituiert mit (C₁₋₆)-Alkyl, (C₁₋₄)-Alkoxy-(C₁₋₄)-alkyl, (C₂₋₆)-Alkenyl, (C₁₋₆)-Alkoxycarbonyl, (C₁₋₆)-Alkylcarbonyl, (C₂₋₆)-Alkenyloxycarbonyl, (C₂₋₆)-Alkenylcarbonyl oder Aminocarbonyl, wobei die Aminogruppe gegebenenfalls substituiert ist mit (C₁₋₆)-Alkyl, (C₂₋₆)-Alkenyl, (C₁₋₆)-Alkylcarbonyl oder (C₂₋₆)-Alkenylcarbonyl; oder
Amino, gegebenenfalls mono- oder disubstituiert mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl, (C₂₋₆)-Alkenyl, (C₁₋₆)-Alkylsulfonyl, (C₂₋₆)-Alkenylsulfonyl oder Aminocarbonyl, wobei die Aminogruppe gegebenenfalls substituiert ist mit (C₁₋₆)-Alkyl oder (C₂₋₆)-Alkenyl; oder Halogen oder Trifluormethyl;
zusätzlich, wenn R³ mit einem Hydroxy oder Amino enthaltenden Substituenten und einem Carboxy enthaltenen Substituenten disubstituiert ist, diese zusammen gegebenenfalls eine cyclische Ester- bzw. Amidbindung bilden können;
R¹⁰ ausgewählt ist aus (C₁₋₄)-Alkyl und (C₂₋₄)-Alkenyl, von denen jedes gegebenenfalls substituiert sein kann mit einem Rest R¹² wie vorstehend definiert; Carboxy; Aminocarbonyl, wobei die Aminogruppe gegebenenfalls substituiert ist mit Hydroxy, (C₁₋₆)-Alkyl, (C₂₋₆)-Alkenyl, (C₁₋₆)-Alkylsulfonyl, Trifluormethylsulfonyl, (C₂₋₆)-Alkenylsulfonyl, (C₁₋₆)-Alkoxycarbonyl, (C₁₋₆)-Alkylcarbonyl, (C₂₋₆)-Alkenyloxycarbonyl oder (C₂₋₆)-Alkenylearbonyl und gegebenenfalls weiterhin substituiert mit (C₁₋₆)-Alkyl oder (C₂₋₆)-Alkenyl; (C₁₋₆)-Alkylsulfonyl; Trifluormethylsulfonyl; (C₂₋₆)-Alkenylsulfonyl; (C₁₋₆)-Alkoxycarbonyl; (C₁₋₆)-Alkylcarbonyl; (C₂₋₆)-Alkenyloxycarbonyl; und (C₂₋₆)-Alkenylcarbonyl;
R³₁ in der Position 2 oder 3 ist und Wasserstoff oder ein vorstehend für R³ angegebener Rest ist, mit der Maßgabe, dass R³₁ in der Position 2 nicht gegebenenfalls substituiertes Hydroxyl, Amino, Trifluormethyl oder Halogen ist;
R⁴ ein Rest -U-V-R⁵₂ ist, wobei R⁵₂ ein gegebenenfalls substituiertes, bicyclisches, carbocyclisches oder heterocyclisches Ringsystem (A) ist: 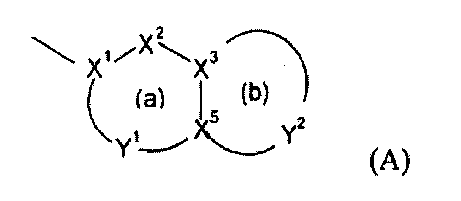
welches bis zu vier Heteroatome in jedem Ring enthält, wobei
mindestens einer der Ringe (a) und (b) aromatisch ist;
X¹ für C oder N steht, wenn Teil eines aromatischen Rings, oder für CR¹⁴ steht, wenn Teil eines nicht aromatischen Rings;
X² N, NR¹³, O, S(O)ₓ, CO oder CR¹⁴ ist, wenn Teil eines aromatischen oder nicht aromatischen Rings, oder zusätzlich CR¹⁴R¹⁵ sein kann, wenn Teil eines nicht aromatischen Rings;
X³ und X⁵ unabhängig voneinander N oder C sind;
Y¹ ein verbrückender Rest mit 0 bis 4 Atomen ist, welche unabhängig voneinander ausgewählt sind aus N, NR¹³, O, S(O)ₓ, CO und CR¹⁴, wenn Teil eines aromatischen oder nicht aromatischen Rings, oder die zusätzlich CR¹⁴R¹⁵ sein können, wenn Teil eines nicht aromatischen Rings;
Y² ein verbrückender Rest mit 2 bis 6 Atomen ist, wobei jedes Atom von Y² unabhängig ausgewählt ist aus N, NR¹³, O, S(O)ₓ, CO und CR¹⁴, wenn Teil eines aromatischen oder nicht aromatischen Rings, oder das zusätzlich CR¹⁴R¹⁵ sein kann, wenn Teil eines nicht aromatischen Rings;
R¹⁴ und R¹⁵ jeweils unabhängig voneinander ausgewählt sind aus H; (C₁₋₄)-Alkylthio; Halogen; Carboxy-(C₁₋₄)-alkyl; Halogen-(C₁₋₄)-alkoxy, Halogen-(C₁₋₄)-alkyl; (C₁₋₄)-Alkyl; (C₂₋₄)-Alkenyl; (C₁₋₄)-Alkoxycarbonyl; Formyl; (C₁₋₄)-Alkylcarbonyl; (C₂₋₄)-Alkenyloxycarbonyl; (C₂₋₄)-Alkenylcarbonyl; (C₁₋₄)-Alkylcarbonyloxy; (C₁₋₄)-Alkoxycarbonyl-(C₁₋₄)-alkyl; Hydroxy; Hydroxy-(C₁₋₄)-alkyl; Mereapto-(C₁₋₄)-alkyl; (C₁₋₄)-Alkoxy; Nitro; Cyano; Carboxy; Amino oder Aminocarbonyl, gegebenenfalls wie für entsprechende Substituenten in R³ substituiert; (C₁₋₄)-Alkylsulfonyl; (C₂₋₄)-Alkenylsulfonyl; oder Aminosulfonyl, wobei die Aminogruppe gegebenenfalls substituiert ist mit (C₁₋₄)-Alkyl oder (C₂₋₄)-Alkenyl; Aryl, Aryl-(C₁₋₄)-Alkyl; Aryl-(C₁₋₄)-alkoxy;
jeder Rest R¹³ unabhängig H; Trifluormethyl; (C₁₋₄)-Alkyl, gegebenenfalls substituiert mit Hydroxy, (C₁₋₆)-Alkoxy, (C₁₋₆)-Alkylthio, Halogen oder Trifluormethyl; (C₂₋₄)-Alkenyl; Aryl; Aryl-(C₁₋₄)-alkyl; Arylcarbonyl; Heteroarylcarbonyl; (C₁₋₄)-Alkoxycarbonyl; (C₁₋₄)-Alkylcarbonyl; Formyl; (C₁₋₆)-Alkylsulfonyl oder Aminocarbonyl ist, wobei die Aminogruppe gegebenenfalls substituiert ist mit (C₁₋₄)-Alkoxycarbonyl, (C₁₋₄)-Alkylcarbonyl, (C₂₋₄)-Alkenyloxycarbonyl, (C₂₋₄)-Alkenylcarbonyl, (C₁₋₄)-Alkyl oder (C₂₋₄)-Alkenyl und gegebenenfalls weiterhin substituiert ist mit (C₁₋₄)-Alkyl oder (C₂₋₄)-Alkenyl; jedes x unabhängig 0, 1 oder 2 ist;
U CO, SO₂ oder CH₂ ist und V CR¹⁷R¹⁸ ist oder U CH₂ ist und V CO, C=NOR¹⁹ oder SO₂ ist;
R¹⁷ und R¹⁸ unabhängig voneinander ausgewählt sind aus Wasserstoff, Hydroxy, gegebenenfalls substituiert mit (C₁₋₆)-Alkyl, (C₂₋₆)-Alkenyl, (C₁₋₆)-Alkoxycarbonyl, (C₁₋₆)-Alkylcarbonyl, (C₂₋₆)-Alkenyloxycarbonyl, (C₂₋₆)-Alkenylcarbonyl; oder Aminocarbonyl, wobei die Aminogruppe gegebenenfalls substituiert ist mit (C₁₋₆)-Alkyl, (C₂₋₆)-Alkenyl, (C₁₋₆)-Alkylcarbonyl oder (C₂₋₆)-Alkenylcarbonyl; und Amino, gegebenenfalls mono- oder disubstituiert mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl, (C₂₋₆)-Alkenyl, (C₁₋₆)-Alkylsulfonyl, (C₂₋₆)-Alkenylsulfonyl oder Aminocarbonyl, wobei die Aminogruppe gegebenenfalls substituiert ist mit (C₁₋₆)-Alkyl oder (C₂₋₆)-Alkenyl;
R¹⁹ Wasserstoff ist oder ausgewählt ist aus (C₁₋₄)-Alkyl und (C₂₋₄)-Alkenyl, gegebenenfalls substituiert mit beliebigen der vorstehend für R³ angegebenen Substituenten, (C₁₋₄)-Alkyl oder Ethenyl;
n 0 oder 1 ist, und AB NR¹¹CO, CONR¹¹, CO-CR⁸R⁹, CR⁶R⁷-CO, O-CR⁸R⁹, CR⁶R⁷-O, NR¹¹-CR⁸R⁹, CR⁶R⁷-NR¹¹, NR¹¹SO₂, CR⁶R⁷-SO₂ oder CR⁶R⁷-CR⁸R⁹ ist,
oder n 0 ist und AB NH-CO-NH oder NH-CO-O ist;
oder n 0 ist und AB CR⁶R⁷SO₂NR¹¹, CR⁶R⁷CONR¹¹ oder CR⁶R⁷CH₂NR¹¹ ist;
mit der Maßgabe, dass wenn n = 0 ist und AB durch ein Heteroatom an Piperidin gebunden ist, R³ gegebenenfalls substituiertes (C₁₋₄)-Alkyl oder Ethenyl ist;
mit der Maßgabe, dass R⁶ und R⁷, und R⁸ und R⁹ nicht beide gegebenenfalls substituiertes Hydroxy oder Amino sind;
und wobei:
R⁶, R⁷, R⁸ und R⁹ jeweils unabhängig ausgewählt sind aus H; (C₁₋₆)-Alkoxy; (C₁₋₆)-Alkylthio; Halogen; Trifluormethyl; Azido; (C₁₋₆)-Alkyl; (C₂₋₆)-Alkenyl; (C₁₋₆)-Alkoxycarbonyl; (C₁₋₆)-Alkylcarbonyl; (C₂₋₆)-Alkenyloxycarbonyl; (C₂₋₆)-Alkenylcarbonyl; Hydroxy, Amino oder Aminocarbonyl, gegebenenfalls wie für entsprechende Substituenten in R³ substituiert; (C₁₋₆)-Alkylsulfonyl; (C₂₋₆)-Alkenylsulfonyl; oder (C₁₋₆)-Aminosulfonyl, wobei die Aminogruppe gegebenenfalls substituiert ist mit (C₁₋₆)-Alkyl oder (C₂₋₆)-Alkenyl;
oder R⁶ und R⁸ zusammen eine Bindung darstellen und R⁷ und R⁹ wie vorstehend definiert sind;
und jeder Rest R¹¹ unabhängig H; Trifluormethyl; (C₁₋₆)-Alkyl; (C₂₋₆)-Alkenyl; (C₁₋₆)-Alkoxycarbonyl; (C₁₋₆)-Alkylcarbonyl; oder Aminocarbonyl ist, wobei die Aminogruppe gegebenenfalls substituiert ist mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl oder (C₂₋₆)-Alkenyl und gegebenenfalls weiterhin substituiert ist mit (C₁₋₆)-Alkyl oder (C₂₋₆)-Alkenyl; oder wenn einer der Reste R³ oder R³₁ und R⁶, R⁷, R⁸ oder R⁹ einen Carboxyrest und der andere eine Hydroxy- oder Aminogruppe enthält, diese zusammen eine cyclische Ester- bzw. Amidbindung bilden können, oder wenn R³ oder R³₁ einen Carboxyrest enthält und A oder B NH ist, diese kondensiert sein können, um ein cyclisches Amid zu bilden.

2. Verbindung gemäß Anspruch 1, wobei Z⁵ CH oder N ist, Z³ CH oder CF ist und Z¹, Z² und Z⁴ jeweils CH sind, oder Z¹ N ist, Z³ CH oder CF ist und Z², Z⁴ und Z⁵ jeweils CH sind.

3. Verbindung gemäß Anspruch 1 oder 2, wobei R¹ Methoxy ist und R^{1a} H ist, oder, wenn Z³ CR^{1a} ist, es C-F sein kann.

4. Verbindung gemäß einem der vorstehenden Ansprüche, wobei R³ OH, NH₂, (C₁₋₄)-Alkyl, (C₁₋₄)-Alkoxy, (C₁₋₄)-Alkoxy-(C₁₋₄)-alkoxy, Carboxy, Cyano, gegebenenfalls substituiertes Aminocarbonyl, (C₁₋₄)-Alkylcarbonyloxy, Fluor, Trifluormethyl oder CH₂OH ist.

5. Verbindung gemäß einem der vorstehenden Ansprüche, wobei R³₁ Wasserstoff ist.

6. Verbindung gemäß einem der vorstehenden Ansprüche, wobei n 0 ist und entweder A CHOH oder CH₂ ist und B CH₂ ist, oder A NH ist und B CO ist.

7. Verbindung gemäß einem der vorstehenden Ansprüche, wobei R⁴ -U-V-R⁵₂ ist, -U-V- -(CH₂)₂-, CH₂CH(OH), CH₂C=NOH oder CH₂CO ist und R⁵₂ ein aromatischer heterocyclischer Ring (A) mit 8 bis 11 Ringatomen einschließlich 2 bis 4 Heteroatomen ist, von denen mindestens eines N oder NR¹³ ist, wobei Y² vorzugsweise 2 bis 3 Heteroatome enthält, von denen eines S und 1 bis 2 N sind, wobei ein N an X³ gebunden ist, oder der heterocyclische Ring (A) einen aromatischen Ring (a), ausgewählt aus gegebenenfalls substituiertem Benzo und Pyrido, und einen nicht aromatischen Ring (b) aufweist und Y² 3 bis 5 Atome, stärker bevorzugt 4 Atome, aufweist, einschließlich eines Heteroatoms, welches an X⁵ gebunden sind, ausgewählt aus O, S oder NR¹³, wobei R¹³ von Wasserstoff verschieden ist, und NHCO, durch N an X³ gebunden, oder O, an X³ gebunden.

8. Verbindung gemäß einem der Ansprüche 1 bis 6, wobei R⁵₂ ausgewählt ist aus 3-Oxo-3,4-dihydro-2H-benzo[1,4]thiazin-6-yl(4H-benzo[1,4]thiazin-3-on-6-yl), 3,4-Dihydro-2H-benzo[1,4]oxazin-6-yl, 3-Oxo-3,4-dihydro-2H-pyrido[3,2-b][1,4]thiazin-6-yl.

9. Verbindung gemäß Anspruch 1, ausgewählt aus:
4-Methyl-1-[2-(3-oxo-3,4-dihydro-2H-benzo[1,4]thiazin-6-yl)ethyl]piperidin-4-carbonsäure-(6-methoxy[1,5]naphthyridin-4-yl)amid;
4-Amino-1-[2-(3-oxo-3,4-dihydro-2H-benzo[1,4]thiazin-6-yl)ethyl]piperidin-4-carbonsäure-(6-methoxy[1,5]naphthyridin-4-yl)amid;
1-(2-Benzo[1,3]dioxol-5-ylethyl)-4-hydroxypiperidin-4-carbonsäure-(6-methoxy[1,5]-naphthyridin-4-yl)amid;
1-(2-Benzo[1,3]dioxol-5-ylethyl)-4-methylpiperidin-4-carbonsäure-(6-methoxy[1,5]-naphthyridin-4-yl)amid;
4-Hydroxy-1-[2-(3-oxo-3,4-dihydro-2H-benzo[1,4]thiazin-6-yl)ethyl]piperidin-4-carbonsäure-(6-methoxy[1,5]naphthyridin-4-yl)amid;
4-(6-Methoxy[1,5]naphthyridin-4-ylcarbamoyl)-1-[2-(3-oxo-3,4-dihydro-2H-benzo[1,4]-thiazin-6-yl)ethyl]piperidin-4-carbonsäure;
6- {1-R,S-Hydroxy-2-[2-(6-methoxy[1,5]naphthyridin-4-yl)-1-oxo-2,8-diazaspiro[4.5]dec-8-yl]ethyl}-4H-benzo[1,4]oxazin-3-on;
4-Hydroxy-1-[2-oxo-2-(3-oxo-3,4-dihydro-2H-benzo[1,4]thiazin-6-yl)ethyl]piperidin-4-carbonsäure-(6-methoxy[1,5]naphthyridin-4-yl)amid;
4-Hydroxy-1-[2-R,S-hydroxy-2-(3-oxo-3,4-dihydro-2H-benzo[1,4]thiazin-6-yl)ethyl]-piperidin-4-carbonsäure-(6-methoxy[1,5]naphthyridin-4-yl)amid;
4-Hydroxymethyl-1-[2-(3-oxo-3,4-dihydro-2H-benzo[1,4]thiazin-6-yl)ethyl]piperidin-4-carbonsäure-(6-methoxy[1,5]napthyridin-4-yl)amid;
4-Amino-1-[2-(7-fluor-3-oxo-3,4-dihydro-2H-benzo[ 1,4]thiazin-6-yl)-2-R,S-hydroxyethyl]piperidin-4-carbonsäure-(6-methoxy[1,5]naphthyridin-4-yl)amid;
4-Amino-1-[2-R,S-hydroxy-2-(3-oxo-3,4-dihydro-2H-benzo[1,4]thiazin-6-yl)ethyl]-piperidin-4-carbonsäure-(6-methoxy[1,5]naphthyridin-4-yl)amid; oder
1-[2-(2,3-Dihydrobenzo[1,4]dioxin-6-yl)-2-(R,S)-hydroxyethyl]-4-hydroxypiperidin-4-carbonsäure-(6-methoxy[1,5]naphthyridin-4-yl)amid;
4-Hydroxy-1-[2-(6-oxo-6,7-dihydro-5H-pyridazino[3,4-b][1,4]thiazin-3-yl)ethyl]-piperidin-4-carbonsäure-(6-methoxy[1,5]naphthyridin-4-yl)amid;
4-Hydroxy-1-[2-R-hydroxy-2-(3-oxo-3,4-dihydro-2H-benzo[1,4]thiazin-6-yl)ethyl]-piperidin-4-carbonsäure-(6-methoxy[ 1,5]naphthyridin-4-yl)amid;
4-Hydroxy-1-[2-S-hydroxy-2-(3-oxo-3,4-dihydro-2H-benzo[1,4]thiazin-6-yl)ethyl]-piperidin-4-carbonsäure-(6-methoxy[1,5]naphthyridin-4-yl)amid;
1-[2-R,S-Hydroxy-2-(3-oxo-3,4-dihydro-2H-benzo[1,4]thiazin-6-yl)ethyl]-4-methoxypiperidin-4-carbonsäure-(6-methoxy[1,5]naphthyridin-4-yl)amid;
1-[2-(2,3-Dihydrobenzo[1,4]dioxin-6-yl)-2-R,S-hydroxyethyl]-4-methoxypiperidin-4-carbonsäure-(6-methoxy[1,5]naphthyridin-4-yl)amid;
(3S/R,4R/S)-3,4-Dihydroxy-1-[2-(3-oxo-3,4-dihydro-2H-benzo[1,4]thiazin-6-yl)ethyl]-piperidin-4-carbonsäure-(6-methoxy[1,5]naphthyridin-4-yl)amid;
4-R/S-Hydroxy-3-S/R-methoxy-1-[2-(3-oxo-3,4-dihydro-2H-benzo[1,4]thiazin-6-yl)-ethyl]piperidin-4-carbonsäure-(6-methoxy[ 1,5]naphthyridin-4-yl)amid;
4-Cyano-1-[2-(3-oxo-3,4-dihydro-2H-benzo[1,4]thiazin-6-yl)ethyl]piperidin-4-carbonsäure-(6-methoxy[1,5]naphthyridin-4-yl)amid;
1-[2-(2,3-Dihydrobenzo[1,4]dioxin-6-yl)-2-R,S-hydroxyethyl]piperidin-4,4-dicarbonsäureamid-(6-methoxy[1,5]naphthyridin-4-yl)amid;
4-Hydroxy-1-[2-(RS)-2-hydroxy-2-(3-oxo-3,4-dihydro-2H-pyrido[3,2-b][1,4]thiazin-6-yl)ethyl]piperidin-4-carbonsäure-(6-methoxy[1,5]naphthyridin-4-yl)amid;
4-Methoxy-1-[2-(RS)-2-hydroxy-2-(3-oxo-3,4-dihydro-2H-pyrido[3,2-b][1,4]thiazin-6-yl)ethyl]piperidin-4-carbonsäure-(6-methoxy[1,5]naphthyridin-4-yl)amid;
1-[2-(6-Oxo-6,7-dihydro-5H-8-thia-1,2,5-triazanaphthalen-3-yl)ethyl]-4-trifluormethylpiperidin-4-carbonsäure-(6-methoxy[1,5]naphthyridin-4-yl)amid;
1-[2-(2,3-Dihydro[1,4]dioxino[2,3-c]pyridin-7-yl)ethyl]-4-hydroxypiperidin-4-carbonsäure-(6-methoxy[1,5]naphthyridin-4-yl)amid;
1-[2-(2,3-Dihydro[1,4]dioxino[2,3-c]pyridin-7-yl)ethyl]-4-hydroxypiperidin-4-carbonsäure-(8-fluor-6-methoxychinolin-4-yl)amid;
6-((R,S)-1-Hydroxy-2-{4-hydroxy-4-[2-(6-methoxy[1,5]naphthyridin-4-yl)ethyl]-piperidin-1-yl}ethyl)-4H-benzo[1,4]thiazin-3-on;
6-(2-{4-Hydroxy-4-[2-(6-methoxy[1,5]naphthyridin-4-yl)ethyl]piperidin-1-yl}ethyl)-4H-benzo[1,4]thiazin-3-on;
6-(2- {4-Fluor-4-[2-(6-methoxy[1,5]naphthyridin-4-yl)ethyl]piperidin-1-yl}ethyl)-4H-benzo[1,4]thiazin-3-on;
oder einem pharmazeutisch verträglichen Derivat davon.

10. Verwendung einer Verbindung gemäß Anspruch 1 zur Herstellung eines Medikaments zur Verwendung bei der Behandlung von bakteriellen Infektionen bei Säugern.

11. Arzneimittel, umfassend eine Verbindung gemäß Anspruch 1 und einen pharmazeutisch verträglichen Träger.

12. Verfahren zur Herstellung von Verbindungen gemäß Anspruch 1, wobei das Verfahren das Umsetzen einer Verbindung der Formel (IV) mit einer Verbindung der Formel (V) 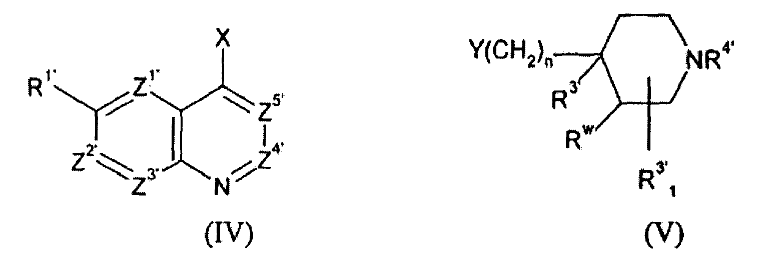 wobei n wie in Formel (I) definiert ist; Z^{1'}, Z^{2'}, Z^{3'}, Z^{4'}, Z^{5'}, R^{1'}, R^{3'}₁ und R^{3'} für Z¹, Z², Z³, Z⁴, Z⁵, R¹, R³₁ und R³ wie in Formel (I) definiert stehen oder Reste sind, die darin umwandelbar sind, und R^{w} Wasserstoff ist oder R^{w} und R^{3'} eine Bindung darstellen;
und X und Y die nachstehenden Kombinationen sein können:
(i) einer der Reste X und Y ist CO₂R^{y} und der andere ist CH₂CO₂R^{x};
(ii) X ist CHR⁶R⁷ und Y ist C(=O)R⁹;
(iii) X ist CR⁷=PR^{z}₃ und Y ist C(=O)R⁹;
(iv) X ist C(=O)R⁷ und Y ist CR⁹=PR^{z}₃;
(v) einer der Reste Y und X ist COW und der andere ist NHR^{11'} oder NR^{11'}COW;
(vi) X ist NHR^{11'} und Y ist C(=O)R⁸ oder X ist C(=O)R⁶ und Y ist NHR^{11'};
(vii) X ist NHR^{11'} und Y ist CR⁸R⁹W;
(viii) X ist W oder OH und Y ist CH₂OH;
(ix) X ist NHR^{11'} und Y ist SO₂W;
(x) einer der Reste X und Y ist (CH₂)ₚ-W und der andere ist (CH₂)_{q}NHR^{11'}, (CH₂)_{q}OH, (CH₂)_{q}SH oder (CH₂)_{q}SCOR^{x}, wobei p + q = 1 ist;
(xi) einer der Reste X und Y ist OH und der andere ist -CH=N₂;
(xii) X ist NCO und Y ist OH oder NH₂;
(xiii) X ist CR⁶R⁷SO₂W, A'COW, CR⁶=CH₂ oder Oxiran und Y ist NHR^{11'};
(xiv) X ist W und Y ist CONHR^{11'} oder OCONH₂;
(xv) X ist W und Y ist -CH=CH₂;
(xvi) X ist W und Y ist -C≡CH (gefolgt von Hydrierung der intermediären -C≡C Gruppe);
(xvii) X ist NHR^{11'} und Y und R³ zusammen sind O und n = 1;
wobei W eine Abgangsgruppe, z.B. Halogen, Methansulfonyloxy, Trifluormethansulfonyloxy oder Imidazolyl ist; R^{x} und R^{y} (C₁₋₆)-Alkyl sind; R^{z} Aryl oder (C₁₋₆)-Alkyl ist; A' und NR^{11'} für A und NR¹¹ wie in Formel (I) definiert stehen oder Reste sind, die darin umwandelbar sind; und Oxiran 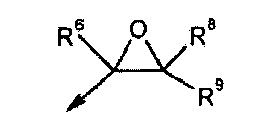 ist;
wobei R⁶, R⁸ und R⁹ wie in Formel (I) definiert sind;
und anschließend gegebenenfalls oder, wenn notwendig, Umwandeln von A', Z^{1'}, Z^{2'}, Z^{3'}, Z^{4'}, Z^{5'}, R^{1'}, R^{3'}, R^{3'}₁, R^{4'} und NR^{11'} in A, Z¹, Z², Z³, Z⁴, Z⁵, R¹, R³, R³₁, R⁴ und NR^{11'}; Umwandeln von R^{3'} und R^{w}, wenn sie eine Bindung sind, in R³ und Wasserstoff oder R³₁; Umwandeln von A-B in ein anderes A-B, ineinander Umwandeln von R¹, R³, R³₁ und/oder R⁴ und/oder Bilden eines pharmazeutisch verträglichen Derivats davon, umfasst.

## Revendications

1. Composé de formule (I) ou un de ses dérivés pharmaceutiquement acceptables : 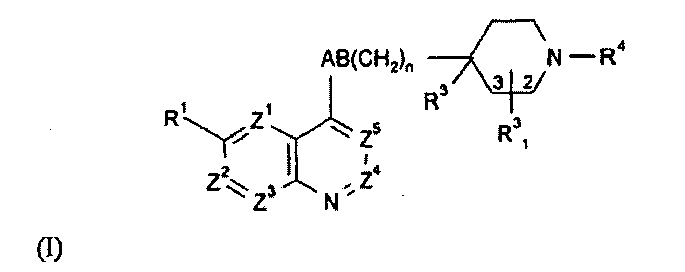 formule dans laquelle :
un de Z¹, Z², Z³, Z⁴ et Z⁵ représente N, l'un représente un groupe CR^{1a} et les groupes restants représentent des groupes CH, ou bien un ou deux de Z¹, Z², Z³, Z⁴ et Z⁵ représentent indépendamment des groupes CR^{1a} et les groupes restants représentent des groupes CH ;
R¹ et R^{1a} sont choisis indépendamment entre un atome d'hydrogène ; des groupes hydroxy ; alkoxy en C₁ à C₆ facultativement substitué avec un substituant alkoxy en C₁ à C₆, amino, pipéridyle, guanidino ou amidino, n'importe lequel de ceux-ci étant facultativement N-substitué avec un ou deux groupes alkyle en C₁ à C₆, acyle ou alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; alkyle en C₁ à C₆ à substituant alkoxy en C₁ à C₆ ; halogéno ; alkyle en C₁ à C₆ ; alkylthio en C₁ à C₆ ; trifluorométhyle ; trifluorométhoxy ; nitro ; azido ; acyle ; acyloxy ; acylthio ; alkylsulfonyle en C₁ à C₆ ; alkylsulfoxyde en C₁ à C₆ ; arylsulfonyle ; arylsulfoxyde ou un groupe amino, pipéridyle, guanidino ou amidino facultativement N-substitué avec un ou deux groupes alkyle en C₁ à C₆, acyle ou alkylsulfonyle en C₁ à C₆,
sous réserve que, lorsque Z¹, Z², Z³, Z⁴ et Z⁵ représentent des groupes CR^{1a} ou CH, alors R¹ ne représente pas un atome d'hydrogène ;
ou, lorsque Z⁵ représente un groupe CR^{1a}, R^{1a} peut représenter, en variante, un groupe cyano, hydroxyméthyle ou carboxy ;
R³ représente un groupe :
carboxy ; (alkoxy en C₁ à C₆)carbonyle ; aminocarbonyle dans lequel le groupe amino est facultativement substitué avec un substituant hydroxy, alkyle en C₁ à C₆, hydroxyalkyle en C₁ à C₆, aminocarbonyl (alkyle en C₁ à C₆), alcényle en C₂ à C₆, alkylsulfonyle en C₁ à C₆, trifluorométhylsulfonyle, alcénylsulfonyle en C₂ à C₆, (alkoxy en C₁ à C₆) carbonyle, (alkyle en C₁ à C₆) carbonyle, (alcényle en C₂ à C₆) oxycarbonyle ou (alcényle en C₂ à C₆)carbonyle, et est en outre facultativement substitué avec un substituant alkyle en C₁ à C₆, hydroxyalkyle en C₁ à C₆, aminocarbonyl (alkyle en C₁ à C₆) ou alcényle en C₂ à C₆ ; cyano ; tétrazolyle ; 2-oxo-oxazolidinyle facultativement substitué avec un substituant R¹⁰ ; 3-hydroxy-3-cyclobutène-1,2-dione-4-yle ; 2,4-thiazolidinedione-5-yle ; tétrazole-5-ylaminocarbonyle ; 1,2,4-triazole-5-yle facultativement substitué par R¹⁰ ; ou 5-oxo-1,2,4-oxadiazole-3-yle ; ou
alkyle en C₁ à C₄ ou éthényle facultativement substitué avec n'importe lequel des groupes énumérés ci-dessus pour R³ et/ou avec 0 à 2 groupes R¹² choisis indépendamment entre des groupes :
halogéno ; alkylthio en C₁ à C₆ ; trifluorométhyle ; (alkoxy en C₁ à C₆) carbonyle ; (alkyle en C₁ à C₆) - carbonyle ; (alcényle en C₂ à C₆)oxycarbonyle ;(alcényle en C₂ à C₆)carbonyle ; hydroxy facultativement substitué avec un substituant alkyle en C₁ à C₆, alcényle en C₂ à C₆, (alkoxy en C₁ à C₆) carbonyle, (alkyle en C₁ à C₆) carbonyle, (alcényle en C₂ à C₆)oxycarbonyle, (alcényle en C₂ à C₆)-carbonyle ou aminocarbonyle dans lequel le groupe amino est facultativement substitué avec un substituant alkyle en C₁ à C₆, alcényle en C₂ à C₆, (alkyle en C₁ à C₆) carbonyle ou (alcényle en C₂ à C₆)carbonyle ; amino facultativement mono- ou disubstitué avec un substituant (alkoxy en C₁ à C₆)carbonyle, (alkyle en C₁ à C₆)carbonyle, (alcényle en C₂ à C₆)oxycarbonyle, (alcényle en C₂ à C₆)carbonyle, alkyle en C₁ à C₆, alcényle en C₂ à C₆, alkylsulfonyle en C₁ à C₆, alcénylsulfonyle en C₂ à C₆ ou aminocarbonyle dans lequel le groupe amino est facultativement substitué avec un substituant alkyle en C₁ à C₆ ou alcényle en C₂ à C₆ ; aminocarbonyle dans lequel le groupe amino est facultativement substitué avec un substituant alkyle en C₁ à C₆, hydroxyalkyle en C₁ à C₆, aminocarbonyl (alkyle en C₁ à C₆), alcényle en C₂ à C₆, (alkoxy en C₁ à C₆) carbonyle, (alkyle en C₁ à C₆)carbonyle, (alcényle en C₂ à C₆)-oxycarbonyle ou (alcényle en C₂ à C₆)carbonyle, et est en outre facultativement substitué avec un substituant alkyle en C₁ à C₆, hydroxyalkyle en C₁ à C₆, aminocarbonyl(alkyle en C₁ à C₆) ou alcényle en C₂ à C₆ ; oxo ; alkylsulfonyle en C₁ à C₆ ; alcénylsulfonyle en C₂ à C₆ ; ou aminosulfonyle en C₁ à C₆ dans lequel le groupe amino est facultativement substitué avec un substituant alkyle en C₁ à C₆ ou alcényle en C₂ à C₆ ; ou
hydroxy ou thiol facultativement substitué avec un substituant alkyle en C₁ à C₆, (alkoxy en C₁ à C₄) (alkyle en C₁ à C₄), alcényle en C₂ à C₆, (alkoxy en C₁ à C₆) carbonyle, (alkyle en C₁ à C₆)carbonyle, (alcényle en C₂ à C₆)oxycarbonyle, (alcényle en C₂ à C₆)carbonyle ou aminocarbonyle dans lequel le groupe amino est facultativement substitué avec un substituant alkyle en C₁ à C₆, alcényle en C₂ à C₆, (alkyle en C₁ à C₆)carbonyle ou (alcényle en C₂ à C₆)carbonyle ; ou
amino facultativement mono- ou disubstitué avec un substituant (alkoxy en C₁ à C₆)carbonyle, (alkyle en C₁ à C₆)carbonyle, (alcényle en C₂ à C₆)oxycarbonyle, (alcényle en C₂ à C₆) carbonyle, alkyle en C₁ à C₆, alcényle en C₂ à C₆, alkylsulfonyle en C₁ à C₆, alcénylsulfonyle en C₂ à C₆ ou aminocarbonyle dans lequel le groupe amino est facultativement substitué avec un substituant alkyle en C₁ à C₆ ou alcényle en C₂ à C₆ ; ou
halogéno ou trifluorométhyle ;
en outre, lorsque R³ est disubstitué avec un substituant à fonction hydroxy ou amino et un substituant à fonction carboxy, ceux-ci peuvent former facultativement conjointement une liaison ester ou amide cyclique, respectivement ;
R¹⁰ est choisi entre des groupes alkyle en C₁ à C₄ et alcényle en C₂ à C₄, chacun pouvant être facultativement substitué avec un groupe R¹² répondant à la définition précitée ; carboxy ; aminocarbonyle dans lequel le groupe amino est facultativement substitué avec un substituant hydroxy, alkyle en C₁ à C₆, alcényle en C₂ à C₆, alkylsulfonyle en C₁ à C₆), trifluorométhylsulfonyle, alcénylsulfonyle en C₂ à C₆, (alkoxy en C₁ à C₆)carbonyle, (alkyle en C₁ à C₆) carbonyle, (alcényle en C₂ à C₆) - oxycarbonyle ou (alcényle en C₂ à C₆)carbonyle, et est en outre facultativement substitué avec un substituant alkyle en C₁ à C₆ ou alcényle en C₂ à C₆ ; alkylsulfonyle en C₁ à C₆ ; trifluorométhylsulfonyle ; alcénylsulfonyle en C₂ à C₆ ; (alkoxy en C₁ à C₆) carbonyle ; (alkyle en C₁ à C₆) - carbonyle ; (alcényle en C₂ à C₆) oxycarbonyle ; et (alcényle en C₂ à C₆)carbonyle ;
R³₁ est en position 2 ou 3 et représente un atome d'hydrogène ou un groupe indiqué ci-dessus pour R³, sous réserve que R³₁ en position 2 ne représente pas un groupe hydroxyle, amino, trifluorométhyle ou halogéno facultativement substitué ;
R⁴ représente un groupe -U-V-R⁵₂ dans lequel R⁵₂ représente un système de noyau carbocyclique ou hétérocyclique bicyclique, facultativement substitué, (A) : 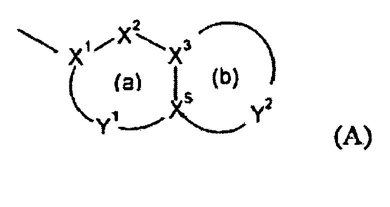
contenant jusqu'à quatre hétéroatomes dans chaque noyau, dans lequel
au moins un des noyaux (a) et (b) est aromatique ;
X¹ représente C ou N lorsqu'il fait partie d'un noyau aromatique, ou un groupe CR¹⁴ lorsqu'il fait partie d'un noyau non aromatique ;
X² représente N, un groupe NR¹³, O, S(O)ₓ, CO ou CR¹⁴ lorsqu'il fait partie d'un noyau aromatique ou non aromatique, ou peut en outre présenter un groupe CR¹⁴R¹⁵ lorsqu'il fait partie d'un noyau non aromatique ;
X³ et X⁵ représentent indépendamment N ou C ;
Y¹ représente un groupe de liaison de 0 à 4 atomes dont chaque atome est choisi indépendamment entre N, NR¹³, O, S(O)ₓ, CO ou CR¹⁴ lorsqu'il fait partie d'un noyau aromatique ou non aromatique, ou peut représenter en outre un groupe CR¹⁴R¹⁵ lorsqu'il fait partie d'un noyau non aromatique ;
Y² représente un groupe de liaison de 2 à 6 atomes, chaque atome de Y² étant choisi indépendamment entre N, NR¹³, O, S(O)ₓ, CO ou CR¹⁴ lorsqu'il fait partie d'un noyau aromatique ou non aromatique, ou peut en outre présenter un groupe CR¹⁴R¹⁵ lorsqu'il fait partie d'un noyau non aromatique ;
chacun de R¹⁴ et R¹⁵ est choisi indépendamment entre : H ; des groupes alkylthio en C₁ à C₉ ; halogéno ; carboxy(alkyle en C₁ à C₄) ; halogénalkoxy en C₁ à C₉ ; halogénalkyle en C₁ à C₄ ; alkyle en C₁ à C₄ ; alcényle en C₂ à C₄ ; (alkoxy en C₁ à C₄)carbonyle ; formyle ; (alkyle en C₁ à C₄) carbonyle ; (alcényle en C₂ à C₄)oxycarbonyle ; (alcényle en C₂ à C₄)carbonyle ; (alkyle en C₁ à C₄)carbonyloxy (alkoxy en C₁ à C₄) carbonyl (alkyle en C₁ à C₄) ; hydroxy ; hydroxyalkyle en C₁ à C₄ ; mercaptoalkyle en C₁ à C₉ ; alkoxy en C₁ à C₄ ; nitro ; cyano ; carboxy ; amino ou aminocarbonyle facultativement substitué comme pour les substituants correspondants dans R³ ; alkylsulfonyle en C₁ à C₄ ; alcénylsulfonyle en C₂ à C₄ ; ou aminosulfonyle dans lequel le groupe amino est facultativement substitué avec un substituant alkyle en C₁ à C₄ ou alcényle en C₂ à C₄ ; aryle ; aryl(alkyle en C₁ à C₄) ; aryl(alkoxy en C₁ à C₄) ;
chaque groupe R¹³ représente indépendamment H ; un groupe trifluorométhyle ; alkyle en C₁ à C₄ facultativement substitué avec un substituant hydroxy, alkoxy en C₁ à C₆, alkylthio en C₁ à C₆, halogéno ou trifluorométhyle ; alcényle en C₂ à C₄ ; aryle ; aryl(alkyle en C₁ à C₄) ; arylcarbonyle ; hétéroarylcarbonyle ; (alkoxy en C₁ à C₄)-carbonyle ; (alkyle en C₁ à C₄)carbonyle ; formyle ; alkylsulfonyle en C₁ à C₆ ; ou aminocarbonyle dans lequel le groupe amino est facultativement substitué avec un substituant (alkoxy en C₁ à C₄) carbonyle, (alkyle en C₁ à C₄) carbonyle, (alcényle en C₂ à C₄) oxycarbonyle, (alcényle en C₂ à C₄) carbonyle, alkyle en C₁ à C₄ ou alcényle en C₂ à C₄, et est en outre facultativement substitué avec un substituant alkyle en C₁ à C₄ ou alcényle en C₂ à C₄ ;
chaque indice x est indépendamment égal à 0, 1 ou 2 ;
U représente un groupe CO, SO₂ ou CH₂ et V représente un groupe CR¹⁷R¹⁸, ou bien U représente un groupe CH₂ et V représente un groupe CO, C=NOR¹⁹ ou SO₂ ;
R¹⁷ et R¹⁸ sont choisis indépendamment entre un atome d'hydrogène, un groupe hydroxy facultativement substitué avec un substituant alkyle en C₁ à C₆, alcényle en C₂ à C₆, (alkoxy en C₁ à C₆)carbonyle, (alkyle en C₁ à C₆)carbonyle, (alcényle en C₂ à C₆)oxycarbonyle, (alcényle en C₂ à C₆) - carbonyle, ou aminocarbonyle dans lequel le groupe amino est facultativement substitué avec un substituant alkyle en C₁ à C₆, alcényle en C₂ à C₆, (alkyle en C₁ à C₆)carbonyle ou (alcényle en C₂ à C₆)carbonyle ; et amino facultativement mono- ou disubstitué avec un substituant (alkoxy en C₁ à C₆)carbonyle, (alkyle en C₁ à C₆)carbonyle ou (alcényle en C₂ à C₆)oxycarbonyle, (alcényle en C₂ à C₆)carbonyle, alkyle en C₁ à C₆, alcényle en C₂ à C₆, alkylsulfonyle en C₁ à C₆, alcénylsulfonyle en C₂ à C₆ ou aminocarbonyle dans lequel le groupe amino est facultativement substitué avec un substituant alkyle en C₁ à C₆ ou alcényle en C₂ à C₆ ;
R¹⁹ représente un atome d'hydrogène ou est choisi entre des groupes alkyle en C₁ à C₉ et alcényle en C₂ à C₉ facultativement substitués avec n'importe lequel des substituants énumérés ci-dessus pour un groupe R³ alkyle en C₁ à C₄ ou éthényle ;
n est égal à 0 ou 1 et AB représente un groupe NR¹¹CO, CONR¹¹, CO-CR⁸R⁹, CR⁶R⁷-CO, O-CR⁸R⁹, CR⁶R⁷⁻O, NR¹¹-CR⁸R⁹, CR⁶R⁷⁻NR¹¹, NR¹¹SO₂, CR⁶R⁷⁻SO₂ ou CR⁶R⁷⁻CR⁸R⁹,
ou n est égal à 0 et AB représente un groupe NH-CO-NH ou NH-CO-O ;
ou bien n est égal à 0 et AB représente un groupe CR⁶R⁷SO₂NR¹¹, CR⁶R⁷CONR¹¹ ou CR⁶R⁷CH₂NR¹¹ ;
sous réserve que, lorsque n = 0 et AB est lié par un hétéroatome à un groupe pipéridine, R³ représente un groupe alkyle en C₁ à C₄ ou éthényle facultativement substitué ;
sous réserve que R⁶ et R⁷, R⁸ et R⁹, ne représentent pas tous deux un groupe hydroxy ou amino facultativement substitué ;
et dans laquelle :
chacun de R⁶, R⁷, R⁸ et R⁹ est choisi indépendamment entre : H ; des groupes alkoxy en C₁ à C₆ ; alkylthio en C₁ à C₆ ; halogéno ; trifluorométhyle ; azido ; alkyle en C₁ à C₆ ; alcényle en C₂ à C₆ ; (alkoxy en C₁ à C₆) carbonyle ; (alkyle en C₁ à C₆) carbonyle ; (alcényle en C₂ à C₆) - oxycarbonyle ; (alcényle en C₂ à C₆)carbonyle ; hydroxy, amino ou aminocarbonyle facultativement substitué comme pour les substituants correspondants dans R³ ; alkylsulfonyle en C₁ à C₆ ; alcénylsulfonyle en C₂ à C₆ ; ou aminosulfonyle en C₁ à C₆ dans lequel le groupe amino est facultativement substitué avec un substituant alkyle en C₁ à C₆ ou alcényle en C₂ à C₆ ;
ou bien R⁶ et R⁸, conjointement, représentent une liaison, et R⁷ et R⁹ répondent aux définitions précitées ;
et chaque groupe R¹¹ représente indépendamment H ; un groupe trifluorométhyle ; alkyle en C₁ à C₆ ; alcényle en C₂ à C₆ ; (alkoxy en C₁ à C₆)carbonyle ; (alkyle en C₁ à C₆)-carbonyle ; ou aminocarbonyle dans lequel le groupe amino est facultativement substitué avec un substituant (alkoxy en C₁ à C₆) carbonyle, (alkyle en C₁ à C₆) carbonyle, (alcényle en C₂ à C₆)oxycarbonyle, (alcényle en C₂ à C₆)-carbonyle, alkyle en C₁ à C₆ ou alcényle en C₂ à C₆, et est en outre facultativement substitué avec un substituant alkyle en C₁ à C₆ ou alcényle en C₂ à C₆ ;
ou bien, lorsqu'un des groupes R³ ou R³₁, et R⁶, R⁷, R⁸ ou R⁹ contient un groupe carboxy et l'autre contient un groupe hydroxy ou amino, ils peuvent former conjointement une liaison ester ou amide cyclique ou bien, lorsque R³ ou R³₁ contient un groupe carboxy et A ou B représente un groupe NH, ils peuvent être condensés pour former un amide cyclique.

2. Composé suivant la revendication 1, dans lequel Z⁵ représente un groupe CH ou N, Z³ représente un groupe CH ou CF et Z¹, Z² et Z⁴ représentent chacun un groupe CH, ou bien Z¹ représente N, Z³ représente un groupe CH ou CF et Z², Z⁴ et Z⁵ représentent chacun un groupe CH.

3. Composé suivant la revendication 1 ou 2, dans lequel R¹ représente un groupe méthoxy et R^{1a} représente H ou bien, lorsque Z³ représente un groupe CR^{1a}, il peut représenter un groupe C-F.

4. Composé suivant l'une quelconque des revendications précédentes, dans lequel R³ représente un groupe OH, NH₂, alkyle en C₁ à C₄, alkoxy en C₁ à C₄, (alkoxy en C₁ à C₄) (alkoxy en C₁ à C₄), carboxy, cyano, aminocarbonyle facultativement substitué, (alkyle en C₁ à C₄)carbonyloxy, fluoro, trifluorométhyle ou CH₂OH.

5. Composé suivant l'une quelconque des revendications précédentes, dans lequel R³₁ représente un atome d'hydrogène.

6. Composé suivant l'une quelconque des revendications précédentes, dans lequel n est égal à 0 et A représente un groupe CHOH ou CH₂ et B représente un groupe CH₂, ou bien A représente un groupe NH et B représente un groupe CO.

7. Composé suivant l'une quelconque des revendications précédentes, dans lequel R⁴ représente un groupe -U-V-R⁵₂, le groupe -U-V- représente un groupe -(CH₂)₂-, CH₂CH(OH), CH₂C=NOH ou CH₂CO et R⁵₂ représente un noyau hétérocyclique aromatique (A) ayant 8 à 11 atomes dans le noyau, y compris 2 à 4 hétéroatomes dont au moins l'un est un atome de N ou un groupe NR¹³ dans lequel, de préférence, Y² contient 2 à 3 hétéroatomes, dont l'un est un atome de S et 1 ou 2 représente des atomes de N, avec un atome de N lié à X³ ou bien le noyau hétérocyclique (A) possède un noyau (a) aromatique choisi parmi des noyaux benzo et pyrido facultativement substitués et le noyau (b) non aromatique, et Y² possède 3 à 5 atomes, plus avantageusement 4 atomes, y compris un hétéroatome lié à X⁵ choisi entre O, S et NR¹³, dans lequel R¹³ est autre qu'un atome d'hydrogène, et un groupe NHCO lié par N à X³, ou bien un atome de O lié à X³.

8. Composé suivant l'une quelconque des revendications 1 à 6, dans lequel R⁵₂ est choisi entre des groupes : 3-oxo-3,4-dihydro-2H-benzo[1,4]thiazine-6-yl(4H-benzo[1,4]thiazine-3-one-6-yle), 3,4-dihydro-2H-benzo[1,4]-oxazine-6-yle et 3-oxo-3,4-dihydro-2*H*-pyrido[3,2-*b*]-[1,4]thiazine-6-yle.

9. Composé suivant la revendication 1, choisi entre :
(6-méthoxy-[1,5]naphtyridine-4-yl)-amide d'acide 4-méthyl-1-[2-(3-oxo-3,4-dihydro-2H-benzo[1,4[thiazine-6-yl)-éthyl]-pipéridine-4-carboxylique ;
(6-méthoxy-[1,5]naphtyridine-4-yl)-amide d'acide 4-amino-1-[2-(3-oxo-3,4-dihydro-2H-benzo[1,4]thiazine-6-yl)-éthyl]-pipéridine-4-carboxylique ;
(6-méthoxy-[1,5]naphtyridine-4-yl)-amide d'acide 1-(2-benzo[1,3]dioxole-5-yl-éthyl)-4-hydroxy-pipéridine-4-carboxylique ;
(6-méthoxy-[1,5]naphtyridine-4-yl)-amide d'acide 1-(2-benzo[1,3]dioxole-5-yl-éthyl)-4-méthylpipéridine-4-carboxylique ;
(6-méthoxy-[1,5]naphtyridine-4-yl)-amide d'acide 4-hydroxy-1-[2-(3-oxo-3,4-dihydro-2H-benzo[1,4]thiazine-6-yl)-éthyl]-pipéridine-4-carboxylique ;
acide 4- (6-méthoxy-[1,5]naphtyridine-4-ylcarbamoyl)-1-[2-(3-oxo-3,4-dihydro-2H-benzo[1,4]thiazine-6-yl)-éthyl]-pipéridine-4-carboxylique
6-{1-*R,S*-hydroxy-2-[2-(6-méthoxy-[1,5]naphtyridine-4-yl)-1-oxo-2,8-diazaspiro[4,5]déc-8-yl]-éthyl}-4H-benzo[1,4]oxazine-3-one ;
(6-méthoxy-[1,5]naphtyridine-4-yl)-amide d'acide 4-hydroxy-1-[2-oxo-2-(3-oxo-3,4-dihydro-2H-benzo[1,4]thiazine-6-yl)-éthyl]-pipéridine-4-carboxylique ;
(6-méthoxy-[1,5]naphtyridine-4-yl)-amide d'acide 4-hydroxy-1-[2-*R,S*-hydroxy-2-(3-oxo-3,4-dihydro-2*H*-benzo-[1,4] thiazine-6-yl)-éthyl]-pipéridine-4-carboxylique ;
(6-méthoxy-[1,5]naphtyridine-4-yl)-amide d'acide 4-hydroxyméthyl-1-[2-(3-oxo-3,4-dihydro-2*H*-benzo[1,4]thiazine-6-yl)-éthyl]-pipéridine-4-carboxylique ;
(6-méthoxy-[1,5]naphtyridine-4-yl)-amide d'acide 4-amino-1-[2- (7-fluoro-3-oxo-3,4-dihydro-2*H*-benzo[1,4]thiazine-6-yl)-2-*R,S*-hydroxyéthyl]-pipéridine-4-carboxylique ;
(6-méthoxy-[1,5]naphtyridine-4-yl)-amide d'acide 4-amino-1-[2-*R,S*-hydroxy-2-(3-oxo-3,4-dihydro-2*H*-benzo[1,4]thiazine-6-yl)-éthyl]-pipéridine-4-carboxylique ; ou
(6-méthoxy-[1,5]naphtyridine-4-yl)-amide d'acide 1-[2-(2,3-dihydro-benzo[1,4]dioxine-6-yl)-2-(R,S)-hydroxy-éthyl]-4-hydroxy-pipéridine-4-carboxylique ;
(6-méthoxy-[1,5]naphtyridine-4-yl)-amide d'acide 4-hydroxy-1-[2-(6-oxo-6,7-dihydro-5H-pyridazino[3,4-b] [1,4]-thiazine-3-yl)-éthyl]-pipéridine-4-carboxylique ;
(6-méthoxy-[1,5]naphtyridine-4-yl)-amide d'acide 4-hydroxy-1-[2-R-hydroxy-2-(3-oxo-3,4-dihydro-2H-benzo[1,4]thiazine-6-yl)-éthyl]-pipéridine-4-carboxylique ;
(6-méthoxy-[1,5]naphtyridine-4-yl)-amide d'acide 4-hydroxy-1-[2-S-hydroxy-2-(3-oxo-3,4-dihydro-2H-benzo[1,4]thiazine-6-yl)-éthyl]-pipéridine-4-carboxylique ;
(6-méthoxy-[1,5]naphtyridine-4-yl)-amide d'acide 1-[2-*R,S*-hydroxy-2-(3-oxo-3,4-dihydro-2*H*-benzo[1,4]thiazine-6-yl)-éthyl]-4-méthoxy-pipéridine-4-carboxylique ;
(6-méthoxy-[1,5]naphtyridine-4-yl)-amide d'acide 1-[2-(2,3-dihydro-benzo[1,4]dioxine-6-yl)-2-*R,S*-hydroxy-éthyl]-4-méthoxy-pipéridine-4-carboxylique ;
(6-méthoxy-[1,5]naphtyridine-4-yl)-amide d'acide (3S/R,4R/S)-3,4-dihydroxy-1-[2-(3-oxo-3,4-dihydro-2*H*-benzo-[1,4]thiazine-6-yl)-éthyl]-pipéridine-4-carboxylique ;
(6-méthoxy-[1,5]naphtyridine-4-yl)-amide d'acide 4-*R*/*S*-hydroxy-3-*S*/*R*-méthoxy-1-[2-(3-oxo-3,4-dihydro-2*H*-benzo-[1,4]thiazine-6-yl)-éthyl]-pipéridine-4-carboxylique ;
(6-méthoxy-[1,5]naphtyridine-4-yl)-amide d'acide 4-cyano-1-[2-(3-oxo-3,4-dihydro-2*H*-benzo[1,4]thiazine-6-yl)-éthyl]-pipéridine-4-carboxylique ;
(6-méthoxy-[1,5]naphtyridine-4-yl)-amide d'acide 1-[2-(2,3-dihydro-benzo[1,4]dioxine-6-yl)-2-*R,S*-hydroxy-éthyl]-pipéridine-4,4-dicarboxylique ;
(6-méthoxy-[1,5]naphtyridine-4-yl)-amide d'acide 4-hydroxy-1-[2-(RS)-2-hydroxy-2-(3-oxo-3,4-dihydro-2H-pyrido-[3,2-*b*][1,4]thiazine-6-yl)-éthyl]-pipéridine-4-carboxylique ;
(6-méthoxy-[1,5]naphtyridine-4-yl)-amide d'acide 4-méthoxy-1-[2-(RS)-2-hydroxy-2-(3-oxo-3,4-dihydro-2H-pyrido-[3,2-*b*] [1,4]thiazine-6-yl)-éthyl]-pipéridine-4-carboxylique ;
(6-méthoxy-[1,5]naphtyridine-4-yl)-amide d'acide 1-[2-(6-oxo-6,7-dihydro-5*H*-8-thia-1,2,5-triazanaphtalène-3-yl)éthyl]-4-trifluorométhylpipéridine-4-carboxylique ;
(6-méthoxy-[1,5]naphtyridine-4-yl)-amide d'acide 1-[2-(2,3-dihydro-[1,4]dioxino[2,3-c]pyridine-7-yl)-éthyl]-4-hydroxypipéridine-4-carboxylique ;
8-fluoro-6-méthoxy-quinoléine-4-yl)-amide d'acide 1-[2-(2,3-dihydro-[1,4]dioxino[2,3-c]pyridine-7-yl)-éthyl]-4-hydroxy-pipéridine-4-carboxylique ;
6-((R,S)-1-hydroxy-2-{4-hydroxy-4-[2-(6-méthoxy-[1,5]-naphtyridine-4-yl)éthyl]pipéridine-1yl}éthyl)-4*H*-benzo[1,4-thiazine-3-one ;
6-(2-{4-hydroxy-4-[2-(6-méthoxy-[1,5]naphtyridine-4-yl)éthyl]pipéridine-1yl}éthyl)-4*H*-benzo[1,4]thiazine-3-one ;
6-(2-{4-fluoro-4-[2-(6-méthoxy-[1,5]naphtyridine-4-yl)éthyl]pipéridine-lyl}éthyl)-4*H*-benzo[1,4]thiazine-3-one ;
ou un de ses dérivés pharmaceutiquement acceptables.

10. Utilisation d'un composé suivant la revendication 1 dans la production d'un médicament destiné à être utilisé dans le traitement d'infections bactériennes chez des mammifères.

11. Composition pharmaceutique comprenant un composé suivant la revendication 1 et un support pharmaceutiquement acceptable.

12. Procédé pour la préparation de composés suivant la revendication 1, procédé qui comprend la réaction d'un composé de formule (IV) avec un composé de formule (V) : 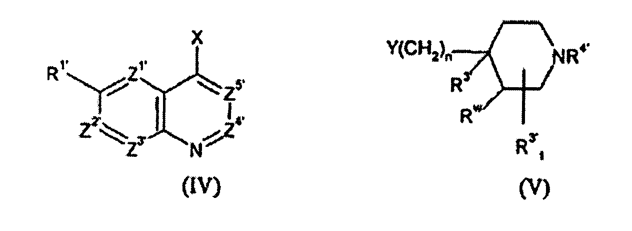 où n est comme défini pour la formule (I) ; Z^{1'}, Z^{2'}, Z^{3'}, Z^{4'} Z^{5'}, R^{1'}, R^{3'}₁ et R^{3'} représentent des groupes Z¹, Z², Z³, Z⁴, Z⁵, R¹, R³₁ et R³ comme défini pour la formule (I) ou des groupes pouvant être convertis en ceux-ci et R^{W} représente un atome d'hydrogène, ou bien R^{W} et R^{3'} représentent une liaison ;
et X et Y peuvent représenter les associations suivantes :
(i) un de X et Y représente un groupe CO₂R^{y} et l'autre représente un groupe CH₂CO₂R^{x} ;
(ii) X représente un groupe CHR⁶R⁷ et Y représente un groupe C(=O)R⁹ ;
(iii) X représente un groupe CR⁷=PR^{z}₃ et Y représente un groupe C(=O)R⁹;
(iv) X représente un groupe C(=O)R⁷ et Y représente un groupe CR⁹=PR^{z}₃ ;
(v) un de Y et X représente un groupe COW et l'autre représente un groupe NHR^{11'} ou NR^{11'}COW ;
(vi) X représente un groupe NHR^{11'} et Y représente un groupe C(=O)R⁸, ou bien X représente un groupe C(=O)R⁶ et Y représente un groupe NHR^{11'} ;
(vii) X représente un groupe NHR^{11'} et Y représente un groupe CR⁸R⁹W ;
(viii) X représente un groupe W ou OH et Y représente un groupe CH₂OH ;
(ix) X représente un groupe NHR^{11'} et Y représente un groupe SO₂W ;
(x) un de X et Y représente un groupe (CH₂)ₚ-W et l'autre représente un groupe (CH₂)_{q}NHR^{11'}, (CH₂)_{q}OH, (CH₂)_{q}SH ou (CH₂)_{q}SCOR^{x} dans lequel p+q=1 ;
(xi) un de X et Y représente un groupe OH et l'autre représente un groupe -CH=N₂ ;
(xii) X représente un groupe NCO et Y représente un groupe OH ou NH₂ ;
(xiii) X représente un groupe CR⁶R⁷SO₂W, A'COW, CR⁶=CH₂ ou l'oxiranne et Y représente NHR^{11'} ;
(xiv) X représente un groupe W et Y représente CONHR^{11'} ou OCONH₂ ;
(xv) X représente un groupe W et Y représente un groupe -CH=CH₂ ;
(xvi) X représente un groupe W et Y représente un groupe -C≡CH (suivi par l'hydrogénation du groupe -C≡C-intermédiaire) ;
(xvii) X représente un groupe NHR^{11'} et, pris conjointement, Y et R³ représentent 0 et n=1 ;
où W représente un groupe partant, par exemple halogéno, méthanesulfonyloxy, trifluorométhanesulfonyloxy ou imidazolyle ; R^{x} et R^{y} représentent des groupes alkyle en C₁ à C₆ ; R^{z} représente un groupe aryle ou alkyle en C₁ à C₆ ; A' et NR^{11'} représentent des groupes A et NR¹¹ tels que définis pour la formule (I) ou des groupes pouvant être convertis en ceux-ci ; et l'oxiranne est un groupe : 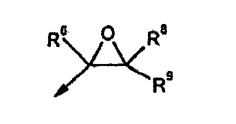 dans lequel R⁶, R⁸ et R⁹ sont définis comme pour la formule (I) ;
et ensuite, facultativement substitué ou comme nécessaire, la conversion de A' Z^{1'}, Z^{2'}, Z^{3'}, Z^{4'}, Z^{5'}, R^{1'}, R^{3'}, R^{3'}₁, R^{4'} et NR^{11'} en A, Z¹, Z², Z³, Z⁴, Z⁵, R¹, R³, R³₁, R⁴ et NR¹¹ ; la conversion de R^{3'} et R^{w} lorsqu'ils représentent une liaison en un groupe R³ et un atome d'hydrogène ou un groupe R³₁ ; la conversion d'un groupe A-B en A-B, l'interconversion de R¹, R³, R³₁ et/ou R⁴, et/ou la formation d'un dérivé pharmaceutiquement acceptable du composé obtenu.
